# EUROPEAN PATENT APPLICATION

(11) **EP 3 808 765 A1**
(43) Date of publication of application: **21.04.2021**
(21) Application number: 19202970.0
(22) Date of filing: 14.10.2019
(51) Int. Cl.: C07K 14/725, C12N 5/0783, G01N 33/50

(54) **CELL LINE FOR TCR DISCOVERY AND ENGINEERING AND METHODS OF USE THEREOF**

(71) Applicant: ETH Zurich, 8092 Zurich (CH)
(72) Inventor: REDDY, Sai, 4051 Basel (CH); VAZQUEZ-LOMBARDI, Rodrigo, 4125 Riehen (CH); JUNG, Johanna, 72135 Dettenhausen (DE)

(57) **Abstract**

The present invention is in the field of immunology and provides a cell line for the discovery and engineering of T cell receptors. The invention further provides methods for the discovery of antigen-specific T cell receptors and for the engineering of T cell receptors with optimized antigen-binding and antigen-induced signaling properties.

## Description

### ABSTRACT

The present invention is in the field of immunology and provides a cell line for the discovery and engineering of T cell receptors. The invention further provides methods for the discovery of antigen-specific T cell receptors and for the engineering of T cell receptors with optimized antigen-binding and antigen-induced signaling properties.

### INTRODUCTION

In recent years, the clinical success of immunotherapies harnessing the specificity and cytotoxicity of T cells has established them as a new pillar of cancer treatment. One example is the use of monoclonal antibodies that target immune checkpoint molecules (e.g. CTLA-4, PD-1) on the surface of tumor infiltrating T lymphocytes (TILs) resulting in potent anti-tumor immunity. Immune checkpoint blockade has led to significant increases in the survival of patients suffering from advanced metastatic melanoma, with some combination treatments resulting in a remarkable objective response rate of 29% to 53%. Despite their success and growing clinical use, there is still a substantial proportion of patients that do not respond to immune checkpoint therapies; one of the primary reasons is that many tumors are poorly infiltrated by immune cells. An immunotherapy strategy that aims to circumvent this limitation is the development of genetically engineered T cells.

The recent regulatory approval of two chimeric antigen receptor (CAR) T cell therapies in both the US and Europe represents a turning point in the field of cancer immunotherapy and personalized medicine. These first-in-class drugs rely on the isolation, genetic modification and re-infusion of autologous patient T cells. A CAR is a synthetic receptor with an antibody binding domain engineered to recognize cancer antigens fused to a signaling domain that triggers T cell cytotoxicity, thus enabling T cells to recognize and destroy malignant cells. Clinical application of CAR T cells has shown a remarkable efficacy for various leukemias and lymphomas: CAR T cells targeting CD19 on malignant B cells have resulted in complete remission rates of up to 90%. While CAR T cells offer a major new therapeutic strategy, they are still limited in that they require surface expression of a well-defined surface antigen present only on tumor cells and not on healthy tissue, which unfortunately is a rarity for most cancers.

TCR gene therapy is an emerging T cell-based immunotherapy that is being actively developed in the clinic and has the potential to become another breakthrough in personalized medicine. Similar to CAR T cells, TCR gene therapy trials currently rely on viral transduction of autologous patient T cells, followed by their expansion and re-infusion. However, instead of antibody-based CARs, T cells are transduced with constructs encoding the α - and β -chains of tumor-reactive TCRs. Advances in single-cell sorting and sequencing technologies enable more efficient identification of tumor-reactive TCRs. Accordingly, a number of recent studies have exploited these technologies to demonstrate the feasibility of personalized TCR gene therapies. In addition, TCRs can recognize intracellular antigen targets in the form of peptides bound to major histocompatibility complexes (peptide-MHC), making them an attractive modality for the treatment of solid tumors, in which extracellular tumor-specific antigens (i.e. CAR T cell targets) have been difficult to identify.

An important feature distinguishing TCR gene therapy from CAR T cell therapy is that TCRs typically bind their peptide-MHC targets with low affinity (1-100 µM). To overcome this, directed evolution and protein engineering methods that rely on display technologies (e.g., phage and yeast display) and aim to increase TCR affinity have been employed. These require reformatting of TCRs into synthetic single-chain Vα- and Vβ-fragments, which are then used as templates for generation of recombinant libraries that are screened on the surface of phage or yeast for binding towards peptide-MHC antigen targets. In one notable example, phage display was used to increase the affinity of a TCR targeting the cancer-testis (C/T) antigen MAGE-A3. When applied as a TCR gene therapy, an unpredicted cross-reactivity was observed towards an antigen presented on cardiac cells, which ultimately resulted in treatment-induced patient deaths in a clinical trial of myeloma and melanoma. This tragic outcome raised serious doubts around TCR gene therapy and highlighted the need to screen for the specificity and cross-reactivity of engineered TCRs. Thus, there is an urgent need to develop a TCR display platform that not only relies on binding (as phage and yeast display do), but also on TCR function (i.e. signaling). Such a platform would enable the simultaneous engineering of TCR specificity and detection of cross-reactivity.

Despite its significant promise, the discovery, engineering and selection of optimal TCRs for cell therapy applications remains a time-consuming process, complicated by the low affinities of TCRs to their pMHC targets and the inherent risk of TCR cross-reactivity. An important bottleneck in current TCR discovery protocols is the utilization of primary T cells. This can become largely impractical, as either freshly isolated or thawed T cell samples (which could lead to a loss in TCR diversity) are required for screening steps. In addition, primary T cells, and TILs in particular, display a wide range of activation and exhaustion patterns. This heterogeneity could therefore result in a reduced ability to detect tumor-reactive TCRs due to the cellular context in which they are presented. Primary T cells are also routinely used for the functional characterization of pre-clinical TCR candidates introduced via viral transduction. While this is a valuable method for TCR screening, it suffers from a number of limitations including the potential for TCR chain mispairing, variable TCR expression due to random viral integration, host cell heterogeneity and low throughput. A second group of technologies aim to discover antigen-specific TCRs using fluorescently-labeled multimeric pMHC reagents. Two major limitations of this approach are that it requires prior knowledge of antigen identity and, more importantly, that many low-affinity TCRs fall below the detection limit of the assay. While recently developed technologies that make use of barcoded peptide-MHC multimer libraries show potential in TCR discovery applications, their inability to detect low-affinity TCRs remains a major limitation.

Previously developed affinity-based approaches including phage and yeast display have been used to diversify and select TCRs with improved affinities at high throughput. For this purpose, multimeric peptide-MHC reagents are utilized to identify TCR variants that bind their targets with increased strength. This has resulted in the engineering of therapeutically-relevant TCRs with hundred- to million-fold increases in affinity. While the initial focus of the TCR engineering field was to maximize affinity to peptide-MHC, it is now evident that TCRs displaying maximal affinities to their targets are not necessarily the most efficacious and may in fact be less favorable. Factors compromising the suitability of such TCRs include their increased propensity for causing T cell dysfunction and inability to undergo serial TCR triggering. However, the most serious consequence of engineering TCRs for high affinity is the introduction of life-threatening cross-reactivity, as highlighted in clinical trials targeting the MAGE-A3 tumour antigen.

WO 2017/044672 discloses a cellular platform for the screening of functional T cell receptors. This platform is based on a murine thymoma-derived cell line that is TCR negative and CD8 positive. To facilitate screening of human TCRs, the cell line is optionally equipped with a chimeric CD8 variant that is composed of the mouse CD8 transmembrane and intracellular regions and the human CD8 extracellular region. In addition, the cell line can further comprise a reporter system that provides an indication of TCR activity. To screen for functional TCRs, the cells are transduced with retroviral vectors comprising genes encoding chimeric TCRs that are composed of a human variable region fused to a murine non-variable region. The main disadvantage of this platform is that the genes encoding the TCRs randomly integrate into the genome of the cell line, including possible multiple TCR integrations per cell. This has the consequence that expression of the TCR by the cell line can significantly vary and thereby bias the screening results.

Accordingly, there is a need for a cellular platform that is not affected by T cell activation or exhaustion status and allows facile and high throughput functional screening of TCRs, wherein, preferably, the TCRs are homogenously expressed from a single locus. The technical problem is solved by the embodiments provided in the claims. That is, the present invention relates to the following items:
1. An isolated cell derived from a T cell, the cell comprising one or more reporter systems, wherein the endogenous T cell receptor (TCR) alpha gene locus and/or beta gene locus of the cell is modified such that surface expression of a TCR-CD3 complex is disrupted.
2. The cell according to item 1, wherein the isolated cell is derived from a human or murine T cell.
3. The cell according to any one of items 1 or 2, wherein the isolated cell is derived from a Jurkat T cell.
4. The cell according to any one of items 1 to 3, wherein the cell further comprises a co-receptor.
5. The cell according to item 4, wherein the co-receptor is encoded by genes that are operably linked to their corresponding promoter or to a recombinant promoter.
6. The cell according to item 5 wherein the recombinant promoter is a constitutive promoter.
7. The cell according to any one of items 4 to 6, wherein the co-receptor is CD4 or CD8.
8. The cell according to any one of items 1 to 7, wherein the one or more reporter system comprises a polynucleotide comprising a promoter region operably linked to a polynucleotide sequence encoding a detectable marker.
9. The cell according to item 8, wherein the promoter region comprises one or more transcriptional response elements.
10. The cell according to item 9, wherein at least one of the one or more transcriptional response elements is the nuclear factor of activated T cell (NFAT) response element.
11. The cell according to any one of items 8 to 10, wherein the promoter region comprises 3 NFAT response elements.
12. The cell according to any one of items 8 to 11, wherein the detectable marker is a fluorescent protein.
13. The cell according to item 12, wherein the fluorescent protein is a green fluorescent protein (GFP).
14. The cell according to any one of items 1 to 13, wherein expression and/or activity of at least one receptor involved in programmed cell death or T cell exhaustion is reduced or disrupted.
15. The cell according to item 14, wherein the receptor involved in programmed cell death or T cell exhaustion is a Fas receptor, TNFR2 and/or PD-1, in particular, wherein the receptor is a Fas receptor.
16. The cell according to any one of items 1 to 15, wherein expression and/or activity of at least one protein involved in antigen presentation is reduced or disrupted, in particular wherein the protein is beta-2 microglobulin.
17. The cell according to any one of items 1 to 16, wherein expression and/or activity of at least one protein promoting non-homologous end joining is reduced or disrupted, in particular wherein the protein is p53 or p53-binding protein 1.
18. The cell according to any one of items 1 to 17, wherein expression and/or activity of at least one protein promoting homology directed repair is increased, in particular, wherein the protein is BRCA1.
19. The cell according to any one of items 1 to 18, wherein the cell comprises a gene encoding a recombinant endonuclease.
20. The cell according to item 19, wherein the recombinant endonuclease is *Streptococcus pyogenes* Cas9.
21. A method for producing a cell expressing a TCR-CD3 complex on the cell surface, the method comprising the steps of:
   a) providing a cell according to any one of items 1 to 20;
      b) introducing a DNA fragment comprising coding sequences encoding a TCR alpha variable domain, a TCR beta variable domain and at least one of a TCR alpha constant domain and/or a TCR beta constant domain into the genome of the cell of step (a);
      c) expressing the coding sequences encoded on the DNA fragment introduced in step (b); and
   d) obtaining a cell expressing a TCR-CD3 complex on the cell surface.
22. The method according to item 21, wherein the DNA fragment of step (b) further comprises homology arms at its 5' and 3' ends that are homologous to the endogenous TCR alpha locus or the endogenous TCR beta gene locus of the cell.
23. The method according to item 22, wherein the DNA fragment further comprises a splice donor site that is located downstream of the most 3' coding sequence of the DNA fragment and upstream of the 3' homology arm.
24. The method according to any one of items 21 to 23, wherein the DNA fragment is introduced into the genome of the cell such that the coding sequences located on the DNA fragment replace the endogenous TCR alpha VJ exon or the TCR beta VDJ exon and are transcriptionally linked to the endogenous gene encoding the TCR alpha constant domain or the TCR beta constant domain, respectively.
25. The method according to any one of items 21 to 24, wherein the DNA fragment is introduced into the genome of the cell through homologous recombination, homology-directed repair, homology-independent targeted insertion or microhomology-mediated end joining.
26. The method according to item 25, wherein the DNA fragment is introduced into the genome of the cell through CRISPR-Cas9-mediated homology-directed repair.
27. A library of DNA fragments, wherein the library comprises DNA fragments comprising a coding sequence encoding a TCR alpha variable domain, a coding sequence encoding a TCR beta variable domain and at least one of a coding sequence encoding a TCR alpha constant domain and/or a coding sequence encoding a TCR beta constant domain.
28. The library of DNA fragments according to item 27, wherein the coding sequences encoding the TCR alpha variable domain, the TCR beta variable domain and at least one of the TCR alpha constant domain and/or the TCR beta constant domain have been obtained by sequencing TCR genes in a population of T cells, in particular, wherein the population of T cells comprises tumor-infiltrating lymphocytes.
29. The library of DNA fragments according to item 28, wherein the coding sequences encoding the TCR alpha and beta variable domains comprised in at least one DNA fragment of the library have been obtained from the same T cell.
30. The library of DNA fragments according to item 27, wherein at least one DNA fragment in the library comprises one or more mutations in at least one coding sequence encoding a TCR alpha variable domain, a TCR beta variable domain and at least one of a TCR alpha constant domain and/or a TCR beta constant domain compared to a DNA fragment with a known sequence.
31. The library of DNA fragments according to item 30, wherein the one or more mutations have been introduced into the coding sequences encoding the complementarity determining regions (CDRs) of the variable domains of the T cell receptor, in particular wherein the one or more mutations have been introduced into the coding sequences encoding the CDR3 of the variable alpha and/or variable beta domains of the T cell receptor.
32. The library of DNA fragments according to any one of items 30 or 31, wherein the one or more mutations have been introduced into the DNA fragments by deep mutational scanning.
33. A method for determining the potential of a cell expressing a T cell receptor to bind to and/or to be activated by an antigen, the method comprising the steps of:
   a) producing a cell expressing a T cell receptor using the method according to any one of items 21 to 26;
   b) contacting the cell of step (a) with an antigen; and
      c) determining the potential of the cell to bind to the antigen and/or to be activated by the antigen.
34. The method according to item 33, wherein the cell is determined to bind to the antigen, if the binding between the cell and the antigen is stronger compared to the binding between the cell and an irrelevant antigen, or, if the binding between the cell and the antigen is stronger compared to the binding between the antigen and a cell that expresses an irrelevant or no T cell receptor;
   and/or
   wherein the cell is determined to be activated by the antigen, if the activation of the cell by the antigen is higher compared to the activation of a cell that has been contacted with an irrelevant or no antigen, or, if the activation of the cell by the antigen is higher compared to the activation of a cell that expresses an irrelevant or no T cell receptor and has been contacted with the antigen.
35. The method according to any one of items 33 or 34, wherein the antigen is part of an antigen library.
36. A method for identifying at least one T cell receptor with optimized antigen-binding or antigen-induced signaling properties, the method comprising the steps of:
   a) producing a plurality of cells using the method according to any one of items 21 to 26, wherein the plurality of cells is produced by introducing the library of DNA fragments according to any one of items 30 to 32 into said cells;
   b) contacting the plurality of cells of step (a) with a target antigen or with one or more non-target antigens;
   c) isolating at least one cell from the plurality of cells, in which the binding between the cell and the target antigen and/or in which the activation of the cell by the target antigen is increased compared to a cell that expresses a T cell receptor that has been used as starting point for the optimized T cell receptor;
   and/or,
   in which the binding between the cell and the non-target antigen and/or in which the activation of the cell by the non-target antigen is decreased compared to a cell that expresses a T cell receptor that has been used as a starting point for the optimized T cell receptor; and
   d) identifying at least one T cell receptor with optimized antigen-binding or antigen-induced signaling properties by sequencing the DNA fragment comprised in the at least one cell isolated in step (c).
37. The method according to item 36, wherein the at least one identified T cell receptor is included in a training set for a machine learning algorithm to predict T cell receptors with improved antigen-binding or antigen-induced signaling properties.
38. A method for identifying at least one antigen-specific T cell receptor, the method comprising the steps of:
   a) producing a plurality of cells using the method according to any one of items 21 to 26, wherein the plurality of cells is produced by introducing the library of DNA fragments according to any one of items 28 to 29 into said cells;
      b) contacting the plurality of cells of step (a) with an antigen;
   c) isolating at least one cell from the plurality of cells, in which the binding between the cell and the antigen of step (b) is stronger compared to the binding between the cell and an irrelevant antigen, or, in which the binding between the cell and the antigen is stronger compared to the binding between the antigen and a cell that expresses an irrelevant or no T cell receptor;
      and/or
   in which the activation of the cell by the antigen of step (b) is higher compared to the activation of a cell that has been contacted with an irrelevant or no antigen, or, in which the activation of the cell by the antigen is higher compared to the activation of a cell that expresses an irrelevant or no T cell receptor and has been contacted with the antigen; and
   d) identifying at least one antigen-specific T cell receptor by sequencing the DNA fragment comprised in the at least one cell isolated in step (c).
39. The method according to item 38, wherein the antigen is presented on the surface of a tumor cell.
40. The method according to item 39, wherein the tumor cell and the polynucleotide sequences of the TCR coding sequences in the library of DNA fragments have been obtained from the same subject.
41. The method according to any one of items 33 to 38, wherein the antigen or the target antigen is a peptide bound to a major histocompatibility complex (MHC), in particular wherein the peptide is presented by an MHC on the surface of an antigen-presenting cell (APC) or by an MHC multimer.
42. The method according to any one of items 36 or 37, wherein the non-target antigen is a peptide that is presented by an APC, by an MHC multimer, or wherein the non-target antigen is a cell that does not present the target antigen on its cell surface.
43. The method according to any one of items 41 or 42, wherein the peptide is pulsed onto the APC or wherein the peptide is genetically encoded by the APC.
44. The method according to any one of items 33 to 43, wherein the binding between an antigen and a cell expressing a T cell receptor is determined by measuring the avidity of the T cell receptor for the antigen, in particular wherein the antigen is a peptide bound to a fluorescently-labeled MHC multimer.
45. The method according to item 44, wherein the avidity is measured by flow cytometry.
46. The method according to any one of items 33 to 43, wherein the activation of a cell expressing a T cell receptor is determined by measuring the expression of the reporter system or by measuring the expression of an activation marker after contact with an antigen, in particular wherein the antigen is a peptide presented on the surface of an APC.
47. The method according to item 46, wherein the activation marker is CD69.
48. A T cell receptor as identified by the method according to any one of items 36 to 47 or a functional portion thereof.
49. The T cell receptor or the functional portion thereof according to item 48, wherein the functional portion thereof is comprised in a soluble T cell receptor.
50. The T cell receptor or the functional portion thereof according to item 49, wherein the soluble T cell receptor is coupled to a biologically active molecule, in particular wherein the biologically active molecule is an agonistic antibody, a cytokine, or a cytotoxic agent.
51. A polynucleotide encoding a T cell receptor or a functional portion thereof according to any one of items 48 to 50.
52. A viral vector comprising the polynucleotide according to item 51, in particular wherein the viral vector is derived from a lentivirus, a retrovirus or an adenovirus.
53. A cell comprising the polynucleotide according to item 51 or the viral vector according to item 52, in particular wherein the cell is a T cell.
54. A T cell receptor or the functional portion thereof according to any one of items 48 to 50, a polynucleotide according to item 51, a viral vector according to item 52 or a cell according to item 53 for use in therapy, in particular for use in cancer therapy.
55. The T cell receptor or the functional portion thereof, the polynucleotide, the viral vector or the cell for use according to item 54, wherein T cell receptor or the functional portion thereof, the polynucleotide, the viral vector or the cell are administered to a subject.
56. The T cell receptor or the functional portion thereof, the polynucleotide, the viral vector or the cell for use according to item 55, wherein the cell has been obtained from the same subject prior to introducing the polynucleotide or viral vector into said cell.

Accordingly, in one embodiment, the invention relates to an isolated cell derived from a T cell, the cell comprising one or more reporter systems, wherein the endogenous T cell receptor (TCR) alpha gene locus and/or beta gene locus of the cell is modified such that surface expression of a TCR-CD3 complex is disrupted.

That is, the invention relates to an engineered cell line, that can be used as an improved platform for T cell receptor discovery and engineering. This platform differs from previous platforms in that it allows measuring both the binding and the cellular stimulation of a TCR in response to peptide-MHC engagement, thus incorporating physiological relevance (functional TCR signaling) to the TCR discovery and engineering process. In this context, one or more reporter systems that are expressed upon stimulation of a TCR with an antigen allow facile screening and selection of cells displaying antigen-specific TCRs at high throughput, for example by using flow cytometry.

The cell of the present invention provides a highly uniform platform for TCR discovery and/or engineering that is not affected by variables such as T cell activation or exhaustion status. While previous platforms for TCR discovery and engineering, as for example the platform described in WO 2017/044672, relied on random integration of TCR genes into the genome of the host cell by viral transduction, the platform of the present invention relies on targeted genomic integration of candidate TCR genes, thus eliminating the potential for TCR chain mispairing and resulting in homogenous TCR expression from a single locus.

Further, the cell of the present invention allows for the efficient incorporation of TCRs from a variety of sources, including patient-derived TCR libraries but also larger targeted mutagenesis and combinatorial synthetic TCR libraries. A crucial advantage of the platform is its high sensitivity, which allows not only for efficient cross-reactivity screening, but also for the detection of low-affinity TCRs that would be otherwise undetectable using currently available technologies. Thus, the cell of the present invention combines highly desirable properties for TCR discovery and engineering, namely high throughput and functional screening, thus representing a major improvement over currently available TCR display platforms.

To facilitate discovery and engineering of TCRs with the cell of the present invention, the endogenous T cell receptor (TCR) alpha gene locus and/or beta gene locus of the cell has been modified such that surface expression of the TCR-CD3 complex is disrupted. In consequence, the cell does not express endogenous TCRs, thereby reducing the bias from endogenous TCR expression and TCR chain mispairing in screening experiments. In addition, the modified TCR alpha and/or beta gene locus of the cell can serve as a recombination site for exogenous DNA fragments encoding entire TCRs or parts of TCRs. Antibodies that are directed towards CD3 or extracellular parts of the TCR allow for facile discrimination of cells that successfully integrated the exogenous DNA fragments from cells that did not integrate the DNA fragments. Thus, the cells according to the invention have the advantage that CD3 is only expressed on the cell surface if the cells successfully integrated an exogenous DNA fragment encoding an entire TCR or parts of a TCR.

The cell according to the invention may comprise any modification in the TCR alpha and/or the TCR beta locus that disrupts the expression of the TCR-CD3 complex. Within the present invention, a gene or a locus is said to be modified, if it has an altered DNA sequence. Modifications include insertions, deletions and substitution of nucleotides of a DNA sequence. Preferably, the modification in the TCR alpha and/or beta chain is an insertion, a deletion or a substitution in one or more exons of the TCR alpha and/or beta chain. More preferably, the modification in the TCR alpha and/or beta chain is an insertion, a deletion or a substitution in one or more exons of the TCR alpha and/or beta chain, wherein the insertion, the deletion or the substitution results in the introduction of one or more premature stop codons in said exons. Even more preferably, the one or more premature stop codons are introduced into the first exon of the gene(s) encoding the TCR alpha constant domain and/or the TCR beta constant domain. Most preferably, the one or more premature stop codons are introduced into the first exon of the gene encoding the TCR alpha constant domain.

Thus, in a preferred embodiment, the invention relates to the cell according to the invention, wherein the modification in the endogenous T cell receptor (TCR) alpha gene locus and/or beta gene locus is an insertion, a deletion or a substitution in one or more exons of genes encoding the TCR alpha and/or beta chain. In a more preferred embodiment, the invention relates to a cell according to the invention, wherein the insertion, the deletion or the substitution results in the introduction of at least one premature stop codon in said exons. In an even more preferred embodiment, the invention relates to a cell according to the invention, wherein at least one premature stop codon is introduced into the first exon of a gene encoding the TCR alpha and/or the TCR beta constant domain. In a most preferred embodiment, the invention relates to a cell according to the invention, wherein at least one premature stop codon is introduced into the first exon of the gene encoding the TCR alpha constant domain

Alternatively, the modification in the endogenous T cell receptor (TCR) alpha gene locus and/or beta gene locus may be an insertion, a deletion or a substitution in a regulatory element that controls the expression of the genes encoding the TCR alpha and/or beta chain. In this case, it is preferred that the modification disrupts or significantly reduces expression of the TCR alpha and/or beta genes, respectively.

Within the present invention, the TCR alpha gene locus and/or beta gene locus may be modified by any method known in the art. In certain embodiments, the TCR alpha gene locus and/or beta gene locus may be modified using CRISPR/Cas9-mediated non-homologous end joining as exemplified in Example 4 of the present application. For that, any gRNA may be used that targets the TCR alpha or beta gene locus. Preferably, a gRNA may be used that targets the first exon of the genes encoding the TCR alpha constant domain or the TCR beta constant domain, respectively. More preferably, targeting the first exons of these genes with a gRNA results in the introduction of premature stop codons in these exons.

Within the present invention, it is preferred that at least one of the genes encoding the TCR alpha constant domain and/or the TCR beta constant domain is not modified and can serve as a splice acceptor for an exogenous DNA fragment comprising coding sequences encoding a TCR alpha variable domain, a TCR beta variable domain and one of a TCR alpha constant domain and/or a TCR beta constant domain. That is, an unmodified gene encoding the TCR alpha constant domain can serve as a splice acceptor for an exogenous DNA fragment comprising coding sequences encoding a TCR alpha variable domain, a TCR beta variable domain and a TCR beta constant domain. Alternatively, an unmodified gene encoding the TCR beta constant domain can serve as a splice acceptor for an exogenous DNA fragment comprising coding sequences encoding a TCR alpha variable domain, a TCR beta variable domain and a TCR alpha constant domain. When the unmodified genes encoding the TCR alpha and/or beta constant domains are used as splice acceptors, it is preferred that these genes stay intact and that the recombination of the DNA fragment with the genome takes place upstream of these genes.

The genes encoding the TCR alpha constant domain or the TCR beta constant domain are said to serve as a splice acceptor for exogenous DNA fragments comprising coding sequences encoding a TCR alpha variable domain, a TCR beta variable domain and one of a TCR alpha constant domain and/or a TCR beta constant domain, if the exogenous DNA fragment can recombine with the genome of the cell such that the coding sequences encoded on the exogenous DNA fragment are transcriptionally linked to the gene encoding the TCR alpha or beta constant domain, respectively. To achieve this, it is preferred that the exogenous DNA fragment comprises a polynucleotide sequence that is homologous to the gene encoding the TCR alpha VJ exon or the TCR beta VDJ exon. In certain embodiments, the intact TCR alpha constant or the intact TCR beta constant domain gene may serve as a recombination site itself. If this is the case, the exogenous DNA fragment can be modified to contain polynucleotide sequences that are homologous to the TCR alpha or the TCR beta constant domain genes.

As used herein, the term "isolated cell" refers to a cell that has been removed from its *in vivo* location.

The terms "T cell receptor" and "TCR", as used herein, are used interchangeably and may refer to full length heterodimeric αβ or γδ TCRs, antigen-binding fragments of TCRs, and molecules comprising TCR CDRs or variable regions. Examples of TCRs may include, but are not limited to, full-length TCRs, antigen-binding fragments of TCRs, soluble TCRs lacking transmembrane and cytoplasmic regions, single-chain TCRs containing variable regions of TCRs attached by a flexible linker, TCR chains linked by an engineered disulfide bond, monospecific TCRs, multi-specific TCRs (including bispecific TCRs), TCR fusions, human TCRs, humanized TCRs, chimeric TCRs, recombinantly produced TCRs, and synthetic TCRs. The term may encompass wild type TCRs and genetically engineered TCRs (e.g., a chimeric TCR comprising a chimeric TCR chain which includes a first portion from a TCR of a first species and a second portion from a TCR of a second species).

As used herein, the term "TCR variable domain" is understood to encompass amino acids of a given TCR which are not included within the non-variable or constant domain as encoded by the TRAC gene for TCR alpha chains and either the TRBC1 or TRBC2 genes for TCR beta chains. In some embodiments, a TCR variable domain encompasses all amino acids of a given TCR which are encoded by a TRAV gene or a TRAJ gene for a TCR alpha chain or a TRBV gene, a TRBD gene, or a TRBJ gene for a TCR beta chain (see, e.g., T cell receptor Factsbook, (2001) LeFranc and LeFranc, Academic Press, ISBN 0-12-441352-8).

As used herein, the term "constant domain" with respect to a TCR refers to the extracellular portion of a TCR that is encoded by the TRAC gene for TCR α chains and either the TRBC1 or TRBC2 genes for TCR beta chains. The term constant domain does not include a TCR variable domain encoded by a TRAV gene or a TRAJ gene for a TCR alpha chain or a TRBV gene, a TRBD gene, or a TRBJ gene for a TCR beta chain (see, e.g., T cell receptor Factsbook, (2001) LeFranc and LeFranc, Academic Press, ISBN 0-12-441352-8).

The term "TCR alpha gene locus", as used herein, refers to the position on a chromosome of a subject that comprises the genes encoding the variable and the constant domain of the TCR alpha chain, as well as the regulatory elements that are required for the expression of these genes. The term "TCR beta gene locus", as used herein, refers to the position on a chromosome of a subject that comprises the genes encoding the variable and the constant domain of the TCR beta chain, as well as the regulatory elements that are required for the expression of these genes.

The "TCR-CD3 complex", as used herein, refers to a complex on the cell surface comprising a TCR heterodimer (either αβ or γδ) and the five clonally invariant CD3 chains δ, ε, γ, ζ and η. A modification in a TCR alpha or beta gene locus is said to disrupt surface expression of a TCR-CD3 complex, if, as a consequence of the modification, a cell fails to display a functional TCR-CD3 complex on the cell surface. Preferably, the cell displays neither a TCR-CD3 complex, nor CD3 alone on the cell surface as a consequence of the modification of the TCR alpha and/or beta gene locus. Thus, in one embodiment, the invention relates to a cell according to the invention, wherein the cell fails to express detectable amounts of CD3 on the cell surface. A protein is said to be expressed on the cell surface if the protein is embedded in or spans the cell membrane and comprises an extracellular domain that is exposed to the external side of the membrane.

A "T cell" is a type of lymphocyte which develops in the thymus gland and plays a central role in the immune response. T cells can be distinguished from other lymphocytes by the presence of a T cell receptor on the cell surface. These immune cells originate as precursor cells, derived from bone marrow, and develop into several distinct types of T cells once they have migrated into the thymus. T cell differentiation continues even after they have left the thymus. The lymphocyte of the present invention may be any T cell, for example a helper CD4+ T cell, a cytotoxic CD8+ T cell, a memory T cell, a regulatory CD4+ T cell, a natural killer T cell or a gamma delta T cell. It is, however, preferred that the cell of the present invention is derived from a cytotoxic CD8+ T cell or a helper CD4+ T cell. Within the present invention, a cell is said to be "derived" from a T cell, if the cell is cloned or a progeny from a known parental T cell.

The term "expression" as used herein is defined as the transcription and/or translation of a particular nucleotide sequence driven by its promoter. In certain instances, the present application refers to the expression of a protein. In these instances, the term "expression" is interchangeably used with the term "production" and refers to the production of the protein by expressing a gene encoding said protein.

The cell of the present invention may be derived from a T cell that has been obtained from any organism. However, it is preferred that the cell of the present invention is derived from a human or murine T cell. Thus, a particular embodiment of the invention relates to the cell according to the invention, wherein the isolated cell is derived from a human or murine T cell. In certain embodiments, the invention relates to a cell according to the invention, wherein the isolated cell is derived from a human T cell. Thus, the cell may be derived from any human T cell. A particular embodiment of the invention relates to the cell according to the invention, wherein the isolated cell is derived from a Jurkat T cell.

The Jurkat cell line is an immortalized T lymphocyte cell line that was originally obtained from the peripheral blood of a boy with T cell leukemia. The Jurkat cell line has most often been used as a prototypical T cell line to study multiple events in T cell biology, including a) T cell signaling and b) molecular events in the HIV infection life cycle.

A particular embodiment of the invention relates to the cell according to the invention, wherein the cell further comprises a co-receptor.

That is, the cell may further comprise a co-receptor that supports a T cell receptor in the recognition of a peptide-MHC complex on a target cell. However, the invention also encompasses cells that do not comprise a co-receptor. Such cells may be used, for example, for the discovery and engineering of co-receptor independent TCRs.

As used herein, the term "co-receptor" refers to a cell surface molecule that binds to a peptide-MHC complex together with a TCR and potentiates T cell activation. Examples of co-receptors include, without limitation, CD4 and CD8. Thus, in a particular embodiment, the invention relates to the cell according to the invention, wherein the co-receptor is CD4 or CD8.

As used herein, the term "CD4" refers to "cluster of differentiation 4", a co-receptor for TCR, or functional portion thereof. The term encompasses wild type CD4 and genetically engineered CD4 (e.g., a chimeric CD4 comprising a first portion from a CD4 of a first species and a second portion from a CD4 of a second species).

As used herein, the term "CD8" refers to "cluster of differentiation 8", a co-receptor for TCR, or functional portion thereof. Examples of CD8 include, but are not limited to, a CD8 alpha/CD8 beta heterodimer, a CD8 alpha/CD8 alpha homodimer, and a CD8 beta/CD8 beta homodimer. The term encompasses wild type CD8 and genetically engineered CD8 (e.g., a chimeric CD8 comprising a chimeric CD8 chain which includes a first portion from a CD8 of a first species and a second portion from a CD8 of a second species).

In a particular embodiment, the invention relates to the cell according to the invention, wherein the co-receptor is encoded by genes that are operably linked to their corresponding promoter or to a recombinant promoter.

Within the present invention, the co-receptors may be expressed from endogenous genes encoding the co-receptors or from a recombinant polynucleotide that has been introduced into the cell according to the invention. Consequently, the genes or recombinant polynucleotides encoding the co-receptors may be operably linked to their corresponding promoters or may be linked to a recombinant promoter that is not naturally associated with the gene encoding the co-receptor.

For example, in certain embodiments, the cell may be derived from a CD8+ T cell that expresses the co-receptor CD8 from its endogenous genes that are under the control of their corresponding promoter(s). In other embodiments, the cell may be derived from a CD4+ T cell that expresses the co-receptor CD4 from its endogenous genes that are under the control of their corresponding promoter(s). In further embodiments, the cell may be derived from a CD4+ or CD8+ T cell in which the expression of the genes encoding the endogenous CD4 or CD8 co-receptor has been disrupted, respectively. These cells may be used for the discovery and engineering of co-receptor independent TCRs. Alternatively, a recombinant polynucleotide encoding a co-receptor, either under the control of its corresponding promoter or under the control of a recombinant promoter, may be introduced into these cells to allow expression of a desired co-receptor. In certain embodiments, the recombinant co-receptor may be introduced into the CCR5 locus.

Within the present invention, an endogenous gene is a naturally-occurring gene that can be found in a given cell at a known position in the chromosome. A recombinant gene, on the other hand, is a gene that has been introduced into a cell by methods of genetic engineering. An endogenous gene and a recombinant gene may have identical DNA sequences and may only differ in their position in the chromosome.

The genes or recombinant polynucleotides encoding the co-receptor may be under the control of the corresponding promoter of the co-receptor or under the control of a recombinant promoter. A "corresponding promoter" is a promoter that is naturally found in combination with the respective co-receptor genes. A "recombinant promoter" on the other hand, is a promoter that is not naturally found in connection with the respective co-receptor genes.

As used herein, the term "promoter" refers to a regulatory region of DNA that is in operable linkage with a nucleotide sequence, e.g., a protein coding sequence. The promoter contains specific DNA sequences and response elements that are recognized by proteins known as transcription factors. These factors bind to and recruit RNA polymerase II to the promoter thereby facilitating transcription of the downstream nucleotide sequence. The transcribed mRNA comprising a protein coding sequence is then translated to produce the expressed protein.

As used herein, the term "operable linkage" refers to the configuration between a promoter sequence and a protein coding sequence that allows for the transcription of the protein coding sequence in a host cell.

The term "encoding" or "encoded" refers to the inherent property of specific sequences of nucleotides in a polynucleotide, such as a gene, a cDNA, or an RNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription of the gene into mRNA and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

In a particular embodiment, the invention relates to the cell according to the invention, wherein the recombinant promoter is a constitutive promoter.

Within the present application, the promoter may be any promoter. In particular, the promoter may be a constitutive or a regulatable promoter. However, it is preferred that the promoter is a constitutive promoter.

Exemplary embodiments of constitutive promoters include, but are not limited to, viral promoters from polyoma, adenovirus, herpes simplex virus, cytomegalovirus (CMV) and simian virus 40 (SV40) or mammalian promoters such as EF1a, PGK1, Ubc, human beta actin and CAG. In an exemplary configuration, the protein coding sequences are flanked upstream (i.e., 5') by the PGK1 promoter and downstream (i.e., 3') by an SV40 poly(A) signal. Thus, in a preferred embodiment, the invention relates to a cell according to the invention, wherein the constitutive promoter is preferably a PGK1 promoter. In certain embodiments, the constitutive promoter can be T cell- specific, e.g., a CD35 T cell-specific promoter.

Suitable regulatable expression systems should have, e.g., the following properties: a low level of basal expression in the non-induced state; inducers that do not promote pleiotropic effects; high levels of expression in the induced state; highly specific induction of expression of a candidate nucleic acid of interest; and modulation of the level of induced expression. Examples of regulatable expression systems having such properties include, but are not limited to: a Tet inducible system; an FK506/rapamycin inducible system; an RU486/mifepristone inducible system; a cumate inducible system or an ecdysone inducible system. Many constitutive, tissue-specific and inducible promoters are now commercially available from vendors such as Origene, Promega, Invitrogen, System Biosciences and Invivogen.

In a particular embodiment, the invention relates to the cell according to the invention, wherein the one or more reporter system comprises a polynucleotide comprising a promoter region operably linked to a polynucleotide sequence encoding a detectable marker.

The cell according to the invention further comprises one or more reporter systems. In certain embodiments, the one or more reporter system provides an indication of one or more activities of the cell selected from the group consisting of nuclear factor of activated T-cells (NFAT) signaling, activator protein-1 (AP-1) signaling, NFKB/Rel signaling, and calcium flux. In certain embodiments, the one or more reporter system comprises a polynucleotide comprising a promoter region operably linked to a polynucleotide sequence encoding a detectable marker.

In a particular embodiment, the invention relates to the cell according to the invention, wherein the promoter region comprises one or more transcriptional response elements.

The one or more reporter system of the cell according to the invention may comprise a promoter region comprising any transcriptional response element. In certain embodiments, the promoter region comprises one or more transcriptional response elements selected from the group consisting of nuclear factor of activated T-cells (NFAT) response elements, activator protein-1 (AP-1) response elements, and NFKB/Rel response elements. In a preferred embodiment, the invention relates to the cell according to the invention, wherein at least one of the one or more transcriptional response elements is the nuclear factor of activated T cells (NFAT) response element. In an even more preferred embodiment, the invention relates to the cell according to the invention, wherein the promoter region comprises 3 NFAT response elements.

As used herein, the term "promoter region" refers to a sequence of DNA, usually upstream (5') to the coding sequence, which controls the expression of the coding region by providing the recognition for RNA polymerase and/or other factors required for transcription to start at the correct site.

A "transcriptional response element" (TRE) is a polynucleotide sequence, preferably a DNA sequence, which regulates transcription of an operably linked polynucleotide sequence in a host cell that allows that TRE to function. A TRE may comprise an enhancer, a promoter, a silencer, an operator, and the like.

"Nuclear factor of activated T-cells" (NFAT) is a family of transcription factors shown to be important in the immune response. One or more members of the NFAT family is expressed in most cells of the immune system. NFAT is also involved in the development of cardiac, skeletal muscle, and nervous systems. NFAT was first discovered as an activator for the transcription of interleukin-2 in T cells, as a regulator for T cell immune response, but has since been found to play an important role in regulating many other body systems. NFAT transcription factors are involved in many physiological processes as well as in the development of several diseases, such as inflammatory bowel disease and several types of cancer. Under resting conditions, NFAT transcription factors are fully phosphorylated and reside in the cytoplasm. In the context of T cell activation, TCR engagement with target peptide-MHC leads to a localized influx of calcium ions that activates calcineurin, a calcium-dependent phosphatase. Dephosphorylation of NFAT by calcineurin then leads to NFAT translocation into the nucleus and subsequent transcription of NFAT-controlled genes. Thus, NFAT transcription factor activity can be used as a measure of early TCR signalling.

The NFAT-inducible promoter may be any transcriptional promoter comprising an NFAT response element, i.e. a promoter that is active in the presence of dephosphorylated NFAT and essentially inactive in the absence of dephosphorylated NFAT. The promoter may be any promoter functional in mammalian cells, comprising one or several copies of an NFAT binding site or of a composite NFAT:AP-1 binding element. More preferably, the promoter comprises one or several (e.g. 2, 3, 4, 5, 6, 7 or 8) copies of the following sequence: 5'-GGAGGAAAAACTGTTTCATACAGAAGGCGT-3' (SEQ ID NO:1) or any derivative or variant thereof. Derivatives include fragments, as well as mutated, modified or deleted sequences, which can be produced by conventional methods known to the skilled artisan. The capacity of said variants/derivatives to confer NFAT-responsiveness to a promoter may be verified by conventional expression technique.

In certain embodiments, the promoter region is an IL-2 promoter. In certain embodiments, the IL-2 promoter is a minimal IL-2 promoter. In certain embodiments, the IL-2 promoter or minimal IL-2 promoter comprises one or more NFAT binding sites. In certain embodiments, the IL-2 promoter or minimal IL-2 promoter comprises two, three, four or five NFAT binding sites.

In a particular embodiment, the invention relates to the cell according to the invention, wherein the detectable marker is a fluorescent protein.

The reporter system comprised in the cell according to the invention may comprise any detectable marker. However, in certain embodiments, the detectable marker is selected from the group consisting of a fluorescent protein, a cell surface marker, and an antibiotic selection marker.

The term "detectable marker" as used herein refers to a gene product that is suitable for screening, sorting and/or enriching the cells expressing the reporter system of the present invention. The gene product may be any polypeptide or protein suitable for the intended use for screening technologies and can be cytoplasmic, membrane-bound or secreted. Examples of useful enzymes include e.g. beta-lactamase, beta-galactosidase or alkaline phosphatase. Examples of fluorescent agents include green, red, far-red, blue or yellow fluorescent proteins. Examples of luminescent agents include e.g. luciferase proteins (such as firefly, *Renilla, Gaussia* luciferase). Examples of cell surface markers include molecules from the cluster of differentiation (CD), artificial epitopes or membrane-bound proteins from agents mentioned above such as fluorescent proteins. The gene product may be truncated or a fusion protein. In a preferred embodiment, the reporter gene may be a fluorescent agent, such as green fluorescent protein (GFP) or red fluorescent protein (RFP), and the screening, sorting and/or enriching of the cells expressing the reporter system may be performed by means of fluorescent cell sorting, for instance cell sorting by flow cytometry such as fluorescence activated cell sorting (FACS). For FACS, the fluorescent protein such as GFP or RFP may be either cytosolic or membrane-bound. If the screening technology is a magnetic cell sorting technology such as MACS, then the reporter protein has to be membrane-bound (resulting in a cell surface marker) to be suitable for sorting by this technique.

In a particular embodiment, the invention relates to the cell according to the invention, wherein the detectable marker is a fluorescent protein.

Within the present invention, the reporter system may comprise a green fluorescent protein. The term "green fluorescent protein" is used broadly herein to refer to a protein that fluoresces green light, for example, *Aequorea* GFP. GFPs have been isolated from the Pacific Northwest jellyfish, *Aequorea victoria,* the sea pansy, *Renilla reniformis,* and *Phialidium gregarium* (Ward et al., Photochem. Photobiol. 35:803-808, 1982; Levine et al., Comp. Biochem. Physiol. 72BJ7-85, 1982). Modified variants of green fluorescent protein, e.g., EGFP, EBFP, EYFP, d2EGFP, ECFP, GFPuv are included within the term green fluorescent protein. EGFP is particularly preferred.

In certain embodiments, the promoter region is an inducible promoter comprising three NFAT:AP-1 composite binding elements, and the detectable marker is enhanced green fluorescent protein (EGFP). Further, the reporter system may comprise a downstream (i.e., 3') SV40 poly(A) signal.

In a particular embodiment, the invention relates to the cell according to the invention, wherein expression and/or activity of a receptor involved in programmed cell death or T cell exhaustion is reduced or disrupted.

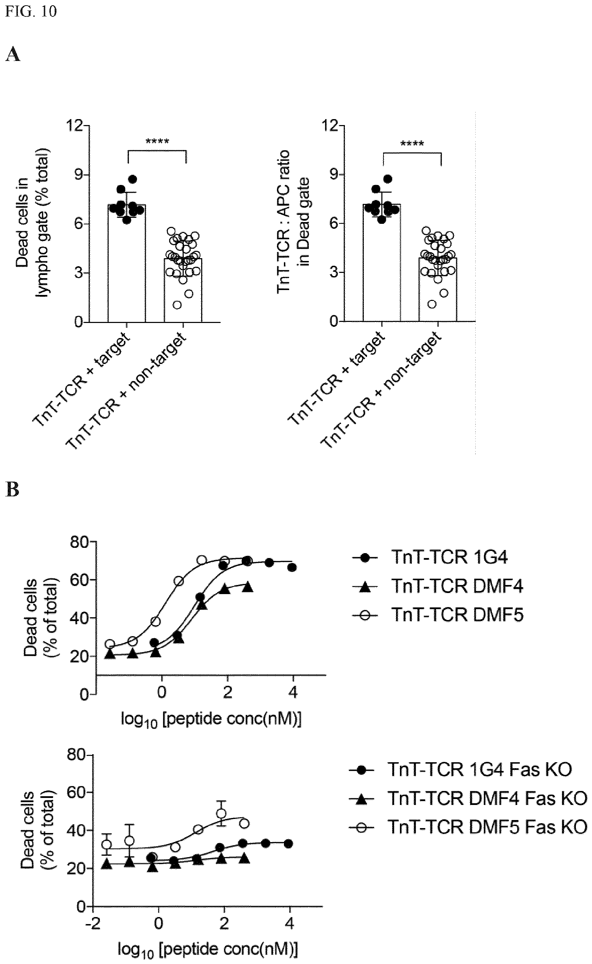

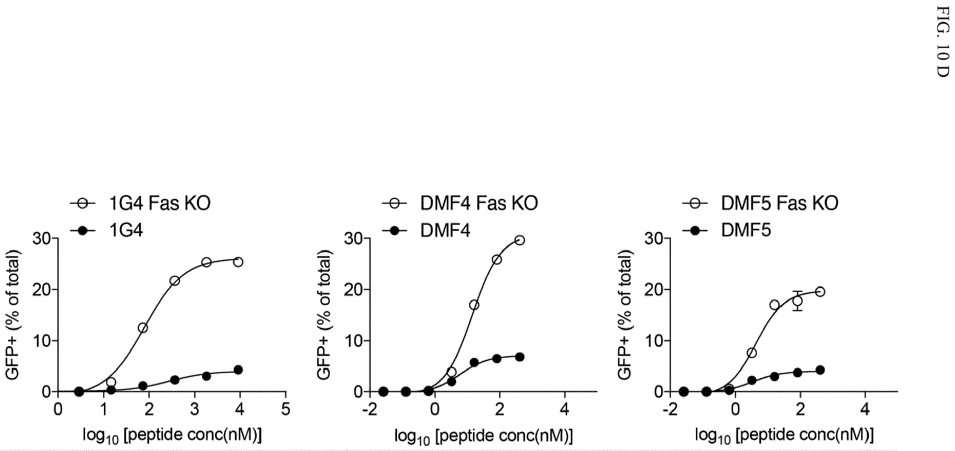

It has been found within the present invention, that expression of a T cell receptor in the cell according to the invention and subsequent stimulation with an antigen results in a high proportion of dead cells. To overcome this limitation, it was found within the present invention that disrupting the expression of at least one gene encoding a receptor involved in programmed cell death or T cell exhaustion results in a decreased proportion of dead cells. Example 7 shows that deletion of the gene encoding the Fas receptor in the cell line of the present invention significantly reduces the proportion of dead cells upon stimulation with an antigen compared to a similar cell line without the deletion (FIG. 10A & B). Further, it has been found that deletion of the gene encoding the Fas receptor in the cell line of the present invention results in a stronger signal and thus in a higher sensitivity of the reporter system (FIG. 10 D).

In a particular embodiment, the invention relates to the cell according to the invention, wherein the receptor involved in programmed cell death or T cell exhaustion is a Fas receptor, TNFR2 and/or PD-1. Preferably, the receptor involved in programmed cell death or T cell exhaustion is a Fas receptor.

Within the present invention, a "receptor involved in programmed cell death or T cell exhaustion" broadly encompasses any receptor that, upon stimulation, may directly or indirectly result in programmed cell death or T cell exhaustion.

Programmed cell death (or PCD) refers to the death of a cell in any form, mediated by an intracellular program, and is also referred to as Cellular Suicide or apoptosis. PCD is carried out in a biological process, which usually confers advantage during an organism's life-cycle. Activation-induced cell death (AICD) is programmed cell death caused by the interaction of Fas receptors (Fas, CD95) and Fas ligands (FasL, CD95 ligand). AICD is a negative regulator of activated T lymphocytes that results from repeated stimulation of their T-cell receptors (TCR) and helps to maintain peripheral immune tolerance. The AICD effector cell is one that expresses FasL, and apoptosis is induced in the cell expressing the Fas receptor. Both activated T cells and B cells express Fas and undergo clonal deletion by the AICD mechanism. Activated T cells that express both Fas and FasL may be killed by themselves or by each other.

As used herein, the term "T cell exhaustion" refers to a state of T cell dysfunction. The T cell exhaustion generally arises during many chronic infections and cancer. T cell exhaustion can be defined by poor effector function, sustained expression of inhibitory receptors, and/or a transcriptional state distinct from that of functional effector or memory T cells. T cell exhaustion generally prevents optimal control of infection and tumors and, as in the present application, may result in reduced expression of the at least one reporter system.

Accordingly, the receptor involved in programmed cell death or T cell exhaustion is preferably a Fas receptor. The Fas receptor or FasR, also known as apoptosis antigen 1 (APO-1 or APT), cluster of differentiation 95 (CD95) or tumor necrosis factor receptor superfamily member 6 (TNFRSF6), is a protein that in humans is encoded by the FAS gene. The Fas receptor is a death receptor on the surface of cells that leads to programmed cell death (apoptosis). It is one of the two primary apoptosis pathways, the other being the mitochondrial pathway.

Alternatively, or in addition to Fas, the receptor involved in programmed cell death or T cell exhaustion may be tumor necrosis factor receptor 2 (TNFR2). TNFR2, also known as tumor necrosis factor receptor superfamily member 1B (TNFRSF1B) and CD120b, is a membrane receptor that binds tumor necrosis factor-alpha (TNFα). Alternatively, or in addition to Fas and/or TNFR2, the receptor involved in programmed cell death or T cell exhaustion may be PD-1. The term "PD-1 " refers to the programmed death-1 protein, a T-cell co-inhibitor, also known as CD279.

That is, the expression and/or activity of one or more receptors involved in programmed cell death or T cell exhaustion may be reduced or disrupted in the cell according to the invention. Further receptors that may be involved in programmed cell death or T cell exhaustion are Tim-3, Lag-3, CD39 and CTLA-4.

The expression of a gene encoding a receptor is said to be reduced if the cell that shows reduced expression of the gene produces lower amounts of the receptor compared to a cell in which the expression of the gene is not reduced. The activity of a receptor is said to be reduced if stimulation of the receptor with an effector results in a lower response of the cell comprising the receptor compared to a cell wherein the activity of the receptor is not reduced, given that both cells express similar amounts of the receptor.

The expression of a gene is said to be disrupted if a cell produces no or undetectable amounts of the gene product. The activity of a receptor is said to be disrupted if stimulation of the receptor with an effector results in no or undetectable response of the cell comprising the receptor, even though if a disrupted variant of the receptor is produced by the cell.

The skilled person is aware of methods to reduce or disrupt the expression of a gene or the activity of a receptor. For example, the expression of a gene encoding a receptor may be reduced or disrupted by modifying regulatory elements or the coding sequence of the gene encoding the receptor. Alternatively, the expression of a gene encoding a receptor may be reduced or disrupted by gene knockdown. The activity of a receptor may be reduced or disrupted by modifying the coding sequence of the gene encoding the receptor or by contacting the cells with a molecule that inhibits the receptor.

Within the present invention, it is preferred that the expression of the Fas receptor is disrupted by modifying the gene encoding the Fas receptor. More preferably, the gene encoding the Fas receptor is modified by Cas9-induced non-homologous end joining.

In a particular embodiment, the invention relates to the cell according to the invention, wherein expression and/or activity of at least one protein involved in antigen presentation is reduced or disrupted. Preferably, the protein involved in antigen presentation is beta-2 microglobulin.

That is, the invention further encompasses a cell wherein the expression and/or activity of at least one protein involved in antigen presentation is reduced or disrupted. As mentioned above, the cell according to the invention may be used for the discovery and engineering of T cell receptors. Without being bound to theory, the presentation of antigens on the cell according to the invention itself may result in the activation of other cells expressing a T cell receptor, thereby potentially creating noise and impeding T cell receptor screening approaches. Therefore, it is desired to facilitate the screening of T cell receptors by using cells according to the invention that expresses no or only reduced amounts of proteins involved in antigen presentation.

Within the present invention, the expression and/or activity of any protein involved in antigen presentation may be reduced or disrupted. In mammals, antigens are commonly presented by major histocompatibility complexes (MHC). Thus, the protein involved in antigen presentation may be any protein that is part of a major histocompatibility complex. MHC class I molecules are heterodimers that consist of two polypeptide chains, the α chain and β2-microglobulin (b2m). The two chains are linked non-covalently via interaction of b2m and the α3 domain within the α chain. Of note, the MHC I α chain is tethered to the cell membrane, while b2m has no linkage to the cell membrane and assembles with the α chain in the endoplasmic reticulum. Like MHC class I molecules, MHC class II molecules are also heterodimers, but in this case consist of two membrane-tethered polypeptides, an α chain and a β chain, both of which are encoded in the MHC locus. The subdesignation α1, α2, etc. refers to separate domains within HLA genes; each domain is usually encoded by a different exon within the gene, and some genes have further domains that encode leader sequences, transmembrane sequences, etc. With the exception of b2m, these molecules are composed of extracellular regions, transmembrane sequences and cytoplasmic tails. In addition to MHC molecules, proteins mediating the transport of target peptides into the endoplasmic reticulum are crucial for antigen presentation. One such protein is the transporter associated with antigen processing (TAP), a heterodimer encoded by the TAP1 and TAP2 genes. Of note, the genes encoding TAP polypeptides reside within the MHC genomic locus.

In certain embodiments, the presentation of antigens by MHC class I molecules on the cell according to the invention may be reduced or prevented by reducing or disrupting the expression or activity of b2m. Without being bound to theory, MHC class I molecules are mainly responsible for the presentation of intracellular antigens on the cell surface. Thus, reducing or disrupting the expression and/or activity of b2m may prevent the presentation of self-antigens which may bias screening results. In other embodiments, the presentation of antigens by MHC molecules on the cell according to the invention may be reduced or prevented by reducing or disrupting the expression and/or activity of individual HLA genes and/or genes encoding TAP.

The skilled person is aware of methods to reduce or disrupt the expression of a gene and/or the activity of a protein involved in antigen presentation. For example, the expression of a gene encoding a protein involved in antigen presentation may be reduced or disrupted by modifying regulatory elements or the coding sequence of the gene encoding the protein involved in antigen presentation. Alternatively, the expression of a gene encoding a protein involved in antigen presentation may be reduced or disrupted by gene knockdown. The activity of a protein involved in antigen presentation may be reduced or disrupted by modifying the coding sequence of the gene encoding the protein involved in antigen presentation.

In a particular embodiment, the invention relates to the cell according to the invention, wherein expression and/or activity of at least one protein promoting non-homologous end joining is reduced or disrupted. Preferably, the protein promoting non-homologous end joining is p53 or p53-binding protein 1.

The cell according to the invention is designed for the discovery and engineering of T cell receptors. It is preferred that DNA fragments encoding entire T cell receptors or parts of T cell receptors are introduced into the cell according to the invention and integrated into the genome by homology-directed repair of a double-strand break. If a double-strand break is detected in the genome of a cell, the double-strand break is most commonly either repaired by homology-directed repair, which would result in the integration of the DNA fragment, or by non-homologous end joining, which would not result in the integration of the DNA fragment. If the frequency of non-homologous end joining can be reduced by reducing or disrupting the expression and/or activity of proteins promoting non-homologous end joining, it is plausible that the frequency of homology-directed repair is increased, and consequently, the discovery and engineering of T cell receptors may be improved.

The skilled person is aware of modifications that result in increased frequency of homology-directed repair. For example, the frequency of homology-directed repair may be increased by reducing or disrupting the expression and/or activity of proteins that are promoting non-homologous end-joining. Proteins promoting non-homologous end joining may be, without limitation, p53 or p53-binding protein 1.

The tumor suppressor protein p53, called the "Guardian of the Genome," controls several central cellular tumor suppressor pathways, including cell cycle arrest, apoptosis (programmed cell death), and cellular senescence, in response to genomic damage and cellular or environmental stress. About half of all human cancers carry mutant p53, making p53 the most commonly mutated protein in human cancers. Most p53 cancer mutants exhibit a single missense mutation, which results in full-length p53 protein with a single amino acid change. The tumor suppressor protein p53 (TP53) plays a central role in directing cellular responses to DNA damage. Tumor protein 53-binding protein 1 (TP53BP1) binds to TP53 and has a potential role in DNA damage responses. Without being bound to theory, 53BP1 restricts homology-directed repair and promotes non-homologous end joining.

The skilled person is aware of methods to reduce or disrupt the expression of a gene or the activity of a protein promoting non-homologous end joining. For example, the expression of a gene encoding a protein promoting non-homologous end joining may be reduced or disrupted by modifying regulatory elements or the coding sequence of the gene encoding the protein promoting non-homologous end joining. Alternatively, the expression of a gene encoding a protein promoting non-homologous end joining may be reduced or disrupted by gene knockdown. The activity of a protein promoting non-homologous end joining may be reduced or disrupted by modifying the coding sequence of the gene encoding the protein promoting non-homologous end joining.

In a particular embodiment, the invention relates to the cell according to the invention, wherein expression and/or activity of at least one protein promoting homology directed repair is increased. Preferably, the protein promoting homology directed repair is BRCA1.

Instead of reducing the frequency of non-homologous end joining as discussed above, the frequency with which exogenous DNA fragments are integrated into the genome of a cell by homology directed repair may also be increased by increasing the expression and/or activity of at least one protein promoting homology directed repair. The skilled person is aware of methods to increase the expression of a gene and/or the activity of a protein in a cell. For example, the activity of a protein promoting homology directed repair may be increased by modifying the coding sequence of the gene encoding a protein promoting homology directed repair such that the activity of the gene product is increased compared to the unmodified variant. The expression of a gene encoding the protein promoting homology directed repair may be increased by modifying regulatory elements or the coding sequence of the gene encoding the protein promoting homology directed repair such that the expression of said protein is increased. For example, the promoter region of the gene encoding the protein promoting homology directed repair may be replaced by a promoter region that causes higher production of the protein in the cell according to the invention compared to a cell wherein the gene encoding the protein promoting homology directed repair is under control of its endogenous promoter region. Alternatively or in addition, the expression of the protein promoting homology directed repair may be increased by increasing the copy number of the gene encoding the protein promoting homology directed repair. For example, a gene or coding sequence encoding the protein promoting homology directed repair may be introduced into the cell according to the invention. The gene or coding sequence encoding the protein promoting homology directed repair may be coupled to its endogenous promoter or to a recombinant promoter. The skilled person is aware of methods to modify genomic DNA and of methods to introduce exogenous DNA into a cell.

The protein promoting homology directed repair may be any protein that is involved in the process of homology directed repair. Preferably, the protein promoting homology directed repair is BRCA1. The term "BRCA1", as used herein, refers to the human BRCA1 protein and to proteins substantially identical thereto. BRCA1 is a human tumor suppressor gene (also known as a caretaker gene) and is responsible for repairing DNA. BRCA1 is part of a protein complex that repairs DNA when both strands are broken. When this happens, it is difficult for the repair mechanism to "know" how to replace the correct DNA sequence, and there are multiple ways to attempt the repair. The double-strand repair mechanism in which BRCA1 participates is homology-directed repair, where the repair proteins copy the identical sequence from the intact sister chromatid.

In a particular embodiment, the invention relates to the cell according to the invention, wherein the cell comprises a gene encoding a recombinant endonuclease.

Within the present invention, it is intended that DNA fragments encoding T cell receptors are integrated into the cell according to the invention by homology-directed repair of a double strand break in the genome of the cell. Said double strand break may be introduced into the cell according to the invention by any method known in the art. For example, double-strand breaks may be introduced into the genome of the cell using an endonuclease.

Endonucleases are enzymes that cleave the phosphodiester bond within a polynucleotide chain. Some, such as deoxyribonuclease I, cut DNA relatively nonspecifically (without regard to sequence), while many, typically called restriction endonucleases or restriction enzymes, cleave only at very specific nucleotide sequences. Endonucleases differ from exonucleases, which cleave the ends of recognition sequences instead of the middle (endo) portion. Within the present invention, it is preferred that the endonuclease is sequence specific. More preferably, the endonuclease introduces a double strand break in the TCR alpha or beta gene locus. Even more preferably, the endonuclease is a programmable endonuclease such as a zinc finger nuclease, a TALEN or a CRISPR nuclease such as Cas9 or Cpf1. The term "programmable" refers to the ability to engineer nuclease-based platforms for recognizing various target sites (i.e. target specificity) in the genome.

The endonuclease may be introduced into the cell according to the invention in any way known in the art. However, it is preferred that the endonuclease is expressed by the cell according to the invention. For that, the cell according to the invention may comprise a polynucleotide encoding the endonuclease. For example, the polynucleotide encoding the endonuclease may be integrated into the genome of the cell according to the invention or may be part of a linear or circular DNA element. However, it is preferred that the polynucleotide encoding the endonuclease is integrated into the genome of the cell according to the invention. The skilled person is aware of methods to introduce polynucleotides encoding recombinant proteins into the cell according to the invention.

In a particular embodiment, the invention relates to the cell according to the invention, wherein the recombinant nuclease is Cas9.

It is preferred that the cell according to the invention comprises a polynucleotide encoding a CRISPR nuclease, such as Cas9 or Cpf1, or any derivative thereof.

The term "Cas9" refers to an enzyme that exhibits at least endonuclease activity (e.g. cleaving the phosphodiester bond within a polynucleotide) guided by a CRISPR RNA (crRNA) bearing complementary sequence to a target polynucleotide. Cas9 polypeptides are known in the art, and include Cas9 polypeptides from any of a variety of biological sources, including, e.g., prokaryotic sources such as bacteria and archaea. Bacterial Cas9 includes Actinobacteria (e.g., *Actinomyces naeslundii*) Cas9, Aquificae Cas9, Bacteroidetes Cas9, Chlamydiae Cas9, Chloroflexi Cas9, Cyanobacteria Cas9, Elusimicrobia Cas9, Fibrobacteres Cas9, Firmicutes Cas9 (e.g., *Streptococcus pyogenes* Cas9, *Streptococcus thermophilus* Cas9, *Listeria innocua* Cas9, *Streptococcus agalactiae* Cas9, *Streptococcus mutans* Cas9, and *Enterococcus faecium* Cas9), Fusobacteria Cas9, Proteobacteria (e.g., *Neisseria meningitides, Campylobacter jejuni*) Cas9, Spirochaetes (e.g., *Treponema denticola*) Cas9, and the like. Archaea Cas 9 includes Euryarchaeota Cas9 (e.g., *Methanococcus inaripaludis* Cas9) and the like. A variety of Cas9 polypeptides are known, and are reviewed in, e.g., Makarova et al. (2011) Nature Reviews Microbiology 9:467-477, Makarova et al. (2011) Biology Direct 6:38, Haft et al. (2005) PLOS Computational Biology 1:e60 and Chylinski et al. (2013) RNA Biology 10:726-737. The term "Cas9" includes a Cas9 polypeptide of any Cas9 family, including any isoform of Cas9.

The term "Cas9" as used herein encompasses wild type Cas9, e.g. a polypeptide comprising an amino acid sequence having at least about 75%, at least about 80%, at least about 85%, at least about 90%, at least about 95%, at least about 98%, at least about 99%, or 100% amino acid sequence identity to a Cas9 polypeptide from *Streptococcus pyogenes* (SEQ ID NO. 2) and having endonuclease activity. Preferably, the cell according to the invention comprises a polynucleotide encoding Cas9 from *Streptococcus pyogenes* (SEQ ID NO. 2). In certain embodiments, the Cas9 is a Cas9 from *Streptococcus pyogenes,* wherein the cysteine residue at position 80 is substituted with a tyrosine residue.

It is intended that the endonuclease is active in the nucleus of the cell according to the invention. Thus, it is preferred that the endonuclease is coupled to a nuclear localization sequence, that directs the endonuclease to the nucleus of the cell. The skilled person is aware of methods and amino acid sequences that direct proteins to the nucleus of a cell.

The polynucleotide encoding the endonuclease may be operably linked to any promoter and, optionally, to any further regulatory element. That is, the polynucleotide encoding the endonuclease may be operably linked to a constitutive or a regulatable promoter and, optionally, further regulatory elements. In certain embodiments, the polynucleotide encoding the endonuclease may be operably linked to a chicken-β-actin (CBA) promoter. In certain embodiments, the gene encoding the endonuclease may further be operably linked to a cytomegalovirus (CMV) enhancer and/or a 5' hybrid intron.

The polynucleotide encoding Cas9 may be introduced into the cell according to the invention by any method known in the art. That is, the polynucleotide encoding Cas9 may be introduced into the cell according to the invention by biological or physical means, such as transformation, transfection, transduction, electroporation, magnetofection, lipofection and the like.

It is preferred that the polynucleotide encoding the endonuclease, preferably Cas9, is integrated into the genome of the cell according to the invention. The skilled person is aware of methods to integrate exogenous polynucleotides into the genome of a cell. In certain embodiments, the polynucleotide encoding the endonuclease is integrated into the genome of the cell according to the invention by homology-directed repair. In certain embodiments, the polynucleotide encoding the endonuclease is integrated into the CCR5 locus of the cell according to the invention. In certain embodiments, the polynucleotide encoding the endonuclease is comprised in a polynucleotide further comprising one or more polynucleotide(s) encoding a selection marker, such as a fluorescent protein or an antibiotic selection marker.

In a particular embodiment, the invention relates to a method for producing a cell expressing a TCR-CD3 complex on the cell surface, the method comprising the steps of: (a) providing a cell according to the invention; (b) introducing a DNA fragment comprising coding sequences encoding a TCR alpha variable domain, a TCR beta variable domain and at least one of a TCR alpha constant domain and/or a TCR beta constant domain into the genome of the cell of step (a); (c) expressing the coding sequences encoded on the DNA fragment introduced in step (b); and (d) obtaining a cell expressing a TCR-CD3 complex on the cell surface.

The cell according to the invention may be used as a platform for the discovery and engineering of T cell receptors. Thus, the cells may be used in a method for producing cells that express a TCR-CD3 complex on the cell surface. The cell according to the invention can be distinguished from previous TCR discovery platforms (WO 2017/044672) in that the cell according to the invention does not express CD3 on the cell surface due to the modification of the TCR alpha or beta gene locus. As a consequence, the restoration of a functional TCR-CD3 complex can serve as a readout to identify cells that successfully obtained a DNA fragment comprising coding sequences encoding parts of a T cell receptor. Accordingly, cells according to the invention that have integrated a DNA fragment and thus express a functional T cell receptor can be readily identified and distinguished with the help of an anti-CD3 and/or an anti-TCR antibody from cells that have not obtained a DNA fragment.

The cell according to the invention does not express a functional TCR due to a modification in the TCR alpha and/or beta gene locus. To express a functional TCR in these cells, it is required to overcome this modification by introducing a DNA fragment comprising coding sequences encoding a TCR alpha variable domain, a TCR beta variable domain and at least one of a TCR alpha constant domain and/or a TCR beta constant domain into the genome of these cells. That is, in certain embodiments, a DNA fragment comprising the coding sequences encoding a TCR alpha variable domain, a TCR beta variable domain, a TCR alpha constant domain and a TCR beta constant domain may be integrated into the genome of the cell according to the invention at any position.

Alternatively, a DNA fragment comprising coding sequences encoding a TCR alpha variable domain, a TCR beta variable domain and one of a TCR alpha constant domain or a TCR beta constant domain may be integrated into the genome of the cell according to the invention. In this case, the missing TCR alpha or beta constant domain that is required for the formation of a functional TCR is expressed from the endogenous TCR alpha or beta constant domain gene in the genome of the cell according to the invention.

The term "coding sequence", as used herein, refers to nucleotide sequences or nucleic acid sequences (including both RNA or DNA) that encode genetic information for the synthesis of a whole RNA, a whole protein, or any portion of such whole RNA or whole protein. Within the present invention, the coding sequence is preferably a DNA sequence that encodes a component of a T cell receptor. It is preferred that a coding sequence encoding a particular component of a T cell receptor is contiguous, i.e. not disrupted by introns. However, coding sequences that comprise introns are also within the scope of the invention.

Within the present invention, a DNA fragment is a polynucleotide. Preferably, the DNA fragment is a linear, double-stranded DNA molecule such as a PCR product or a restriction fragment obtained from a plasmid. However, in certain embodiments, the DNA fragment may be a single-stranded DNA molecule that is obtained from a double-stranded DNA template or that is generated by oligonucleotide synthesis. In certain embodiments, DNA fragments may include one or more phosphorothioate bonds in their structure to inhibit exonuclease degradation.

In a particular embodiment, the invention relates to the method according to the invention, wherein the DNA fragment of step (b) further comprises homology arms at its 5' and 3' ends that are homologous to the endogenous TCR alpha gene locus or the endogenous TCR beta gene locus of the cell.

Within the present invention, it is preferred that the DNA fragment comprising the coding sequences encoding a TCR alpha variable domain, a TCR beta variable domain and one of a TCR alpha constant domain or a TCR beta constant domain is integrated into the TCR alpha or beta gene locus of the cell according to the invention by means of homologous recombination. For that, the DNA fragment preferably comprises homology arms at its 5' and 3' ends that are homologous to the endogenous TCR alpha gene locus or the endogenous TCR beta gene locus of the cell and thus facilitate genome integration of the DNA fragment into the TCR alpha or beta gene locus.

It is preferred that DNA fragments comprising a coding sequence encoding the TCR alpha constant domain integrate into the TCR beta gene locus and that DNA fragments comprising a coding sequence encoding the TCR beta constant domain integrate into the TCR alpha gene locus.

The term "homology arm" refers to a portion of a nucleic acid molecule having a nucleotide sequence that enables homologous recombination between two nucleic acids that have highly similar nucleotide sequences in discrete regions. The preferred size range of the homology arm is from about 10 to about 1000 nucleotides in length, more preferably from about 500 to about 1000 nucleotides in length. Within the present invention, the two nucleic acids may be the DNA fragment comprising the TCR coding sequences and the genome of the cell according to the invention.

The "5' end" is that end of a DNA fragment which terminates in a 5' phosphate group and the "3' end" is that end of a DNA fragment which terminates in a 3' phosphate or hydroxyl group.

In a particular embodiment, the invention relates to the method according to the invention, wherein the DNA fragment further comprises a splice donor site that is located downstream of the most 3' coding sequence of the DNA fragment and upstream of the 3' homology arm.

That is, the DNA fragment preferably comprises a splice donor site between the most 3' protein coding sequence of the DNA fragment and the 3' homology arm. When transcribed into an mRNA, the splice donor site can recognize a splice acceptor site downstream on the same mRNA, which is required for splicing of the mRNA.

As used herein, the term "splice donor site" is intended to refer to any individual functional splice donor or functional splice donor consensus sequence that permits the DNA fragment of the invention to be processed such that it is included in any mature, biologically active mRNA, provided that it is integrated in an active chromosomal locus and transcribed as a contiguous part of the pre-messenger RNA of the chromosomal locus. An example of splice donor consensus sequence for mammalian cells is 5'-GTRAGT-3'.

As used herein, the term "upstream" of any particular point reference refers to the region occurring 5' of that reference point. If no point of reference is given, "upstream" is meant to be interpreted taking as reference the 5' to 3' direction of transcription of the DNA or RNA in question. As used herein, the term "downstream" of any particular reference point is intended to refer to the region occurring 3' to that particular reference point. If no point of reference is given, "downstream" is meant to be interpreted taking as reference the 5' to 3' direction of transcription of the DNA or RNA in question.

In a particular embodiment, the invention relates to the method according to the invention, wherein the DNA fragment is introduced into the genome of the cell such that the coding sequences located on the DNA fragment replace the endogenous TCR alpha VJ exon or the TCR beta VDJ exon and are transcriptionally linked to the endogenous gene encoding the TCR alpha constant domain or the TCR beta constant domain, respectively.

As mentioned above, the DNA fragment may be introduced into the genome of the cell according to the invention at any position. However, it is preferred that the DNA fragment replaces the endogenous TCR alpha VJ exon or the TCR beta VDJ exon. More preferably, the DNA fragment may be introduced into the genome of the cell such that the coding sequences located on the DNA fragment are transcriptionally linked to the endogenous TCR alpha constant domain gene or to the TCR beta constant domain gene, respectively.

Thus, in certain embodiments, the DNA fragment comprises coding sequences encoding a TCR alpha variable domain, a TCR alpha constant domain and a TCR beta variable domain, preferably in this order in a 5' to 3' direction. In addition, this fragment may comprise a splice donor site downstream of the 3' coding sequence encoding the TCR beta variable domain. Further, the DNA fragment may comprise 5' and 3' homology arms that are homologous to the TCR beta gene locus, preferably such that the DNA fragment, when integrated into the genome of the cell according to the invention, replaces the TCR beta VDJ exon and such that the coding sequences comprised in the DNA fragment will be, after integration into the genome, transcriptionally linked to the endogenous gene encoding the TCR beta constant domain. In certain embodiments, the 5' homology arm that allows replacing the TCR beta VDJ exon has the polynucleotide sequence 5'-GCATGCCTCTGTGCCAACAGCAAAAATGTGCCCTTCTCTTTTGTTGGCAAGTAACTT AACCAACCAACCCAAAAAAAAGATCTTTCTCTCAGCTTTCCATAATCTCTGAGACGA AGTAGGTTTGGAGAAGTGGGGTTACAGGGGAAAAAGCCAGGTGTTAATGATGAAAA AACATTGAACTTTTCTAGGGGTAGTAATAAGATTTAATTCAAGACTAGAACATTTTA GCTGCAAATCTTCAAGAATAAGACAATATTATCCCCTTTCTGTTTTATTGTGGGACTA GAGAATGTGAGAGAGGTTACATTCCATGGGCTTTGGGAATTTAATATGGTTCAAGGA TAAACACACCCAGGTTTTTCACTGCAGAGAAGAGCTTCAAATATAATCAGTTTTCAG GTCATCAGCTCAGCTCTTGTATCCCTAACAATGCAGTTGACATGCGTCTTCTCAGATG TCTAACTCCTAACTCACTGAGGGATACTTTAAGTACATATAAAGGACTAGAAGCACC AAGCTACCAGTGAGAGGAAGAGGAGAGTTTGCAGAGAAGCTGGCTTGAAATAAGA CAATGAGTTCATCTTTAAATACTTGCCATTTGAGGTGCAGATGGATATAGTTGGCAG GCTCCTATGTAAGGCATGTTATGGAGAAGCTACCGTGAATTGATAATATCAAAACAA ATATCCAGGGAGCCTCTGCAAGTGTGCATCTCTATTTCACACCAATTATAGTTGAGT TAATTCCTGCCTGATTCATCTCCCAGAGATGCAGCCTCCTCTTAAAGAAGTTGGGGG TGGTGGCCCATTCAGTGATGTCACTGACAGATGCATTCTGTGGGGATAAAATGTCAC AAAATTCATTTCTTTGCTCATGCTCACAGAGGGCCTGGT-3' (SEQ ID NO.3) and the 3' homology arm that allows replacing the TCR beta VDJ exon has the polynucleotide sequence 5'-AGGAGGGGTGCGATGAGGGAGGACTCTGTCCTGGGAAATGTCAAAGAGAACAGAG ATCCCAGCTCCCGGAGCCAGACTGAGGGAGACGTCATGTCATGTCCCGGGATTGAG TTCAGGGGAGGCTCCCTGTGAGGGCGAATCCACCCAGGCTTCCCAGAGGCTCTGAG CAGTCACAGCTGAGCCCAGGGTGATGGGGCAGAAGAGGGAAGGGGAGGGGGCCTC TCATCATAGTTCCCTGAGATAGCCCAGAGAAAGCCCGGTGGGTAATGAATGAGCCA CAACACCTCTCCATCTATCTGCTTCACTGACAGAGGTTCTCTGTAGATTCTTCGTATA TTCCTGTGCTGGATTTTATAGGAGGCCACTCTGTGTCTCTTTTTGTCACCTGCCTGAG TCTTGGGCAAGCTCTGGAAGGGAACACAGAGTACTGGAAGCAGAGCTGCTGTCCCT GTGAGGGAAGAGTTCCCATGAACTCCCAACCTCTGCCTGAATCCCAGCTGTGCTCAG CAGAGACTGGGGGGTTTTGAAGTGGCCCTGGGAGGCTGTGCTCTGGAAACACCATA TATTTTGGAGAGGGAAGTTGGCTCACTGTTGTAGGTGAGTAAGTCAAGGCTGGACA GCTGGGAACTTGCAAAAAGGGGCTGGAATCCAGACGGAGCCTTTGTCTCTAGTGCTT AGGTGAAAGTGTATTTTTGTCAGGAAGGCCTATGAGGCAGATGAGGAGGGGATAGC CTCCCTCTCCTCTCCACTATTTTGTAGACTGCCTGTGCCAAGTTAGGTTCCCCTACTG AGAGATGGGTAGACTCAGCTTGGAAGGGGTCACCTTGAACATCTCCTGTCTCCTTGA AGGGTGCCGGTCACGGCCATGACAGATAAAA-3' (SEQ ID NO.4).

In certain embodiments, The DNA fragment comprises coding sequences encoding a TCR beta variable domain, a TCR beta constant domain and a TCR alpha variable domain, preferably in this order in a 5' to 3' direction. In addition, this fragment may comprise a splice donor site downstream of the 3' coding sequence encoding the TCR alpha variable domain. Further, the DNA fragment may comprise 5' and 3' homology arms that are homologous to the TCR alpha gene locus, preferably such that the DNA fragment, when integrated into the genome of the cell according to the invention, replaces the TCR alpha VJ exon and such that the coding sequences comprised in the DNA fragment will be, after integration into the genome, transcriptionally linked to the endogenous gene encoding the TCR alpha constant domain.

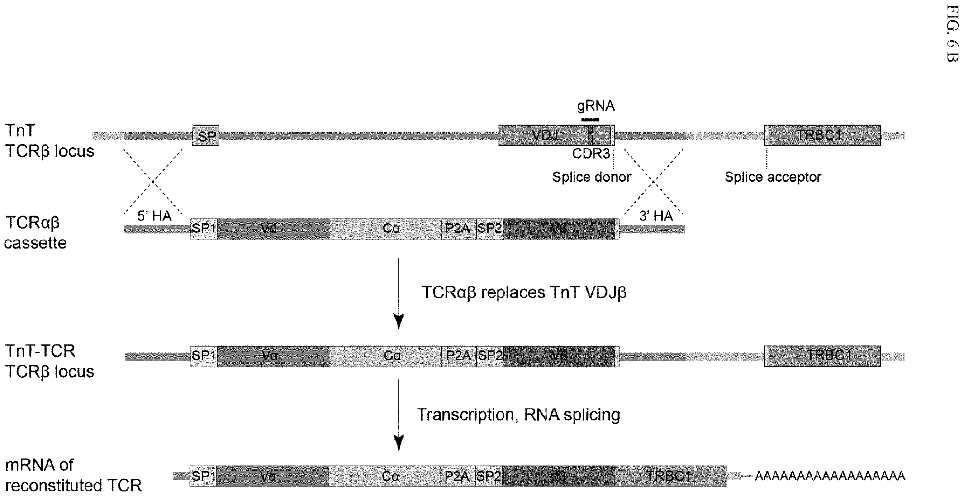

The term "transcriptionally linked", as used herein, means that the coding sequences comprised in the DNA fragment and the gene downstream of the recombination site, i.e., the endogenous TCR alpha or beta constant domain gene, are, after successful integration of the DNA fragment into the genome, transcribed as a single mRNA (see for example Figure 6B). Preferably, the coding sequences comprised in the DNA fragment and the endogenous gene downstream of the recombination site are, after successful integration of the DNA fragment into the genome, transcriptionally linked such that the transcribed mRNA encodes a TCR alpha variable domain, a TCR alpha constant domain, a TCR beta variable domain and a TCR beta constant domain.

In certain embodiments, the splice donor site downstream of the 3' coding sequence of the DNA fragment, e.g., the coding sequence encoding the TCR alpha variable domain, will recognize the splice acceptor site upstream of the endogenous TCR alpha constant domain gene located downstream of the recombination site, such that a mature mRNA encoding a functional TCR alpha chain can be formed. In certain embodiments, the splice donor site downstream of the 3' coding sequence of the DNA fragment, e.g., the coding sequence encoding the TCR beta variable domain, will recognize the splice acceptor site upstream of the endogenous TCR beta constant domain gene located downstream of the recombination site, such that a mature mRNA encoding a functional TCR beta chain can be formed.

The "TCR alpha VJ exon", as used herein, refers to the part of the TCR alpha gene locus that encompasses a variable (V) alpha gene segment and a joining (J) alpha gene segment that are joined as a result of V(D)J recombination. The "TCR beta VDJ exon", as used herein, refers to the part of the TCR beta gene locus that encompasses a variable (V) beta gene segment, a diversity (D) beta gene segments and a joining (J) beta gene segment that are joined as a result of V(D)J recombination.

Further the DNA fragment may comprise one or more coding sequences encoding a signal peptide, preferably located upstream of the polynucleotides encoding the TCR alpha or beta variable domains. In certain embodiments, the signal peptide is a signal peptide that is naturally associated with T cell receptors. However, the signal peptide may also be a synthetic signal peptide or may be a signal peptide from another secreted mammalian protein. In addition, the DNA fragment may encode a self processing peptide that is located between the coding sequences encoding the TCR alpha chain and the TCR beta chain to allow for the separation of the TCR alpha and the TCR beta polypeptides during translation of the mRNA. In certain embodiments, the self-processing peptide is the 2A peptide derived from the porcine teschovirus-1 polyprotein (SEQ ID NO. 5). In certain embodiments, the DNA fragment may further comprise a detectable marker.

In a particular embodiment, the invention relates to the method according to the invention, wherein the DNA fragment is introduced into the genome of the cell through homologous recombination, homology-directed repair, homology-independent targeted insertion or microhomology-mediated end joining.

The DNA fragment may be introduced into the genome of the cell according to the invention by any method known in the art. However, it is preferred that the DNA fragment is introduced into the genome of the cell according to the invention in a targeted manner, more preferably such that the DNA fragment is integrated into the TCR alpha or beta gene locus of the cell. The skilled person is aware of methods for targeted introduction of DNA fragments into the genome of a cell. For example, the DNA fragment may be introduced into the genome of the cell according to the invention by homologous recombination, homology-directed repair, homology-independent targeted insertion or microhomology-mediated end joining.

The term "homologous recombination" as used herein refers to the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency.

Homology-directed repair (HDR) is a mechanism in cells to repair double-stranded and single stranded DNA breaks. Homology-directed repair includes homologous recombination (HR) and single-strand annealing (SSA) (Lieber. (2010) Annu. Rev. Biochem. 79: 181-211). The most common form of HDR is called homologous recombination (HR), which has the longest sequence homology requirements between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks.

Homology-independent targeted insertion (HITI) is a CRISPR/Cas9-based strategy that enables gene insertion in non-dividing cells *in vivo* and *in vitro.* Site-specific transgene integration can be achieved by the homology-directed repair (HDR) pathway such as short-fragment homologous recombination (SFHR). This process is inefficient and the process is not active in non-dividing cells. The other major double strand break (DSB) repair pathway, non-homologous end joining (NHEJ), is active in both proliferating and non-dividing cells and is generally more efficient than HDR in mammals. Whereas HDR can replace the target sequence, NHEJ can add an ectopic DNA sequence at the target locus instead of replacing the original DNA sequence. However, NHEJ is recognized as error-prone.

Microhomology-mediated end joining (MMEJ) is an alternative NHEJ pathway that repairs DSB in a manner similar to SSA but potentially at robustness comparable to NHEJ. MMEJ repairs DSBs by apposing regions of local microhomology (3-25 bp in length) on both sides of the DSB, which are then used as templates for DNA elongation. The most likely outcome of MMEJ is stereotyped deletion mutant generation in the absence of an exogenous donor. However, insertion and chromosomal rearrangement events are common with MMEJ. In spite of these possible outcomes, MMEJ has been successfully deployed for precise integration of DNA fragments into the genome.

In a particular embodiment, the invention relates to the method according to the invention, wherein the DNA fragment is introduced into the genome of the cell through CRISPR-Cas9-mediated homology-directed repair.

Within the present invention, it is preferred that the DNA fragment is integrated into the genome of the cell according to the invention by CRISPR-Cas9-mediated homology directed repair. CRISPR-Cas9-mediated homology-directed repair is characterized as homology directed repair of a DNA double strand break that has been introduced into the genome of a cell via the CRISPR/Cas9 method.

The skilled person is aware of methods to introduce exogenous DNA fragments into the genome of a cell by CRISPR-Cas9-mediated homology-directed repair. Targeted integration of exogenous DNA fragments with this method requires the targeted induction of a DNA double strand break in the genome of the cell using the endonuclease Cas9 and the introduction of an exogenous DNA fragment with regions that are homologous to regions upstream and downstream of the double strand break in the genome into the same cell.

Within the present invention, the DNA fragment is preferably introduced into the TCR alpha or beta gene locus. Thus, in certain embodiments, a DNA double strand break is introduced into the TCR alpha or beta gene locus using the endonuclease Cas9. The skilled person is aware of methods to introduce a DNA double strand break in the TCR alpha or beta gene locus. For example, the sequence specificity of the endonuclease Cas9 may be determined by the sequence of a CRISPR RNA or crRNA. In certain embodiments, a crRNA is designed to target the TCR alpha or beta gene locus, preferably the VJ exon of the TCR alpha gene locus or the VDJ exon of the TCR beta gene locus. In certain embodiments, a crRNA is designed to target the endogenous Jurkat TCRβ complementarity determining region 3 and has the sequence 5'-TCGACCTGTTCGGCTAACTA-3' (SEQ ID NO: 6). The skilled person is aware of methods to introduce a crRNA into a cell. For example, a crRNA may be introduced into the cell either alone, or together with a tracrRNA, by transfection.

The endonuclease Cas9 may be introduced into the cell by any method known in the art, for example by directly introducing Cas9 protein into the cell or by introducing a polynucleotide encoding Cas9 into the cell. Within the present invention, it is preferred that Cas9 is expressed within the cell from a polynucleotide that has been integrated into the genome of the cell. The polynucleotide encoding Cas9 as defined above may be introduced into the cell by any method known in the art, preferably by transfection. In some embodiments, the rates of Cas9-mediated homology-directed repair may be enhanced by the addition of chemicals into the cell culture media (e.g., HDR enhancer from Integrated DNA Technologies) and/or by adjusting the length of 5' and/or 3' homology arms so that the exogenous DNA fragment is asymmetric (Richardson, C.D., et al. (2016) Nature biotechnology 34.3: 339).

In a particular embodiment, the invention relates to a library of DNA fragments, wherein the library comprises DNA fragments comprising a coding sequence encoding a TCR alpha variable domain, a coding sequence encoding a TCR beta variable domain and at least one of a coding sequence encoding a TCR alpha constant domain and/or a coding sequence encoding a TCR beta constant domain.

That is, the application further relates to a library of DNA fragments that may be used to identify T cell receptors with the cell according to the invention. To allow identification of T cell receptors with the cell according to the invention, at least one element of the library, e.g. one DNA fragment, has to comprise a coding sequence encoding a TCR alpha variable domain, a coding sequence encoding a TCR beta variable domain and at least one of a coding sequence encoding a TCR alpha constant domain and/or a coding sequence encoding a TCR beta constant domain. However, it is preferred that at least 20%, 30%, 40%, 50%, 60%, 70%, 80%, 90, 95%, 99% or 100% of the DNA fragments in the library comprise a coding sequence encoding a TCR alpha variable domain, a coding sequence encoding a TCR beta variable domain and at least one of a coding sequence encoding a TCR alpha constant domain and/or a coding sequence encoding a TCR beta constant domain.

Within the present invention, it is preferred that the DNA fragments in the library comprise a coding sequence encoding a TCR alpha variable domain, a coding sequence encoding a TCR beta variable domain and a coding sequence encoding a TCR alpha constant domain when intending to introduce the DNA fragments into the genome of the cell according to the invention such that the coding sequences encoded on the DNA fragment are transcriptionally linked to the endogenous TCR beta constant domain gene. Alternatively, the DNA fragments in the library may comprise a coding sequence encoding a TCR alpha variable domain, a coding sequence encoding a TCR beta variable domain and a coding sequence encoding a TCR beta constant domain when intending to introduce the DNA fragments into the genome of the cell according to the invention such that the coding sequences encoded on the DNA fragment are transcriptionally linked to the endogenous TCR alpha constant domain gene.

Besides the coding sequence encoding a TCR alpha variable domain, the coding sequence encoding a TCR beta variable domain and at least one of the coding sequences encoding a TCR alpha constant domain and/or TCR beta constant domain, the DNA fragments of the library may comprise additional features. As disclosed in other parts of this application, the DNA fragments of the library may preferably comprise coding sequences for signal peptides and/or self-processing peptides, splice donor sites and/or homology arms. More preferably, the DNA fragments of the library may comprise 5' and 3' homology arms that enable homologous recombination of the DNA fragment with the TCR alpha or beta gene locus, even more preferably such that the coding sequences encoded on the DNA fragment are transcriptionally linked to the TCR alpha or beta constant domain gene, respectively.

Within the present invention, the DNA fragments of the library are preferably linear, double-stranded DNA molecules. However, in certain embodiments, the DNA fragment may be linear, single-stranded DNA molecules. Double-stranded or single-stranded DNA fragments may include one or more phosphorothioate bonds in their structure to inhibit exonuclease degradation. Alternatively, the DNA fragments of the library may also be comprised in circular polynucleotides, such as vectors. These circular polynucleotides may in turn be comprised in a plurality of cells or virus particles.

The term "library", as used herein, refers to a diverse collection or mixture of DNA fragments comprising coding sequences encoding different recombinant polypeptides. The size and complexity of the libraries to be used in the methods of the present invention may be varied. For example, the methods of the invention may be used to screen libraries with only several tens or hundreds of different elements, or libraries with several million or more elements. Within the present invention, an element of the library is a DNA fragment, preferably a DNA fragment comprising coding sequences encoding a TCR alpha variable domain, a TCR beta variable domain and at least one TCR alpha constant domain and/or TCR beta constant domain.

In a particular embodiment, the invention relates to the library of DNA fragments according to the invention, wherein the coding sequences encoding the TCR alpha variable domain, the TCR beta variable domain and at least one of the TCR alpha constant domain and/or the TCR beta constant domain have been obtained by sequencing a population of T cells. Preferably, the population of T cells comprises tumor-infiltrating lymphocytes.

That is, the coding sequences comprised in the DNA fragments of the library may be obtained by sequencing polynucleotides encoding TCRs in a population of T cells. The skilled person is aware of methods for sequencing polynucleotides encoding TCRs. For example, the sequence of a polynucleotide encoding a TCR may be obtained by sequencing of genomic DNA, in particular, genomic DNA comprising the TCR alpha and beta gene locus. Several methods for sequencing genomic DNA are known in the art. Further, bioinformatic tools are known in the art that enable the identification of coding sequences encoding the TCR alpha and beta chain from the obtained sequences.

Preferably, the coding sequences comprised in the DNA fragments of the library may be obtained by sequencing of cDNAs encoding TCRs. For that, mature mRNAs are obtained from a T cell and reverse transcribed into cDNA, which is then sequenced by methods known in the art. For the generation of the library according to the invention, it is preferred that mature mRNAs are obtained from a population of T cells, reverse transcribed into cDNA and sequenced by massive parallel sequencing. An example for a method to generate a library of DNA fragments based on a population of T cells is given in Example 9.

The coding sequences comprised in the DNA fragments of the library may have been obtained from any T cell or population of T cells. That is, the T cell may be an isolated T cell line or a T cell that has been obtained from a patient. Preferably, the coding sequences comprised in the DNA fragments of the library have been obtained from a population of T cells that have been obtained from a patient. More preferably, the population of T cells may comprise tumor infiltrating lymphocytes that have been obtained from a patient. A coding sequence is said to be obtained by sequencing a T cell, if the coding sequence corresponds to a polynucleotide sequence that can be found in said T cell. Preferably, a coding sequence that has been obtained by sequencing a T cell corresponds in sequence to an mRNA comprised in said T cell.

The term "population" of cells refers to more than one cell of the same cell type. For example, the term "population of T cells" refers to more than one T cell.

Tumor-infiltrating lymphocytes (TILs) are lymphoid cells that infiltrate solid tumors. T cells found within TILs often appear naturally reactive to autologous tumor antigens. Thus, TILs are an attractive source for TCRs and may facilitate the identification of TCRs that specifically recognize tumor cells in a patient. The skilled person is aware of methods to obtain TILs from a subject.

In a particular embodiment, the invention relates to the library of DNA fragments according to the invention, wherein the coding sequences encoding the TCR alpha and beta variable domains comprised in at least one DNA fragment of the library have been obtained from the same T cell.

Within the present invention, the coding sequences comprised in a single DNA fragment of the library may be obtained from the same or from different T cells. In certain embodiments, at least one DNA fragment of the library comprises coding sequences encoding a TCR alpha variable domain and a TCR beta variable domain that have been obtained from the same T cell. In certain embodiments, at least one DNA fragment of the library comprises coding sequences encoding a TCR alpha variable domain, a TCR beta variable domain and at least one of a TCR alpha constant domain and/or a TCR beta constant domain, wherein all coding sequences have been obtained from the same T cell. Preferably, more than one DNA fragment of the library comprises coding sequences encoding a TCR alpha variable domain and a TCR beta variable domain that have been obtained from the same T cell. For example, 20%, 30%, 40%, 50%, 60%, 70%, 80%, 90, 95%, 99% or 100% of the DNA fragments in the library may comprise coding sequences encoding a TCR alpha variable domain and a TCR beta variable domain, and optionally a TCR alpha and/or beta constant domain, that have been obtained from the same T cell.

Obtaining the coding sequences of TCR alpha and beta chains from the same T cell may be achieved by single cell sequencing. For that, it is preferred that the mRNAs from single T cells are reverse transcribed into cDNA and barcoded. The barcoded cDNAs from all T cells in a population may then be sequenced and the coding sequences encoding the naturally occurring TCR alpha/beta chain pairs from the same cell may be identified according to the barcode by bioinformatic methods known in the art. An example for the sequencing of TCR genes in a T cell population is given in Example 9.

The skilled person is aware of methods to produce libraries of DNA fragments comprising coding sequences obtained from a population of T cells. Preferably, the DNA fragments of the library are designed *in silico* based on the sequencing results and synthesized chemically. Methods for analyzing sequencing results and designing libraries, as well as methods for synthesizing DNA are well known in the art.

In a particular embodiment, the invention relates to the library of DNA fragments according to the invention, wherein at least one DNA fragment in the library comprises one or more mutations in at least one coding sequence encoding a TCR alpha variable domain, a TCR beta variable domain and at least one of a TCR alpha constant domain and/or a TCR beta constant domain compared to a DNA fragment with a known sequence.

The present invention also encompasses libraries of DNA fragments comprising coding sequences encoding a TCR alpha variable domain, a TCR beta variable domain and at least one of a TCR alpha constant domain and/or a TCR beta constant domain, wherein at least one DNA fragment of the library comprises one or more mutations in at least one coding sequence of the DNA fragment. Preferably, more than one DNA fragment of the library comprises coding sequences, wherein one or more mutations have been introduced into at least one coding sequence of the DNA fragment. For example, 20%, 30%, 40%, 50%, 60%, 70%, 80%, 90, 95%, 99% or 100% of the DNA fragments in the library may comprise coding sequences, wherein one or more mutations have been introduced into at least one coding sequence of the DNA fragment.

The library of DNA fragments comprising the one or more mutations is based on a DNA fragment with a known nucleotide sequence comprising coding sequences encoding a TCR alpha variable domain, a TCR beta variable domain and at least one of a TCR alpha constant domain and/or a TCR beta constant domain. This "DNA fragment with a known sequence" may comprise coding sequences that have been obtained from a naturally occurring T cell receptor by cloning or sequencing or coding sequences from an engineered T cell receptor that has been generated by means of genetic engineering. The "DNA fragment with a known sequence" may be comprised in the library or may not be comprised in the library.

In general, a DNA sequence is said to comprise one or more mutations, if the DNA fragment is highly similar in sequence compared to a DNA fragment with a known sequence, but comprises one or more functional or non-functional nucleic acid changes, insertions or deletions. However, if the mutation is a deletion or insertion, it is preferred that the deletion or insertion does not change the reading frame of the coding sequence comprised in the DNA fragment.

The skilled person is aware of methods to generate libraries of DNA fragments comprising one or more mutations. For example, the library according to the invention may be generated by means of genetic engineering starting from a DNA fragment with a known sequence that serves as a template. Numerous methods of genetic engineering are known in the art that may be used to introduce one or more mutations into DNA fragments in a random or targeted manner. The production of libraries generated by means of genetic engineering preferably involves polymerase chain reaction (PCR). Thus, when random mutations are introduced into a DNA fragment, an error-prone polymerase may be used or the PCR conditions may be altered such that the fidelity of a polymerase is decreased. Targeted mutations in the DNA fragment may be introduced by adding oligonucleotides or polynucleotides with the desired sequence variation during the PCR reaction. Alternatively, a library of DNA fragments comprising one or more mutations may be designed *in silico* and synthesized by means of chemical DNA synthesis.

In a particular embodiment, the invention relates to the library of DNA fragments according to the invention, wherein the one or more mutations have been introduced into the coding sequences encoding complementarity determining regions (CDRs) of the variable domains of the T cell receptor. Preferably, the one or more mutations have been introduced into the coding sequence encoding the CDR3 of the alpha or beta variable domain of the T cell receptor.

Within the present invention, the one or more mutations may be introduced into any part of the coding sequences encoding the components of a T cell receptor. However, it is intended that the mutations result in altered recognition of a target or non-target antigen. In a T cell receptor, the binding of an antigen is mainly determined by the T cell receptor variable domains and, more specifically, by the complementary determining regions (CDRs) of the variable domains. Thus, within the present invention, it is preferred that the one or more mutations are introduced into a coding sequence encoding the alpha or beta variable domain of a T cell receptor and, more preferably, into the coding sequences encoding the CDRs of a T cell receptor.

Without being bound to theory, the CDR3 of the TCR beta chain is heavily involved in recognition of antigens and thus a preferred target for TCR engineering. Accordingly, within the present invention, it is even more preferred that the one or more mutations are introduced into the coding sequences encoding the CDR3 of the TCR alpha and/or beta variable domains. Most preferably, the one or more mutations are introduced into the coding sequence encoding the CDR3 of the TCR beta variable domains.

The term "complementarity determining region" (CDR), as used herein, refers to amino acid sequences which together define the binding affinity and specificity of the natural variable binding region of a native immunoglobulin binding site, a T cell receptor, or a synthetic polypeptide which mimics this function.

In a particular embodiment, the invention relates to the library of DNA fragments according to the invention, wherein the one or more mutations have been introduced into the DNA fragments by deep mutational scanning.

As mentioned above, the one or mutations may be introduced into the DNA fragment by any method known in the art. In certain embodiments, the one or more mutations may be introduced into the DNA fragment by deep mutational scanning (DMS). In certain embodiments, DMS may be performed by generating single-position saturation mutagenesis using a plasmid-based approach as described by Wrenbeck et al., Nature methods 13, 928-930 or as exemplified in Example 8 of the present invention.

In certain embodiments, the template or "DNA fragment with a known sequence" is a DNA fragment comprising coding sequences encoding the TCR alpha variable domain, the TCR beta variable domain and at least one of the TCR alpha constant domain and/or the TCR beta constant domain of a naturally occurring or genetically engineered anti-MAGE-A3 TCR. In certain embodiments, the one or more mutations may be introduced into a DNA fragment comprising coding sequences encoding a naturally occurring or genetically engineered anti-MAGE-A3 TCR. In certain embodiments, the one or more mutations may be introduced into a coding sequence encoding a TCR alpha or beta variable domain of a naturally occurring or genetically engineered anti-MAGE-A3 TCR. In certain embodiments, the one or more mutations may be introduced into a coding sequence encoding a CDR of a naturally occurring or genetically engineered anti-MAGE-A3 TCR. In certain embodiments, the one or more mutations may be introduced into a coding sequence encoding the CDR3 of the TCR beta chain of a naturally occurring or genetically engineered anti-MAGE-A3 TCR. In certain embodiments, each codon of a coding sequence encoding the CDR3 of the TCR beta chain of a naturally occurring or genetically engineered anti-MAGE-A3 TCR may be substituted with a degenerate NNK codon, respectively. In certain embodiments, the TCR beta chain of the naturally occurring or genetically engineered anti-MAGE-A3 TCR comprises the amino acid sequence CASSPNMADEQYF (SEQ ID NO. 7). Alternatively, the coding sequence encoding the TCR beta chain of the naturally occurring or genetically engineered anti-MAGE-A3 TCR comprises the polynucleotide sequence 5'-TGCGCCAGCAGCCCGAATATGGCGGATGAACAGTACTTC-3' (SEQ ID NO. 8).

In a particular embodiment, the invention relates to a method for determining the potential of a cell expressing a T cell receptor to bind to and/or to be activated by an antigen, the method comprising the steps of: (a) producing a cell expressing a T cell receptor using the method according to the invention; (b) contacting the cell of step (a) with an antigen; and (c) determining the potential of the cell to bind to the antigen and/or to be activated by the antigen.

That is, the cell according to the invention may be used in a method that allows to determine the potential of a T cell receptor to bind to an antigen and/or to be stimulated by an antigen. Due to the optimized cell of the present invention and the targeted integration of exogenous TCR genes into the genome of the host cell, the present invention allows more accurate and reliable characterization of T cell receptors than previous methods known in the art.

The main advantage of the method according to the invention compared to previous methods for the discovery and engineering of T cell receptors (e.g., the method disclosed in WO 2017/044672) is that the DNA fragment encoding the TCR genes is integrated into a predetermined locus of the cell according to the invention and not randomly by means of viral gene delivery. Only due to this targeted integration of the DNA fragment can it be assured that all cells that have integrated the DNA fragment express similar amounts of T cell receptors on the cell surface, which results in a lower variation when measuring antigen-binding and antigen-induced signaling of the TCR and, thus, in more reliable results compared to previous methods.

For determining the potential of a T cell receptor to bind to or to or be stimulated by an antigen, first, a cell expressing said T cell receptor may be produced as described herein by introducing a DNA fragment encoding a TCR alpha variable domain, a TCR beta variable domain and at least one of a TCR alpha constant domain and/or a TCR beta constant domain into a cell according to the invention. The resulting cell may then be contacted with an antigen and the potential of the cell to bind to the antigen or to be activated by the antigen may be determined.

The term "contacted" as used in the inventive method refers to any situation in which two or more molecules or biological entities, such as cells or antigens, are brought into intimate physical contact with one another, e.g. form part of the same aqueous solution.

In a particular embodiment, the invention relates to the method according to the invention, wherein the cell is determined to bind to the antigen, if the binding between the cell and the antigen is stronger compared to the binding between the cell and an irrelevant antigen, or, if the binding between the cell and the antigen is stronger compared to the binding between the antigen and a cell that expresses an irrelevant or no T cell receptor; and/or wherein the cell is determined to be activated by the antigen, if the activation of the cell by the antigen is higher compared to the activation of a cell that has been contacted with an irrelevant or no antigen, or, if the activation of the cell by the antigen is higher compared to the activation of a cell that expresses an irrelevant or no T cell receptor and has been contacted with the antigen.

That is, the cell according to the invention may be used to physically characterize T cell receptors by measuring the binding of an antigen to a T cell receptor and/or to functionally characterize T cell receptors by measuring the activation of a cell expressing a T cell receptor after contacting the cell with an antigen.

Within the present invention, a cell expressing a T cell receptor is said to bind to an antigen, if the binding of the cell expressing the T cell receptor to the antigen is stronger than the binding of a similar cell expressing an identical T cell receptor to an irrelevant antigen. An "irrelevant antigen" as used herein, is an antigen that is known not to bind to or to stimulate a particular T cell receptor. Preferably, the antigen and the irrelevant antigen belong to the same class of molecule. For example, if the antigen is an MHC-bound peptide, the irrelevant antigen may be an MHC-bound peptide that is incapable of binding to or stimulating a different T cell receptor.

Alternatively, a cell expressing a T cell receptor is said to bind to an antigen, if the binding of the cell expressing the T cell receptor to the antigen is stronger compared to the binding between the same antigen and a similar cell expressing an irrelevant or no T cell receptor. In this case, an "irrelevant T cell receptor", as used herein, is a T cell receptor that does not bind or is not stimulated by the antigen.

Within the present invention, a cell expressing a T cell receptor is said to be activated by an antigen, if the activation of the cell expressing the T cell receptor by the antigen is stronger compared to the activation of a similar cell expressing an identical T cell receptor by an irrelevant antigen. Alternatively, a cell expressing a T cell receptor is said to be activated by an antigen, if the activation of the cell expressing the T cell receptor by the antigen is stronger compared to the activation of a similar cell expressing an irrelevant or no T cell receptor by the same antigen.

In a particular embodiment, the invention relates to the method according to the invention, wherein the antigen is part of an antigen library.

That is, the method according to the invention may be used to determine if a T cell receptor can bind to or be stimulated by a particular antigen. However, the method according to the invention may also be used to screen antigen libraries. For that, a plurality of cells expressing a particular T cell receptor on the cell surface may be produced with the method according to the invention. The resulting cells may then be divided into separate cultures and a different antigen may be added to each culture. Accordingly, the potential of each antigen in a library to bind to the T cell receptor or to stimulate the T cell receptor may be determined and the antigens with the highest potential for binding and/or stimulating the T cell receptor may be identified.

In a particular embodiment, the invention relates to a method for identifying at least one T cell receptor with optimized antigen-binding or antigen-induced signaling properties, the method comprising the steps of: (a) producing a plurality of cells using the method according to the invention, wherein the plurality of cells is produced by introducing the library of DNA fragments according to the invention into said cells; (b) contacting the plurality of cells of step (a) with a target antigen or with one or more non-target antigens; (c) isolating at least one cell from the plurality of cells, in which the binding between the cell and the target antigen and/or in which the activation of the cell by the target antigen is increased compared to a cell that expresses a T cell receptor that has been used as starting point for the optimized T cell receptor; and/or, in which the binding between the cell and the non-target antigen and/or in which the activation of the cell by the non-target antigen is decreased compared to a cell that expresses a T cell receptor that has been used as starting point for the optimized T cell receptor; and (d) identifying at least one T cell receptor with optimized antigen-binding or antigen-induced signaling properties by sequencing the DNA fragment comprised in the at least one cell isolated in step (c).

The method according to the invention may be used for engineering T cell receptors. That is, the method according to the invention may preferably be used to identify T cell receptors with optimized antigen-binding and/or antigen-induced signaling properties. Within the present invention, the term "optimized" is to be seen in relation to a known T cell receptor and depends on the antigen. Thus, the term "optimized" may relate to either increased or decreased antigen-binding or antigen-induced signaling properties compared to a known T cell receptor that has been used as starting point for the optimized T cell receptor. For example, if the antigen is a target antigen, the aim is to increase the antigen-binding and/or antigen-induced signaling properties of the T cell receptor for this specific target antigen. In this case, an optimized T cell receptor shows increased antigen-binding and/or antigen-induced signaling properties in response to a target antigen compared to the T cell receptor that has been used as starting point for the optimized T cell receptor. If, on the other hand, the antigen is a non-target antigen, the aim is to decrease the antigen-binding and/or antigen-induced signaling properties of the T cell receptor for this specific non-target antigen, for example to reduce cross-reactivity with an unwanted target. In this case, an optimized T cell receptor shows decreased antigen-binding or antigen-induced signaling properties in response to a non-target antigen compared to the T cell receptor that has been used as starting point for the optimized T cell receptor.

For that, a plurality of cells expressing different T cell receptors on the cell surface may be produced by introducing a library of DNA fragments encoding T cell receptors or parts of T cell receptors into the cell according to the invention. To identify a T cell receptor with optimized properties, it is preferred that the library of DNA fragments is a library according to the invention, wherein one or more mutations have been introduced into at least one coding sequence encoding a TCR alpha variable domain, a TCR beta variable domain or at least one of a TCR alpha constant domain and/or a TCR beta constant domain of a DNA fragment encoding a T cell receptor with a known sequence.

The plurality of cells may then be contacted with a target antigen or with one or multiple non-target antigens. A "target antigen" is an antigen that is intended to be recognized by the optimized T cell receptor that is to be identified with the method according to the invention. It is further intended, that a T cell receptor with optimized antigen-binding or antigen-induced signaling properties binds to or is stimulated more efficiently by the target antigen compared to a T cell receptor with a known sequence that has been used as starting point for the T cell receptor with optimized antigen-binding and/or antigen-induced signaling properties. A "non-target antigen" or "off-target antigen" is an antigen that is not intended to be recognized by the optimized T cell receptor that is to be identified with the method according to the invention. For example, a non-target antigen may be an antigen that is recognized by a T cell receptor due to cross-reactivity. It is further intended that a T cell receptor with optimized antigen-binding or antigen-induced signaling properties binds to or is stimulated less efficiently by the non-target antigen compared to a T cell receptor with a known sequence that has been used as the starting point for the T cell reporter with optimized antigen-binding or antigen-induced signaling properties. Preferably, a T cell receptor with optimized antigen-binding or antigen-induced signaling properties does not bind to and is not stimulated by the non-target antigen.

Cells expressing an optimized T cell receptor may then be isolated from the plurality of cells. Within the present invention, different criteria may be applied to isolate cells expressing a T cell receptor with the desired properties. In case a T cell receptor is optimized for improved binding of a target antigen, cells may be isolated from the plurality of cells that show improved binding of the target antigen compared to cells expressing a T cell receptor with a known sequence that has been used as a starting point or template for the optimized T cell receptor. If a T cell receptor is optimized for increased stimulation by a target antigen, cells may be isolated from the plurality of cells that show increased activation by the target antigen compared to cells expressing a T cell receptor with a known sequence that has been used as a starting point or template for the optimized T cell receptor. In case a T cell receptor is optimized for decreased binding of a non-target antigen, cells may be isolated from the plurality of cells that show reduced binding of the target antigen compared to cells expressing a T cell receptor with a known sequence that has been used as a starting point or template for the optimized T cell receptor. If a T cell receptor is optimized for decreased stimulation by a target antigen, cells may be isolated from the plurality of cells that show reduced activation by the target antigen compared to cells expressing a T cell receptor with a known sequence that has been used as a starting point or template for the optimized T cell receptor. In addition, any combination of the scenarios described above may be applied. In certain embodiments, optimized T cell receptors may be identified by performing massively parallel sequencing of TCR alpha or TCR beta genes in order to compare the frequencies of specific TCRs in cells that are positive relative to cells that are negative for antigen binding and/or antigen-induced signaling.

Within the present invention, a "T cell receptor that has been used as a starting point for the optimized T cell receptor" is a T cell receptor that serves as template for the generation of the T cell receptor library, for example by introducing one or more mutations into the coding sequences encoding the T cell receptor. This T cell receptor may further serve as a reference when measuring the properties of the T cell receptors of the library. Preferably, the nucleotide and amino acid sequences encoding this T cell receptor are known.

Once a cell expressing an optimized T cell receptor has been isolated, the optimized T cell receptor may be identified by sequencing the polynucleotide encoding the T cell receptor. The skilled person is aware of methods for determining the polynucleotide and amino acid sequences of a T cell receptor in an isolated cell.

In certain embodiments, the T cell receptor to be optimized is a T cell receptor that recognizes the MAGE-A3 cancer testis antigen.

A low avidity anti-MAGE-A3 TCR (A3-WT, Vα: METLLGLLILWLQLQWVSSKQEVTQIPAALSVPEGENLVLNCSFTDSAIYNLQWFRQDP GKGLTSLLLIQSSQREQTSGRLNASLDKSSGRSTLYIAASQPGDSATYLCAVRPGGAGSY QLTFGKGTKLSVIP (SEQ ID NO. 9) and Vβ: MGSRLLCWVLLCLLGAGPVKAGVTQTPRYLIKTRGQQVTLSCSPISGHRSVSWYQQTPG QGLQFLFEYFSETQRNKGNFPGRFSGRQFSNSRSEMNVSTLELGDSAL YLCASSPNMAD EQYFGPGTRLTVT (SEQ ID NO. 10)) has been previously isolated from a patient vaccinated with the MAGE-A3 HLA-A*0101-restricted peptide EVDPIGHLY (SEQ ID NO. 11). Importantly, the A3-WT TCR has been previously engineered for high-affinity binding to its target using phage display, resulting in the a3a TCR (Vα: METLLGLLILWLQLQWVSSKQEVTQIPAALSVPEGENLVLNCSFTDSAIYNLQWFRQDP GKGLTSLLLVRPYQREQTSGRLNASLDKSSGRSTLYIAASQPGDSATYLCAVRPGGAGS YQLTFGKGTKLSVIP (SEQ ID NO. 12) and Vβ: MGSRLLCWVLLCLLGAGPVKAGVTQTPRYLIKTRGQQVTLSCSPISGHRSVSWYQQTPG QGLQFLFEYFSETQRNKGNFPGRFSGRQFSNSRSEMNVSTLELGDSAL YLCASSPNMAD EQYFGPGTRLTVT (SEQ ID NO. 13)) (WO 2012/013913). A clinical trial in patients with myeloma and melanoma using the engineered a3a TCR resulted in unexpected and fatal cardiac toxicity in two patients. Cross-reactivity to a peptide derived from Titin (ESDPIVAQY, SEQ ID NO. 14) was identified retrospectively as causal to cardiac toxicity and suggests that high-affinity engineering of A3-WT TCR contributed to this effect.

Accordingly, in certain embodiments, the T cell receptor that has been used as the starting point for the optimized T cell receptor is an anti-MAGE-A3 TCR, in particular the low avidity anti-MAGE-A3 TCR with SEQ IDs NO. 9 and 10. In certain embodiments, the method according to the invention may be used to engineer an anti-MAGE-A3 TCR with increased binding to a target antigen, in particular the MAGE-A3 antigen comprising the peptide EVDPIGHLY (SEQ ID NO. 11). In this case, a TCR with optimized antigen-binding properties may be identified by isolating cells that show increased binding between the cell expressing the optimized TCR and the MAGE-A3 antigen compared to the binding between the MAGE-A3 antigen and a similar cell expressing the anti-MAGE-A3 TCR, in particular the low avidity anti-MAGE-A3 TCR with SEQ IDs NO. 9 and 10.

In certain embodiments, the method according to the invention may be used to engineer the previously engineered anti-MAGE-A3 TCR a3a (SEQ IDs NO. 12 and 13) to decrease the binding to a non-target antigen, in particular wherein the non-target antigen comprises a peptide derived from Titin, such as a peptide with the sequence ESDPIVAQY (SEQ ID NO. 14). In this case, a TCR with optimized antigen-binding properties may be identified by isolating cells with decreased binding between the cell expressing the optimized TCR and the non-target antigen comprising a peptide derived from Titin compared to the binding between the non-target antigen comprising a peptide derived from Titin and a cell expressing the previously engineered anti-MAGE-A3 TCR a3a with SEQ IDs NO. 12 and 13.

In certain embodiments, the method according to the invention may be used to engineer the anti-MAGE-A3 TCR of SEQ IDs NO 9 and 10 to increase the activation of a cell expressing this TCR by a target antigen, in particular the MAGE-A3 antigen comprising the peptide EVDPIGHLY (SEQ ID NO. 11). In this case, a TCR with optimized antigen-induced signaling properties may be identified by isolating cells that show increased activation by the MAGE-A3 antigen comprising the peptide EVDPIGHLY (SEQ ID NO. 11) compared to a cell expressing the anti-MAGE-A3 TCR with SEQ ID NOs 9 and 10.

In certain embodiments, the method according to the invention may be used to engineer the previously engineered anti-MAGE-A3 TCR a3a (SEQ IDs NO. 12 and 13) to reduce the activation of a cell expressing this TCR by a non-target antigen, in particular wherein the non-target antigen is a peptide derived from Titin, such as a peptide with the sequence ESDPIVAQY (SEQ ID NO. 14). In this case, a TCR with optimized antigen-induced signaling properties may be identified by isolating cells that show reduced activation by the peptide derived from Titin compared to a cell expressing the previously engineered anti-MAGE-A3 TC a3aR with SEQ IDs NO. 12 and 13.

In a particular embodiment, the invention relates to the method according to the invention, wherein the at least one identified T cell receptor is included in a training set for a machine learning algorithm to predict T cell receptors with improved antigen-binding or antigen-induced signaling properties.

That is, the optimized T cell receptors that are identified with the method according to the invention may be added to a training set for a machine learning algorithm. Protein engineering through machine-learning-guided directed evolution enables the optimization of protein functions and to predict how sequence influences function in a data-driven manner without requiring a detailed model of the underlying physics, structural features or biological pathways. Such methods accelerate directed evolution by learning from the properties of characterized variants and using that information to select sequences that are likely to exhibit improved properties.

The term "machine learning" as used herein refers to a computer-assisted algorithm used to extract useful information from a database by building probabilistic models in an automated way. The term "training set," as used herein, refers to the set of T cell receptors used to predict a T cell receptor with desired properties by using the machine learning algorithm. The training set of TCRs is preferably chosen from previously identified TCRs with optimized binding or antigen-induced signaling properties for a specific target or non-target antigen.

In a particular embodiment, the invention relates to a method for identifying at least one antigen-specific T cell receptor, the method comprising the steps of: (a) producing a plurality of cells using the method according to the invention, wherein the plurality of cells is produced by introducing the library of DNA fragments according to the invention into said cells; (b) contacting the plurality of cells of step (a) with an antigen; (c) isolating at least one cell from the plurality of cells, in which the binding between the cell and the antigen of step (b) is stronger compared to the binding between the cell and an irrelevant antigen, or, in which the binding between the cell and the antigen is stronger compared to the binding between the antigen and a cell that expresses an irrelevant or no T cell receptor; and/or in which the activation of the cell by the antigen of step (b) is higher compared to the activation of a cell that has been contacted with an irrelevant or no antigen, or, in which the activation of the cell by the antigen is higher compared to the activation of a cell that expresses an irrelevant or no T cell receptor and has been contacted with the antigen; and (d) identifying at least one antigen-specific T cell receptor by sequencing the DNA fragment comprised in the at least one cell isolated in step (c).

The method according to the invention may be used for discovering T cell receptors. That is, the method according to the invention may preferably be used to identify T cell receptors with desired antigen-binding and/or antigen-induced signaling properties. Humans and other mammals have a highly diverse repertoire of T cell receptors. Thus, it still remains challenging to identify T cell receptors with desired characteristics from this pool of T cell receptors.

To overcome this problem, a plurality of cells expressing different T cell receptors on the cell surface may be produced by introducing a library of DNA fragments encoding T cell receptors or parts of T cell receptors into the cell according to the invention. To identify a T cell receptor with the desired properties, it is preferred that the library of DNA fragments is a library according to the invention, wherein the coding sequences encoding the TCR alpha variable domain, the TCR beta variable domain and at least one of the TCR alpha constant domain and/or the TCR beta constant domain have been obtained by sequencing TCRs in a population of T cells, in particular wherein the population of T cells is a population of tumor-infiltrating lymphocytes (TILs).

The plurality of cells may then be contacted with an antigen as described above and cells expressing a T cell receptor with the desired antigen-binding and/or antigen-induced signaling properties may be isolated from the plurality of cells. For that, different criteria may be applied to isolate cells expressing a T cell receptor with the desired antigen-binding and/or antigen-induced signaling properties.

In case a T cell receptor that binds to a specific antigen is to be discovered, cells may be isolated that exhibit stronger binding of the antigen compared to the binding between a cell expressing a similar T cell receptor and an irrelevant antigen. Preferably, cells may be isolated that exhibit stronger binding of an antigen compared to the binding between this antigen and a cell expressing an irrelevant or no T cell receptor. In case a T cell receptor that can be stimulated by an antigen is to be discovered, cells may be isolated that are activated more efficiently by this antigen compared to cells expressing a similar T cell receptor that have been contacted with an irrelevant antigen. Preferably, cells may be isolated that are activated more efficiently by the antigen compared to a cell that has been contacted with the same antigen but expresses an irrelevant or no T cell receptor. Once a cell expressing an antigen-specific T cell receptor has been isolated, the antigen-specific T cell receptor may be identified by sequencing the polynucleotide encoding the T cell receptor. The skilled person is aware of methods for determining the polynucleotide and amino acid sequence of a T cell receptor in an isolated cell.

In a particular embodiment, the invention relates to the method according to the invention, wherein the antigen is presented on the surface of a tumor cell.

That is, the method according to the invention may be used to identify T cell receptors that can recognize tumor cells and thus be used in the treatment of cancer. Thus, in certain embodiments, it is preferred that the antigen that is used for the discovery of T cell receptors is an antigen that is presented on the surface of a tumor cell. An antigen is "presented on the surface of a tumor cell" if it is located on the extracellular side of the cell membrane such that it is accessible to a T cell receptor on the surface of another cell. In certain embodiments, the antigen that is presented on the surface of a tumor cell is a peptide, in particular a peptide that is presented on the surface of a tumor cell by a major histocompatibility complex.

In a particular embodiment, the invention relates to the method according to the invention, wherein the tumor cell and the TCR coding sequences in the library of DNA fragments have been obtained from the same subject.

The method according to the invention may also be used in personalized medicine. Thus, in certain embodiments, it is preferred that both the tumor cell presenting the antigen and the polynucleotide sequences of the TCR genes in the library of DNA fragments have been obtained from the same subject. In a subject suffering from cancer, often only a small fraction of T cells comprise T cell receptors that can recognize the subject's tumor. Preferably, the TCR coding sequences in the library of DNA fragments have been obtained from tumor-infiltrating lymphocytes, since tumor-infiltrating lymphocytes may be more likely to comprise T cell receptors that can recognize a subject's tumor. Accordingly, the method of the present invention allows identification of tumor-reactive T cell receptors, which may then be genetically engineered into the subject's T cells *ex vivo* prior to their re-introduction into the subject. Thus, the method according to the invention may be used to identify T cell receptors that recognize the subject's tumor and that may be used in the treatment of that subject.

In a particular embodiment, the invention relates to the method according to the invention, wherein the antigen or the target antigen is a peptide bound to a major histocompatibility complex (MHC). Preferably, the peptide is presented by an MHC on the surface of an antigen-presenting cell (APC) or by an MHC multimer.

Within the present invention, the antigen comprises a peptide. This peptide may be contacted with the plurality of cells expressing the TCR library in any form. In certain embodiments, a free peptide may be contacted with the plurality of cells. However, without being bound to theory, the binding and stimulation of a TCR also depends on interactions between the TCR and the major histocompatibility complex which presents the peptide on the cell surface. Thus, in a preferred embodiment, the antigen is a peptide that is bound to a major histocompatibility complex. Without being bound to theory, the specificity of a T cell receptor for an antigen is mainly determined by the amino acid sequence of the peptide that is comprised in the peptide-MHC complex. In several instances in this application, it is stated that the antigen is a specific peptide or a peptide in general. However, the skilled person understands that in these instances it is meant that the peptide is a peptide that is comprised in an MHC molecule.

In certain embodiments, the peptide is bound to an MHC molecule that is located on the surface of an antigen-presenting cell. For that, the peptide may be pulsed on the APC or may be a peptide that is genetically encoded by the APC and has been processed within the APC to be presented by the APC. Thus, in a particular embodiment, the invention relates to the method according to the invention, wherein the peptide is pulsed onto the APC or wherein the peptide is genetically encoded by the APC. The skilled person is aware of methods to pulse peptides on an APC. The term "pulsing" as employed herein is intended to refer simply to exposing the relevant cells, such as APCs to a single peptide or a pool of peptides in an appropriate medium. In certain embodiments, the peptide may also be pulsed on an artificial MHC multimer.

The term "peptide" as used herein refers to a series of amino acid residues that are connected to one another typically by peptide bonds between the alpha-amino and carbonyl groups of the adjacent amino acids. The peptides preferably have a length between about 8 and about 15 amino acids.

The term "MHC" as used herein will be understood to refer to the Major Histocompatibility Complex, which is defined as a set of gene loci specifying major histocompatibility antigens. The term "HLA" as used herein will be understood to refer to Human Leukocyte Antigens, which is defined as the histocompatibility antigens found in humans. As used herein, "HLA" is the human form of "MHC".

The MHC gene family is divided into three subgroups: MHC class I, MHC class II, and MHC class III. Class I MHC molecules are composed of an a subunit and a β₂ microglobulin subunit, and are recognized by CD8+ T cells. Class II MHC molecules are composed of an a subunit and a β subunit and are recognized by CD4+ T cells. In this way MHC molecules determine which type of lymphocytes may bind to the given antigen with high affinity, since different lymphocytes express different T cell receptor (TCR) co-receptors (i.e., CD8 or CD4).

The term "MHC multimer" refers to oligomeric forms of MHC molecules. MHC multimers are commercially available or may be produced by methods known in the art or as described by Bentzen and Hadrup, Cancer Immunology, Immunotherapy 66.5: 657-666. Commercially available MHC multimers can comprise between 2 and 160 MHC molecules. Preferably, the MHC multimers of the invention comprise 4 or more MHC molecules. In certain embodiments, the MHC multimer may further comprise a fluorescent label. In certain embodiments, the MHC multimer is a fluorescently-labeled MHC dextramer, for example as commercially provided by Immudex.

The term "antigen presenting cell" as used herein will be understood to include any cell that can present peptides in the context of MHC molecules. Accordingly, the term "APC" encompasses both professional and non-professional antigen-presenting cells.

In a particular embodiment, the invention relates to the method according to the invention, wherein the non-target antigen is a peptide that is presented by an APC, by an MHC multimer, or wherein the non-target antigen is a cell that does not present the target antigen on its cell surface.

That is, the non-target antigen may have similar characteristics as defined above for the target antigen. In addition, the non-target antigen may be a cell that does not present the target antigen on its cell surface. In this case, the non-target antigen may be an APC that presents an irrelevant antigen or a cell that does not present an antigen on the cell surface via an MHC molecule.

As described above, the antigen, target antigen or non-target antigen of the present invention preferably comprises a peptide. Even more preferably, the antigen, target antigen or non-target antigen comprises a peptide that is derived from a tumor antigen.

The term "tumor antigen" as used herein indicates a molecule (e.g., a protein or peptide) that is expressed by a tumor cell and either (a) differs qualitatively from its counterpart expressed in normal cells, or (b) is expressed at a higher level in tumor cells than in normal cells. Thus, a tumor antigen can differ from (e.g., by one or more amino acid residues where the molecule is a protein) or it can be identical to its counterpart expressed in normal cells. Some tumor antigens are not expressed by normal cells, or are expressed at a level at least about two-fold higher (e.g., about two-fold, three-fold, five-fold, ten-fold, 20-fold, 40-fold, 100-fold, 500-fold, 1,000-fold, 5,000-fold, or 15,000-fold higher) in a tumor cell than in the tumor cell's normal counterpart.

Any suitable tumor antigen may be used in the practice of the present invention. Tumor antigens include without limitation naturally occurring tumor antigens and modified forms thereof that induce an immune response in a subject, and further include antigens associated with tumor cells and antigens that are specific to tumor cells and modified forms of the foregoing that induce an immune response in a subject. The term tumor antigen further encompasses antigens that correspond to proteins that are correlated with the induction of tumors such as oncogenic virus antigens (e.g., human papilloma virus antigens). Exemplary tumor antigens include, without limitation, HER2/neu, BRCAl and Fra-1 antigens for breast cancer, MART-1/MelanA and NY-ESO-I (melanoma antigens), NY-BR62, NY-BR85, hTERT, gp100, tyrosinase, TRP-I, TRP-2, NY-ESO-I, CDK-4, β-catenin, MUM-I, Caspase-8, KIAA0205, SART-I, PRAME, and pi 5 antigens, members of the MAGE family (melanoma antigens), the BAGE family (melanoma antigens), the DAGE/PRAME family (such as DAGE- 1), the GAGE family (melanoma antigens), the RAGE family (such as RAGE-I), the SMAGE family, NAG, TAG-72, CAl 25, mutated proto-oncogenes such as p21ras, NPM1, IDH1 and IDH2, mutated tumor suppressor genes such as p53, tumor associated viral antigens (e.g., HPV E6 and E7), the SSX family, HOM-MEL-55, NY-COL-2, HOM-HD-397, HOM-RCC-1.14, HOM-HD- 21, HOM-NSCLC-11, HOM-MEL-2.4, HOM-TES-11, RCC-3.1.3, and the SCP family. Members of the MAGE family include, but are not limited to, MAGE-I, MAGE-2, MAGE-3, MAGE-4, MAGE-6, MAGE-11 and MAGE- 12. Members of the GAGE family include, but are not limited to, GAGE-I, GAGE-6. See, e.g., the review by Van den Eynde and van der Bruggen, (1997) Curr. Opin. Immunol. 9: 684-693; and Sahin et al., (1997) Curr. Opin. Immunol. 9: 709-716.

The tumor antigen can also be, but is not limited to human epithelial cell mucin (Muc- 1 ; a 20 amino acid core repeat for the Muc-1 glycoprotein, present on breast cancer cells and pancreatic cancer cells), MUC-2, MUC-3, MUC-18, carcino-embryonic antigen (CEA), the raf oncogene product, CA- 125, GD2, GD3, GM2, TF, sTn, gp75, EBV-LMP 1 & 2, prostate- specific antigen (PSA), prostate-specific membrane antigen (PSMA), GnT-V intron V sequence (N-acetylglucosaminyltransferase V intron V sequence), Prostate Ca psm, MUM-I- B (melanoma ubiquitous mutated gene product), alpha-fetoprotein (AFP), COl 7-1 A, GA733, gp72, β-HCG, gp43, HSP-70 , pi 7 mel, HSP-70, gp43, HMW, HOJ-I, melanoma gangliosides, TAG-72, mutated proto-oncogenes such as p21ras, mutated tumor suppressor genes such as p53, estrogen receptor, milk fat globulin, telomerases, nuclear matrix proteins, prostatic acid phosphatase, protein MZ2-E, polymorphic epithelial mucin (PEM), folate- binding-protein LK26, truncated epidermal growth factor receptor (EGFR), Thomsen- Friedenreich (T) antigen, GM-2 and GD-2 gangliosides, polymorphic epithelial mucin, folate- binding protein LK26, human chorionic gonadotropin (HCG), pancreatic oncofetal antigen, cancer antigens 15-3,19-9, 549, 195, squamous cell carcinoma antigen (SCCA), ovarian cancer antigen (OCA), pancreas cancer associated antigen (PaA), EBNA (Epstein-Barr Virus nuclear antigen) 1-6, gp75, chimeric protein P210BCR-ABL, lung resistance protein (LRP) Bcl-2, and Ki-67. See, e.g., U.S. Patent No. 6,537,552; see also U.S. Patent Nos. 6,815,531 ; 6,773,707; 6,682,928; and 6,623,739.

In a particular embodiment, the invention relates to the method according to the invention, wherein the binding between an antigen and a cell expressing a T cell receptor is determined by measuring the binding avidity of the T cell receptor for the antigen. Preferably, the antigen is a peptide bound to a fluorescently-labeled MHC multimer.

The present invention relates to methods wherein the binding between a T cell receptor and an antigen is determined. In general, the binding between a T cell receptor and an antigen may be determined by any method known in the art. Most commonly, the binding of a T cell receptor to an antigen is determined by measuring the affinity and/or the avidity of the T cell receptor for the antigen.

The term "affinity" as used herein means the strength of a single interaction between, for example, a peptide (presented in the context of an MHC) and a TCR. Affinity is measured in a cell-free context, for example, where the MHC-presented peptide is immobilized on a solid interface and the TCR of interest is in solution.

The term "avidity" as used herein refers to a measure of multiple affinities and reflects the overall binding strength or the observed functional response between a target peptide-MHC of interest and a TCR. In this context, the peptide-MHC and the TCR can be presented on the surface of a cell or may be provided as soluble binding reagents (e.g., monomers or multimers). Preferably, the TCR is presented on the surface of the cell according to the invention, and the peptide-MHC target is in the form of a soluble reagent (i.e., peptide-MHC multimers) or presented by an APC. The term "functional avidity" as used herein is the concentration of an antigen (e.g. peptide) required to achieve 50% of maximal response in a functional assay. For each functional assay, the maximal response is determined. Functions measured include for example antigen-induced signaling as described in the invention, cytokine secretion and lysis of target cells (i.e., cytotoxicity). The maximal response is obtained when T cells are maximally stimulated. Therefore, maximal responses depend on the functional capabilities of given T cell(s) and can for example be expressed as EC50 in µM or other molar units. The term "structural avidity" or "binding avidity" as used herein, which can be expressed as EC50 in µg/mL or other concentration units, is the concentration of an antigen (e.g. a peptide bound by an MHC multimer) required to achieve half-maximal antigen staining (e.g. multimer staining). Functional and structural avidity can for example be calculated with software known in the art such as GraphPad prism 6.

Functional avidity can be measured for example using an enzyme-linked immunospot (ELISpot) assay and measuring, for example, interferon-gamma (IFNγ) and/or IL-2 production and/or a combination thereof. Structural avidity can be measured by staining TCR expressing cells, optionally T cells or the cells according to the invention expressing a TCR, using graded concentrations of peptide-MHC multimers. In some embodiments, avidity may be assessed by flow cytometric analysis for specific TCR binding to fluorochrome-labeled multimeric synthetic peptide-MHC complexes, and/or functional assessment with peptide-pulsed APCs and screening for IFN-gamma production using standard enzyme-linked immunosorbent assay (ELISA) or ELISpot assays. In some embodiments, T cell avidity may be detected via a dual parameter cell sorting protocol that detects cells bound to the fluorescently labeled multimer in conjunction with, indicating that the cell was activated by the recognition of the TCR-multimer complex.

Examples 1 and 6 further describe how the binding between a T cell receptor and an antigen can be determined by measuring the binding avidity of a T cell receptor expressed on the surface of the cell according to the invention for a fluorescently-labeled peptide-bound MHC multimer (MHC dextramer from Immudex). Thus, in a preferred embodiment, the structural avidity of a T cell receptor is determined with a fluorescently-labeled MHC multimer, in particular a fluorescently labeled MHC dextramer from Immudex. MHC dextramers are fluorescently-labeled MHC multimers that comprise a dextran polymer backbone carrying an optimized number of MHC molecules and fluorochrome molecules. Common fluorochrome molecules that may be comprised in MHC dextramers, or MHC multimers in general, are fluorescein isothiocyanate (FITC), allophycocyanin (APC) or phycoerythrin (PE). The advantage of fluorescently-labeled MHC multimers is that binding of the multimer to a TCR, and thus the binding avidity of the TCR, can be readily determined using flow cytometry. Thus, in a particular embodiment, the invention relates to the method according to the invention, wherein the binding avidity is measured by flow cytometry.

The term "flow cytometry", as used herein, refers to a tool for interrogating the phenotype and characteristics of cells. It detects cells or particles as they move in a liquid stream through a laser (light amplification by stimulated emission of radiation)/light beam past a sensing area. The relative light-scattering and fluorescence of the microscopic particles is measured. Analysis and differentiation of the cells is based on size, granularity, and whether the cells are carrying fluorescent molecules in the form of either antibodies, fluorescent proteins or dyes. Flow cytometers are multiparameter, recording several measurements on each cell; therefore, it is possible to identify a homogeneous subpopulation within a heterogeneous population (Marion G. Macey, Flow cytometry: principles and applications, Humana Press, 2007). Within the present invention, it is intended that the flow cytometer detects, for example, the fluorescent dye that is comprised in the MHC multimer and eGFP produced by cells according to the invention.

In certain embodiments of the invention, the potential of a T cell receptor to bind to an antigen is determined. It is preferred that the binding is determined by measuring the binding avidity of a TCR for an antigenic peptide that is presented on a fluorescently-labeled MHC dextramer. In this case, the binding avidity is measured as the concentration of peptide-bound MHC dextramers that is required to achieve half-maximal staining of the cells expressing the TCR. Thus, a lower EC50 value generally correlates with a higher avidity and a higher antigen-binding potential of the TCR. In general, a TCR is said to have the potential to bind an antigenic peptide, if staining of a TCR-expressing cell with a fluorescently-labeled MHC dextramer comprising the antigenic peptide can be achieved. Preferably, a TCR is said to have the potential to bind to an antigenic peptide, if a concentration of the fluorescently-labeled MHC dextramer comprising the antigenic peptide of below 6.4 nM, 4 nM, 2 nM, 1.5 nM, 1.0 nM, 0.9 nM, 0.8 nM, 0.7 nM, 0.6 nM, 0.5 nM, 0.4 nM, 0.3 nM or 0.267 nM is sufficient to achieve half-maximal staining of the TCR-expressing cells according to the invention. It should be noted that the concentrations listed above apply to peptide-MHC dextramer reagents (Immudex, Copenhagen, Denmark) only, and that half-maximal binding concentrations would certainly be higher for less avid peptide-MHC reagents such as pentamers, tetramers, trimers, dimers and monomers. In the context of affinity measurements between monomeric TCR and monomeric peptide-MHC using surface plasmon resonance or biolayer interferometry, a TCR is said to have the potential to bind an antigenic peptide if its affinity constant (K_{D}) is below 100000 nM, 50000 nM, 10000 nM, 1000 nM, 100 nM or 10 nM.

In certain embodiments of the inventions, T cell receptors are identified that show optimized binding of a target or non-target antigen. In this case, it is preferred that a T cell receptor with optimized antigen-binding properties has a lower EC50 and a higher avidity for a target antigen than a TCR that has been used as the starting point for the optimized T cell receptor. Alternatively, a T cell receptor with optimized antigen-binding properties may have a higher EC50 and a lower avidity for a non-target antigen than a TCR that has been used as the starting point for the optimized T cell reporter.

In a particular embodiment, the invention relates to the method according to the invention, wherein the activation of a cell expressing a T cell receptor is determined by measuring the expression of the reporter system and/or by measuring the expression of an activation marker after contact with an antigen. Preferably, the antigen is a peptide presented on the surface of an APC.

In certain embodiments, the potential of an antigen to activate a cell expressing a T cell receptor is determined. Within the present invention, a cell expressing a T cell receptor is said to be activated by an antigen if the antigen is capable of stimulating the cell harboring such T cell receptor. The skilled person is aware of methods to determine the potential of an antigen to activate a cell expressing a T cell receptor. In general, the potential of an antigen to activate a cell expressing a T cell receptor may be determined during or after the cell has been contacted with the antigen. For that, the cell may be contacted with the antigen for any amount of time, ranging from seconds to several days.

In certain embodiments, the potential of an antigen to activate a cell expressing a T cell receptor may be determined by measuring the production of cytokines such as interferon gamma, interleukin-2 or tumor necrosis factor α by the cell upon contacting the cell with the antigen. The skilled person is aware of methods to contact cells with an antigen and to measure the production of cytokines. In particular, commercial kits for determining the concentration of cytokines are available.

Alternatively, the potential of an antigen to activate a cell expressing a T cell receptor may be determined by measuring the expression of an activation marker during or after contact of the cell with the antigen. The term "activation marker" as used herein refers to proteins specifically expressed on the cell surface of T cells following their activation via their specific antigen-receptor, usually within 1-48 hours after activation. Such activation markers may be, for example, CD69, CD154, CD137, secreted or membrane-anchored cytokines, e.g. IL-4, IL-5, IL-13, IFN-gamma, IL-10, IL-2, IL-22, TNF-alpha or "latent TGF-beta" (LAP), GARP (LRRC32), CD121a/b. Preferably, the activation marker is CD69. Thus, in a particular embodiment, the invention relates to the method according to the invention, wherein the activation marker is CD69. The skilled person is aware of methods to measure the expression of an activation of the surface of a cell. For example, the expression of an activation marker may be measured with the help of antibodies that specifically bind to the activation markers.

The term "CD69" as used herein, refers to Cluster of Differentiation 69 which is a human transmembrane C-Type lectin protein encoded by the CD69 gene. Activation of T lymphocytes and natural killer (NK) cells, both in vivo and in vitro, induces expression of CD69. CD69 functions as a signal-transmitting receptor involved in the cellular activation of lymphocytes, including natural killer cells and T cells.

However, within the present invention, it is preferred that the activation of a cell expressing a T cell receptor with an antigen is determined by measuring the expression of the reporter system comprised in the cell according to the invention. Depending on the detectable marker of the reporter system, different means to measure the expression of the reporter system may be applied. Preferably, the detectable marker of the reporter system is a fluorescent protein. In this case, the expression of the reporter system may be measured by analyzing the fluorescence of the cells expressing the reporter protein, for example by flow cytometry. Within the present invention, a cell expressing a T cell receptor is determined to be activated by an antigen, if the cell expresses detectable amounts of the reporter protein. That is, a cell expressing a T cell receptor is determined to be activated by an antigen if the cell expresses higher amounts of the reporter protein than a cell with the same genotype that has been contacted with an irrelevant antigen.

In certain embodiments, the detectable marker of the reporter system is eGFP. In this case, a cell expressing a T cell receptor is determined to be activated by an antigen, if the cell expresses higher amounts of eGFP than a cell with the same genotype that has been contacted with an irrelevant antigen.

In certain embodiments, the potential of an antigen to activate a cell expressing a T cell receptor may be determined by a combination of two or more methods. For example, in certain embodiments, the potential of an antigen to activate a cell expressing a T cell receptor may be determined by measuring the activity of one or more reporter systems in the cell according to the invention and in addition by measuring the production of one or more cytokines and/or the expression of one or more activation markers by the cell according to the invention. In certain embodiments, the potential of an antigen to activate a cell expressing a T cell receptor may be determined by measuring the expression of the detectable marker eGFP and the expression of the activation marker CD69 in the cell according to the invention.

In certain embodiments, a method for identifying T cell receptors with optimized antigen-induced signaling properties is described. The term "antigen-induced signaling properties" refers to the ability of a T cell receptor to be stimulated by an antigen such that the cell expressing the T cell receptor is activated by the antigen. In certain embodiments, a T cell receptor is determined to have optimized antigen-induced signaling properties if a cell expressing the T cell receptor expresses higher levels of the reporter protein and/or the activation marker, and/or produces higher amounts of a cytokine during or after contacting with an antigen compared to a cell expressing the T cell receptor that was used as a starting point.

When determining the potential of an antigen to activate a cell expressing a T cell receptor, it is generally preferred that the antigen is a peptide that is presented on the surface of an antigen-presenting cell. Without being bound to theory, it is assumed that the activation of T cells by a peptide-MHC complex involves further co-stimulatory signals between the cell to be activated and the APC, such as, for example, the interaction between CD28 and CD80/CD86. Thus, it is preferred that the antigenic peptide is displayed by an APC when determining the antigen-induced signaling properties of a T cell receptor. If a particular APC lacks expression of certain co-stimulatory receptors, the antigen-induced signaling properties of a T cell receptor may also be determined by contacting the cell expressing the T cell receptor with a soluble peptide-MHC complex, a peptide-MHC multimer or any other immobilized peptide-MHC complex and an appropriate mixture of co-stimulatory molecules.

In a particular embodiment, the invention relates to a T cell receptor as identified by the method according to the invention or a functional portion thereof.

The methods of the present invention can be used for the discovery of antigen-specific T cell receptors and for engineering T cell receptors with optimized antigen-binding or antigen-induced signaling properties. Accordingly, the invention further encompasses antigen-specific T cell receptors and T cell receptors with optimized antigen-binding and/or antigen-induced signaling properties that have been obtained with the methods according to the invention.

In particular, the invention relates to a T cell receptor with optimized antigen-binding and/or antigen-induced signaling properties for the target antigen MAGE-A3 comprising the peptide EVDPIGHLY (SEQ ID NO. 11). Thus, in certain embodiments, the invention relates to a T cell receptor that binds the MAGE-A3 antigen and/or is stimulated by the MAGE-A3 antigen more efficiently than the parental anti-MAGE-A3 TCR with SEQ IDs NO. 9 and 10. In particular, the invention relates to a T cell receptor that binds the MAGE-A3 antigen and/or is stimulated by the MAGE-A3 antigen more efficiently than the parental anti-MAGE-A3 TCR with SEQ IDs NO. 9 and 10, but does not show cross reactivity with an antigen comprising the Titin-derived peptide ESDPIVAQY (SEQ ID NO. 14).

In certain embodiments, the invention relates to a T cell receptor with optimized antigen-binding and/or antigen-induced signaling properties for a non-target antigen derived from Titin, in particular an antigen comprising the peptide sequence ESDPIVAQY (SEQ ID NO. 14). Thus, in certain embodiments, the invention relates to a T cell receptor that binds the ESDPIVAQY antigen and/or is stimulated by the ESDPIVAQY antigen less efficiently than the engineered anti-MAGE-A3 TCR a3a (SEQ IDs NO. 12 and 13). In particular, the invention relates to a T cell receptor that binds the ESDPIVAQY antigen and/or is stimulated by the ESDPIVAQY antigen less efficiently than the engineered anti-MAGE-A3 TCR a3a (SEQ IDs NO. 12 and 13), but binds to or is stimulated by the MAGE-A3 antigen EVDPIGHLY (SEQ ID NO. 11).

In a particular embodiment, the invention relates to the T cell receptor or the functional portion thereof according to the invention, wherein the functional portion is comprised in a soluble T cell receptor.

The term "functional portion" when used in reference to a TCR refers to any part or fragment of the TCR of the invention, which part or fragment retains the biological activity of the TCR, of which it is a part (the parent TCR). Functional portions encompass, for example, those parts of a TCR that retain the ability to specifically bind to the antigen (in an HLA-dependent manner) to a similar extent, the same extent, or to a higher extent, as the parent TCR. In reference to the parent TCR, the functional portion can comprise, for instance, about 10%, 25%, 30%, 50%, 68%, 80%, 90%, 95%, or more, of the parent TCR variable sequences.

Some embodiments of the invention also pertain to TCRs, or functional portions and polypeptides thereof, which are soluble TCRs. As used herein, the term "soluble T cell receptor" refers to heterodimeric truncated variants of native TCRs, which comprise extracellular portions of the TCR α-chain and β-chain, for example linked by a disulfide bond, but which lack the transmembrane and cytosolic domains of the native protein. The skilled person is aware of methods for producing soluble T cell receptors and functional portions of T cell receptors.

In a particular embodiment, the invention relates to the T cell receptor or the functional portion thereof according to the invention, wherein the soluble T cell receptor is coupled to a biologically active molecule, in particular wherein the biologically active molecule is an agonistic antibody, a cytokine, or a cytotoxic agent.

That is, the soluble T cell receptor according to the invention may further be coupled to a biologically active molecule, such as a therapeutic agent. Therapeutic agents which may be associated with the TCRs of the invention include:
- immunomodulators, radioactive compounds, enzymes (perforin for example) or chemotherapeutic agents (cisplatin for example). To ensure that toxic effects are exercised in the desired location the toxin may be inside a liposome linked to TCR so that the compound is released slowly. This will prevent damaging effects during the transport in the body and ensure that the toxin has maximum effect after binding of the TCR to the relevant target cells.

Other suitable therapeutic agents include:
- small molecule cytotoxic agents, i.e. compounds with the ability to kill mammalian cells having a molecular weight of less than 700 Daltons. Such compounds could also contain toxic metals capable of having a cytotoxic effect. Furthermore, it is to be understood that these small molecule cytotoxic agents also include pro-drugs, i.e. compounds that decay or are converted under physiological conditions to release cytotoxic agents. Examples of such agents include cis-platin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E vincristine and doxorubicin;
- peptide cytotoxins, i.e. proteins or fragments thereof with the ability to kill mammalian cells. For example, ricin, diphtheria toxin, pseudomonas bacterial exotoxin A, DNase and RNase;
- radio-nuclides, i.e. unstable isotopes of elements which decay with the concurrent emission of one or more of α or β particles, or γ rays. For example, iodine 131 , rhenium 186, indium 111 , yttrium 90, bismuth 210 and 213, actinium 225 and astatine 213; chelating agents may be used to facilitate the association of these radio-nuclides to the high affinity TCRs, or multimers thereof;
- immuno-stimulants, i.e. immune effector molecules which stimulate immune response. For example, cytokines such as IL-2, IL-15 and IFN-γ,
- superantigens and mutants thereof;
- TCR-HLA fusions;
- chemokines such as IL-8, platelet factor 4, melanoma growth stimulatory protein, etc;
- antibodies or fragments thereof, including anti-T cell or NK cell determinant antibodies (e.g. anti-CD3, anti-CD28 or anti-CD16), and the antibody Fc region;
- alternative protein scaffolds with antibody-like binding characteristics;
- complement activators;
- xenogeneic protein domains, allogeneic protein domains, viral/bacterial protein domains, viral/bacterial peptides. One preferred embodiment is provided by a TCR of the invention associated (usually by fusion to an N-or C-terminus of the alpha or beta chain) with an anti-CD3 antibody, or a functional fragment or variant of said anti-CD3 antibody. Antibody fragments and variants/analogues which are suitable for use in the compositions and methods described herein include minibodies, Fab fragments, F(ab')2 fragments, dsFv and scFv fragments, Nanobodies™ (these constructs, marketed by Ablynx (Belgium), comprise synthetic single immunoglobulin variable heavy domain derived from a camelid (e.g. camel or llama) antibody) and Domain Antibodies (Domantis (Belgium), comprising an affinity matured single immunoglobulin variable heavy domain or immunoglobulin variable light domain) or alternative protein scaffolds that exhibit antibody like binding characteristics such as Affibodies (Affibody (Sweden), comprising engineered protein A scaffold) or Anticalins (Pieris (Germany), comprising engineered anticalins) to name but a few.

Within the present invention, a soluble TCR may be "coupled" to a biologically active molecule via a covalent or non-covalent bond.

In a particular embodiment, the invention relates to a polynucleotide encoding the T cell receptor or the functional portion thereof according to the invention.

The application further encompasses polynucleotides that encode the T cell receptors or the functional portions thereof according to the invention. The skilled person is aware of methods to produce or obtain polynucleotides encoding the T cell receptors or the functional portions thereof.

The term "polynucleotide" as used herein refers to a sequence of nucleotides connected by phosphodiester linkages. A polynucleotide of this invention can be a deoxyribonucleic acid (DNA) molecule or ribonucleic acid (RNA) molecule in either single- or double-stranded form. Nucleotide bases are indicated herein by a single letter code: adenine (A), guanine (G), thymine (T), cytosine (C), inosine (I) and uracil (U). A polynucleotide of this invention can be prepared using standard techniques well known to one of ordinary skill in the art. This term is not to be construed as limiting with respect to the length of a polymer, and encompasses known analogues of natural nucleotides, as well as nucleotides that are modified in the sugar and/or phosphate moieties. This term also encompasses nucleic acids containing modified backbone residues or linkages, which may be synthetic or naturally-occurring, and which have similar binding properties as the reference nucleic acid and are metabolized in a manner similar to the reference nucleotides.

In a particular embodiment, the invention relates to a viral vector comprising a polynucleotide according to the invention. Preferably, the viral vector is derived from a lentivirus, a retrovirus or an adeno-associated virus.

That is, the polynucleotide according to the invention may be comprised in a viral vector. Viral vectors are commonly used for delivering polynucleotides into target cells. Thus, the polynucleotide according to the invention may preferably be comprised in a viral vector that can be used for the delivery of polynucleotides into target cells. In human gene therapy, most commonly adeno-associated viruses and retroviruses such as lentiviruses are used for the delivery of transgenes into human cells. Thus, in a preferred embodiment, the invention relates to a viral vector comprising a polynucleotide according to the invention, wherein the viral vector is derived from a lentivirus, a retrovirus or an adeno-associated virus. The skilled person is aware of methods for introducing polynucleotides encoding T cell receptors into viral vectors and of methods for delivering these polynucleotides to human cells.

The term "viral vector" as used herein denotes vectors that are derived from viruses including but not limited to: retrovirus, including lentivirus, adeno-associated virus, adenovirus, herpesvirus, hepatitis virus or the like. Typically, but not necessarily, viral vectors are replication-deficient, as they have lost the ability to propagate in a given cell since viral genes essential for replication have been eliminated from the viral vector. However, some viral vectors can also be adapted to replicate specifically in a given cell, such as e.g. a cancer cell, and are typically used to trigger cell-specific (onco)lysis. Within the present invention, a viral vector is said to be derived from a lentivirus, a retrovirus or an adeno-associated virus if the viral vector comprises viral genes from a lentivirus, a retrovirus or an adeno-associated virus, respectively.

In a particular embodiment, the invention relates to a cell comprising the polynucleotide according to the invention or the viral vector according to the invention. Preferably, the cell is a T cell.

The polynucleotide or the viral vector encoding the T cell receptor or the functional portion thereof according to the invention may be comprised in any cell. In certain embodiments, a cell may comprise a polynucleotide encoding a T cell receptor that can be expressed by the cell. In certain embodiments, this cell is a T cell that may be used in cell therapy. In certain embodiments, this T cell is an autologous or an allogeneic T cell that may be re-introduced into a subject. Methods to introduce polynucleotides or viral vectors into cells are well known in the art and can be carried out by the skilled person.

Within the present invention, it is preferred that the polynucleotide encoding a T cell receptor according to the invention is delivered to a cell, preferably a T cell, by viral transduction as described above and as known in the art. However, the invention also encompasses cells that comprise the polynucleotide according to the invention, wherein the polynucleotide according to the invention has been integrated into the cell by other means. For example, the polynucleotide according to the invention may be introduced into the cell by any method known in the art, for example by transfection, and integrated into the genome of the cell through homologous recombination or homology directed repair of a double strand break that has been introduced by an endonuclease, for example Cas9 or a TALEN. Alternatively, the polynucleotide according to the invention may be comprised in a transposable element that has integrated into the genome of the cell.

In a particular embodiment, the invention relates to the T cell receptor or the functional portion thereof according to the invention, the polynucleotide according to the invention, the viral vector according to the invention or the cell according to the invention for use in therapy. Preferably for use in cancer therapy.

That is, the T cell receptor, the functional portion thereof, the soluble T cell receptor, the polynucleotide, the viral vector or the cell according to the invention may be used in any form of therapy. Preferably, the T cell receptor, the functional portion thereof, the soluble T cell receptor, the polynucleotide, the viral vector or the cell according to the invention may be used for treating cancer.

Thus, in certain embodiments, the provided T cell receptor, the functional portion thereof, the soluble T cell receptor, the polynucleotide, the viral vector or the cell according to the invention may be used for treating a hyperproliferative disorder that is a hematological malignancy or a solid cancer. For example, the hematological malignancy to be treated may be acute lymphoblastic leukemia (ALL), acute myeloid leukemia (AML), chronic myelogenous leukemia (CML), chronic eosinophilic leukemia (CEL), myelodysplastic syndrome (MDS), non-Hodgkin's lymphoma (NHL), or multiple myeloma (MM). Exemplary solid cancers to be treated may be biliary cancer, bladder cancer, bone and soft tissue carcinoma, brain tumor, breast cancer, cervical cancer, colon cancer, colorectal adenocarcinoma, colorectal cancer, desmoid tumor, embryonal cancer, endometrial cancer, esophageal cancer, gastric cancer, gastric adenocarcinoma, glioblastoma multiforme, gynecological tumor, head and neck squamous cell carcinoma, hepatic cancer, lung cancer, mesothelioma, malignant melanoma, osteosarcoma, ovarian cancer, pancreatic cancer, pancreatic ductal adenocarcinoma, primary astrocytic tumor, primary thyroid cancer, prostate cancer, renal cancer, renal cell carcinoma, rhabdomyosarcoma, skin cancer, soft tissue sarcoma, testicular germ-cell tumor, urothelial cancer, uterine sarcoma, or uterine cancer.

Even more preferably, the T cell receptor, the functional portion thereof, the soluble T cell receptor, the polynucleotide, the viral vector or the cell according to the invention may be used for treating melanoma, in particular melanoma that is associated with the expression of the MAGE-A3 antigen.

In a particular embodiment, the invention relates to the T cell receptor or the functional portion thereof, the polynucleotide, the viral vector or the cell for use according to the invention, wherein the T cell receptor or the functional portion thereof, the polynucleotide, the viral vector or the cell are administered to a subject.

That is, for use in therapy, the TCRs, the functional portions thereof, the polynucleotides, the viral vectors and the cells according to the invention may be administered to a subject. Administration to a subject preferably comprises formulating the active ingredient into a pharmaceutical composition.

The inventive TCRs, the functional portions thereof, the polynucleotides, the viral vectors and cells according to the invention, all of which are collectively referred to as "inventive TCR materials" hereinafter, can be formulated into a composition, such as a pharmaceutical composition. In this regard, the invention provides a pharmaceutical composition comprising any of the TCRs, the functional portions thereof, polynucleotides, viral vectors and cells, and a pharmaceutically acceptable carrier. The inventive pharmaceutical compositions containing any of the inventive TCR materials may comprise more than one inventive TCR material, e.g., a soluble portion of a TCR and a viral vector, or two or more soluble portions of TCRs. Alternatively, the pharmaceutical composition can comprise an inventive TCR material in combination with another pharmaceutically active agents or drugs, such as a chemotherapeutic agent.

Preferably, the carrier is a pharmaceutically acceptable carrier. With respect to pharmaceutical compositions, the carrier can be any of those conventionally used and is limited only by chemico-physical considerations, such as solubility and lack of reactivity with the active compound(s), and by the route of administration. The pharmaceutically acceptable carriers described herein, for example, vehicles, adjuvants, excipients, and diluents, are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular inventive TCR material, as well as by the particular method used to administer the inventive TCR material. Accordingly, there are a variety of suitable formulations of the pharmaceutical composition of the invention. The following formulations for oral, aerosol, parenteral, subcutaneous, intravenous, intramuscular, intraarterial, intrathecal, intraperitoneal, rectal, and vaginal administration are exemplary and are in no way limiting. More than one route can be used to administer the inventive TCR materials, and in certain instances, a particular route can provide a more immediate and more effective response than another route.

The term "administration," as used herein to refer to the delivery of an inventive TCR material to a subject, is not limited to any particular route but rather refers to any route accepted as appropriate by the medical community. The term "subject" as used herein denotes any animal, preferably a mammal, and more preferably a human. Examples of subjects include humans, non-human primates, rodents, guinea pigs, rabbits, sheep, pigs, goats, cows, horses, dogs and cats. Within the present invention, the term "subject" is used interchangeably with the term "patient".

In a particular embodiment, the invention relates to the T cell receptor or the functional portion thereof, the polynucleotide, the viral vector or the cell for use according to the invention, wherein the cell has been obtained from the same subject prior to introducing the polynucleotide or viral vector into said cell.

In certain embodiments a cell comprising a polynucleotide according to the invention may be used for treating cancer. For that, the polynucleotide according to the invention or the viral vector according to the invention may be introduced into a cell. Preferably that cell is a T cell and even more preferably a CD8+ T cell. To ensure compatibility between the T cell and the subject to be treated, the cell is most preferably a T cell that has been obtained from the subject to be treated. Thus, in certain embodiments, T cells may be isolated from a subject and a polynucleotide or a viral vector encoding a T cell receptor according to the invention is introduced into these T cells. The T cells comprising the polynucleotide or the viral vector may then be re-injected into the subject that the T cells have been obtained from. In certain embodiments, this subject may be a subject suffering from cancer. In certain embodiments, the subject may be suffering from a cancer that is associated with the production of a tumor antigen that can be recognized by the T cell receptor that is expressed from the polynucleotide or viral vector comprised in the cell. In certain embodiments, it has been determined with one of the methods according to the invention that the T cell receptor that is expressed from the polynucleotide or viral vector comprised in the cell is specific for an antigen that is produced by the subject's tumor.

### BRIEF DESCRIPTION OF THE FIGURES

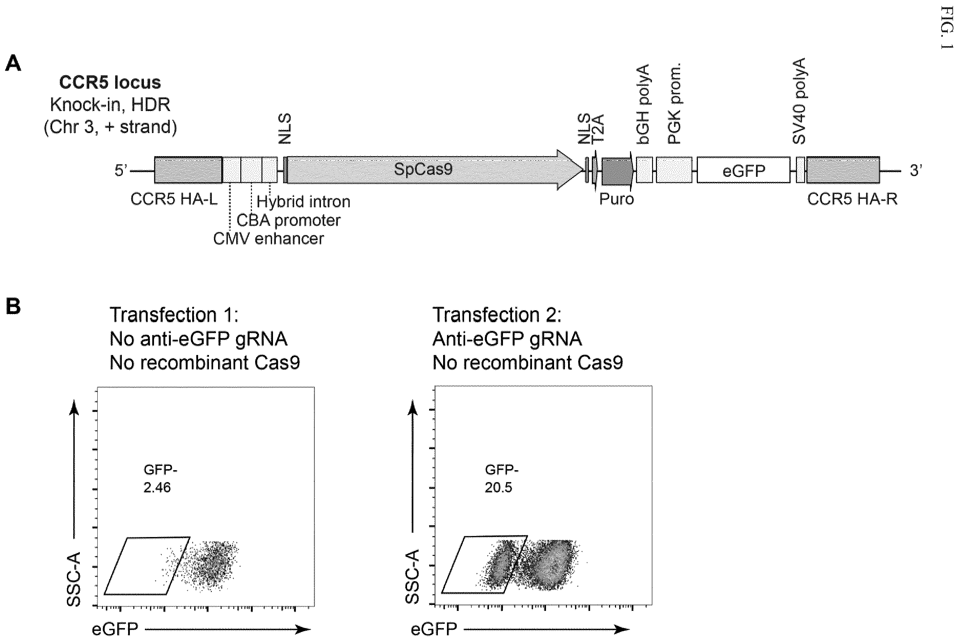

**FIG 1****. A.** Dual promoter cassette encoding Cas9 nuclease and eGFP introduced at the CCR5 safe harbor locus via CRISPR-Cas9 HDR. **B.** Validation of transgenic Cas9 activity through transfection of an anti-eGFP gRNA in the absence of exogenous recombinant Cas9 protein.

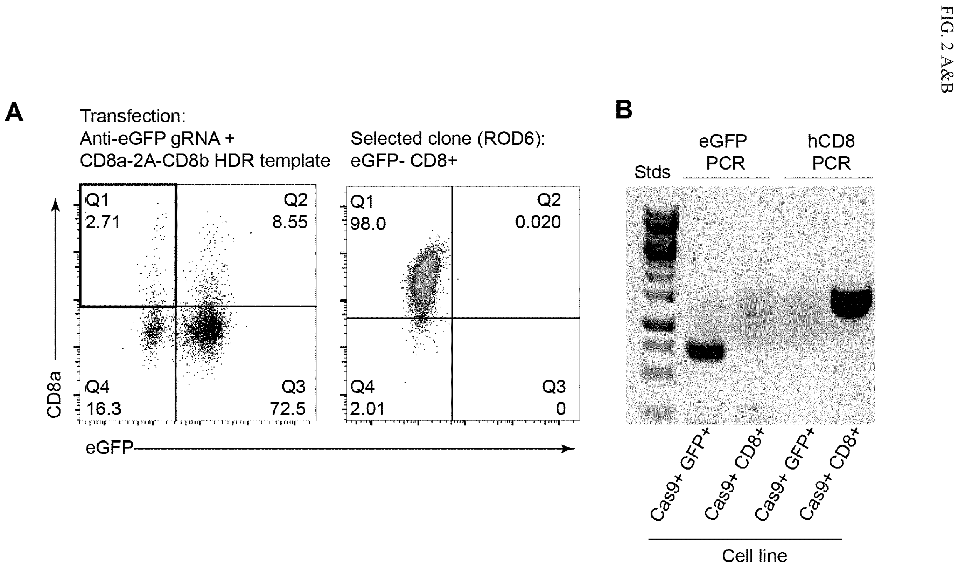

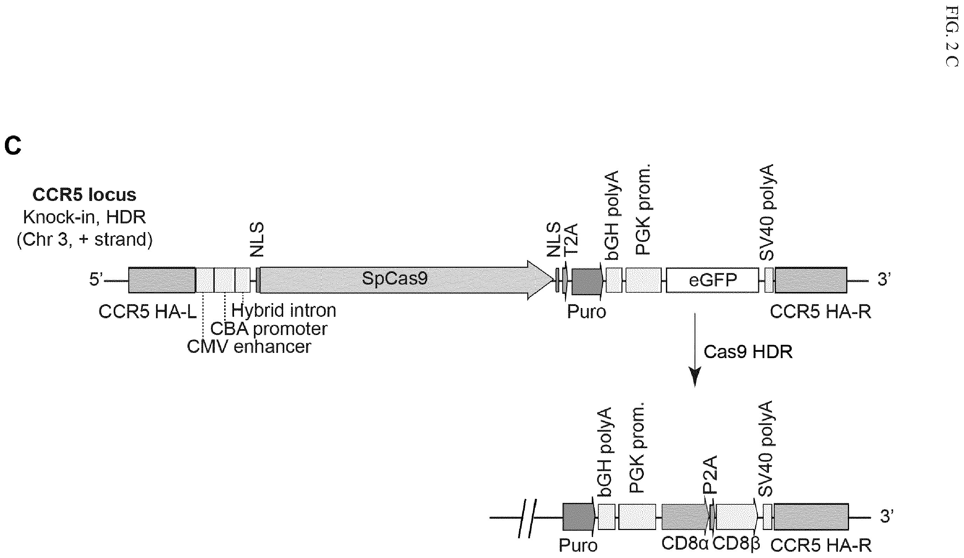

**FIG 2****. A.** Flow cytometric assessment of CRISPR-Cas9 targeted replacement of eGFP for human CD8 at the CCR5 locus. The selected clone ROD6 is CD8 positive and eGFP negative. **B.** Assessment of CRISPR-Cas9 targeted replacement of eGFP for human CD8 at the CCR5 locus by means of genomic PCR. **C.** Schematic representation of the engineered CCR5 locus present in ROD6 cells after replacement of eGFP for human CD8.

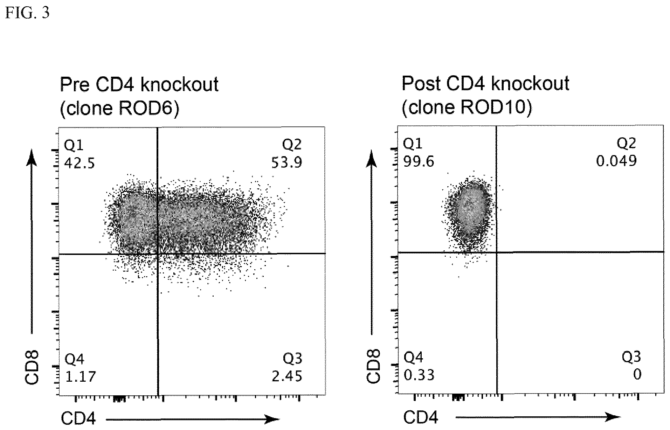

**FIG 3****.** Flow cytometric assessment of endogenous Jurkat CD4 co-receptor knockout through CRISPR-Cas9 NHEJ. The selected clone ROD10 is CD8 positive and CD4 negative.

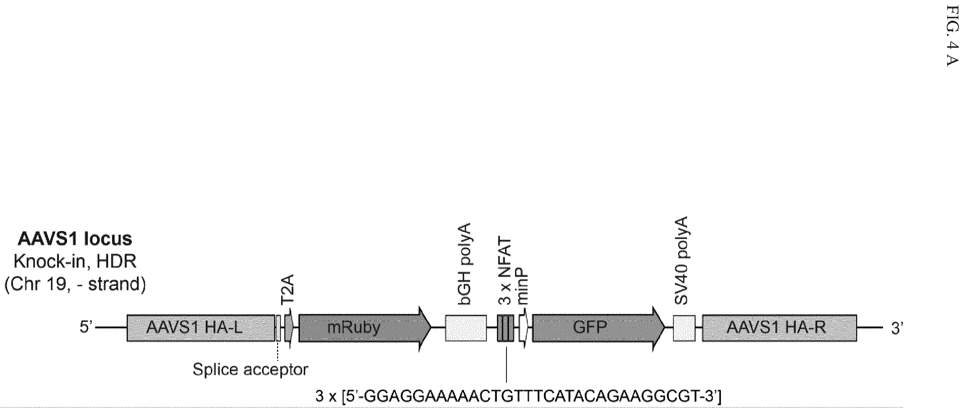

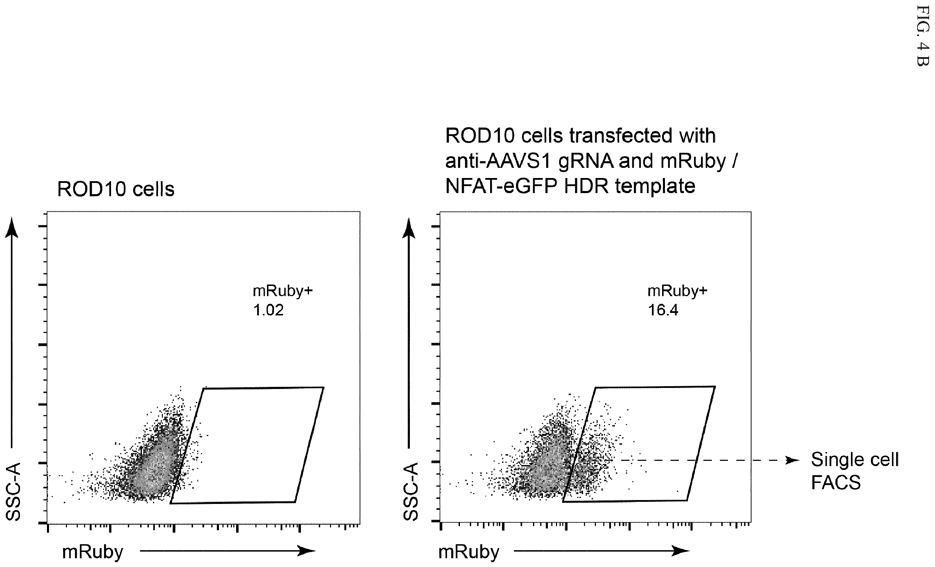

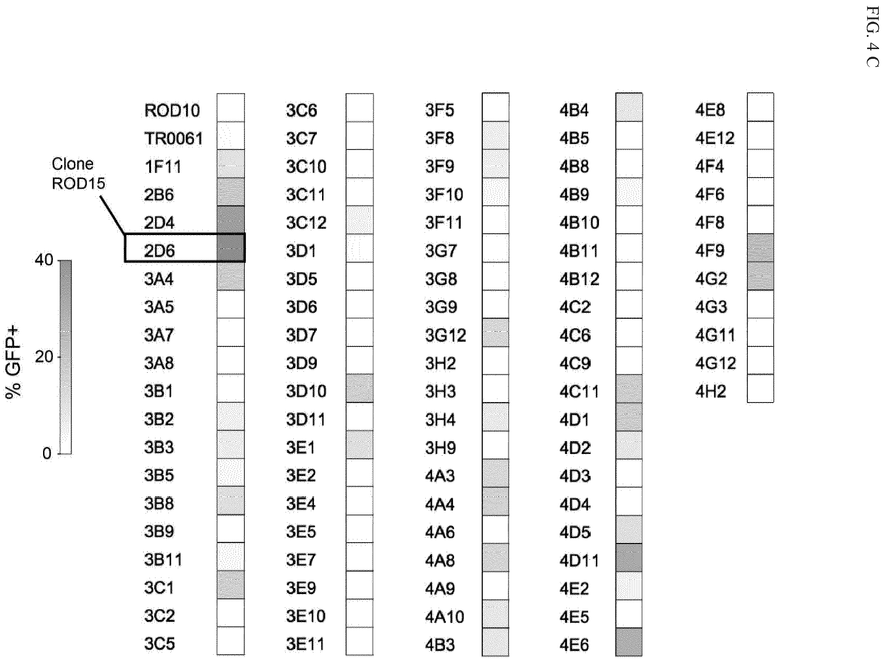

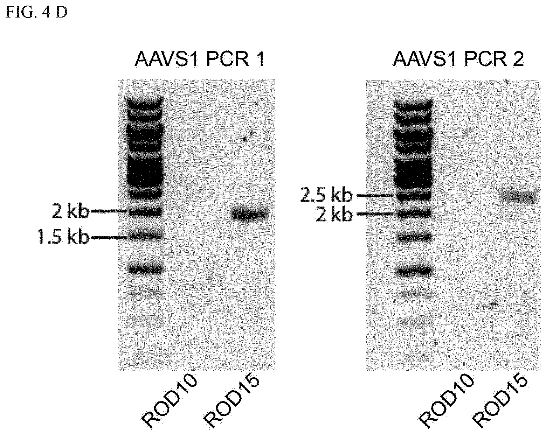

**FIG 4****. A.** Schematic representation of the HDR template utilized for CRISPR-Cas9 targeted insertion of mRuby and NFAT-eGFP genes into the AAVS1 locus. The DNA sequence of the NFAT response element (SEQ ID NO. 1) is displayed. **B.** Flow cytometric assessment of transgenic mRuby expression after CRISPR-Cas9 HDR. **C.** Flow cytometric screening and selection of mRuby+ clones based on their eGFP expression levels after overnight stimulation with plate-bound anti-CD3 agonistic antibody (clone OKT3). The selected clone ROD15 displayed the highest proportions of eGFP+ cells after stimulation. **D.** Assessment of CRISPR-Cas9 targeted insertion of the mRuby / NFAT-GFP cassette into the AAVS1 locus by means of genomic PCR. Two different primer pairs were designed so that each pair contained one primer outside and the other primer inside the transfected AAVS1 HDR template. The selected clone ROD15 yielded expected DNA bands after gel electrophoresis, while its parental cell line ROD10 yielded no bands.

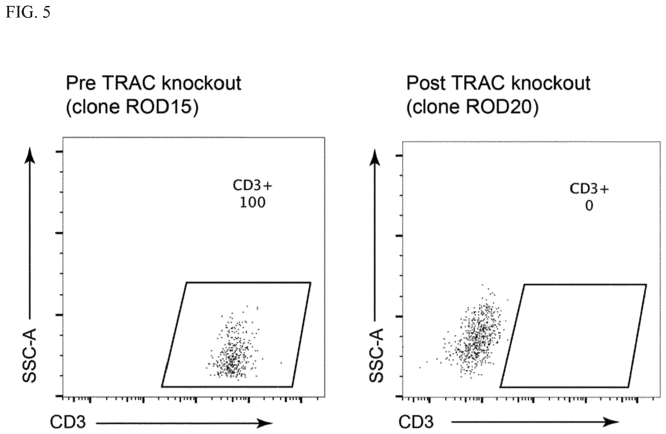

**FIG 5****.** Flow cytometric assessment of endogenous Jurkat TCRα knockout after CRISPR-Cas9 NHEJ. Targeting of the TCRα constant domain (TRAC) with anti-TRAC gRNA resulted in abolished surface expression of the TCR:CD3 complex in the selected ROD20 clone.

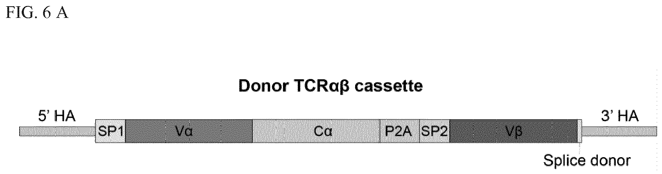

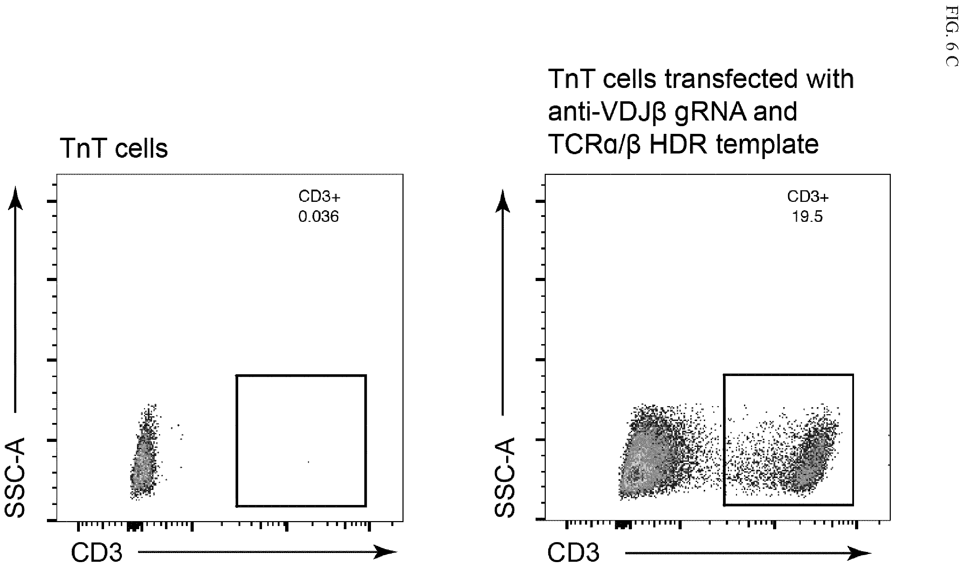

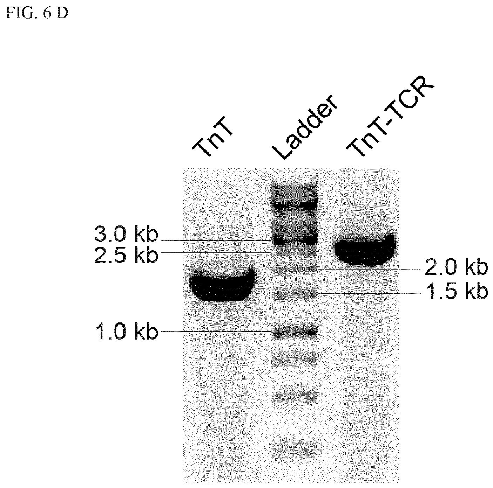

**FIG 6****. A.** Example TCRα/β HDR template containing DNA sequences encoding the TCR alpha variable domain, TCR alpha constant domain, viral 2A self-processing peptide and variable TCR beta domain flanked by 5' and 3' homology arms mapping to the Jurkat VDJβ exon. **B.** Example targeted replacement of the Jurkat VDJβ exon for a transgenic TCRα/β cassette via CRISPR-Cas9 HDR. RNA splicing of the introduced cassette with the endogenous Jurkat TRBC1 (TCR beta constant domain 1) is displayed. **C.** Flow cytometric assessment of CD3 restoration in TnT cells after targeted TCR reconstitution by means of CRISPR-Cas9 HDR. **D.** Validation of targeted TCR reconstitution in TnT cells through genomic PCR of the Jurkat VDJβ locus.

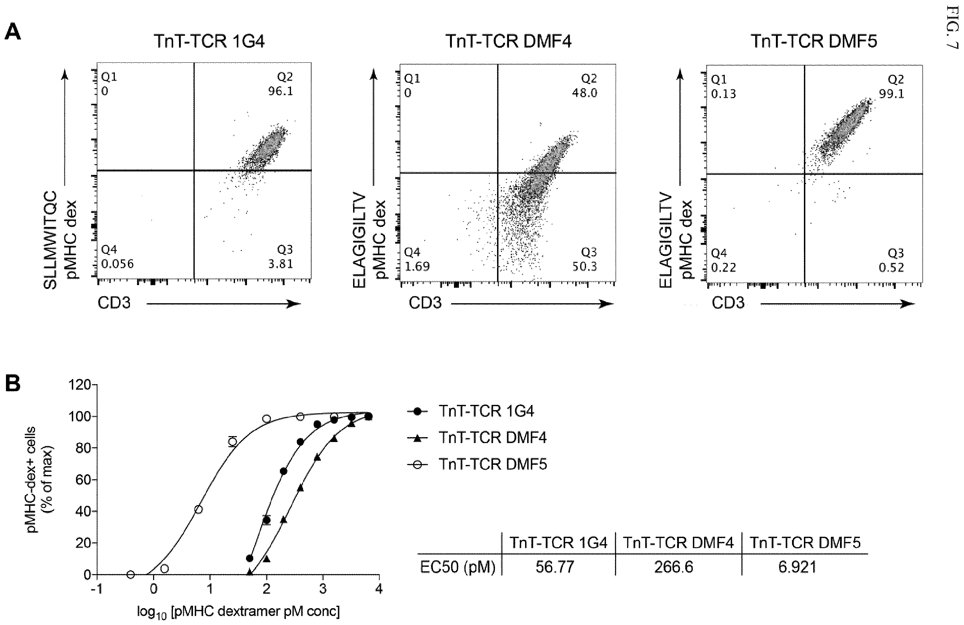

**FIG 7****. A.** Flow cytometric assessment of target peptide-MHC binding in TCR-reconstituted TnT cells. The selected TnT-TCR 1G4 clone recognized fluorescently-labelled SLLMWITQC (SEQ ID NO.15) pMHC dextramer, while selected TnT-TCR DMF4 and TnT-TCR DMF5 clones both bound to fluorescently-labelled ELAGIGILTV (SEQ ID NO.16) pMHC dextramer. **B.** Serial dilutions of target pMHC dextramers were used to assess relative binding avidities in TnT-TCR 1G4, TnT-TCR DMF4 and TnT-TCR DMF5 cells (n = 3). pMHC dextramer concentrations resulting in half-maximal proportions of dextramer positive cells are displayed for each TnT-TCR cell line. Non-linear fits were generated using the GraphPad Prism software: log(agonist) vs. response (three parameters).

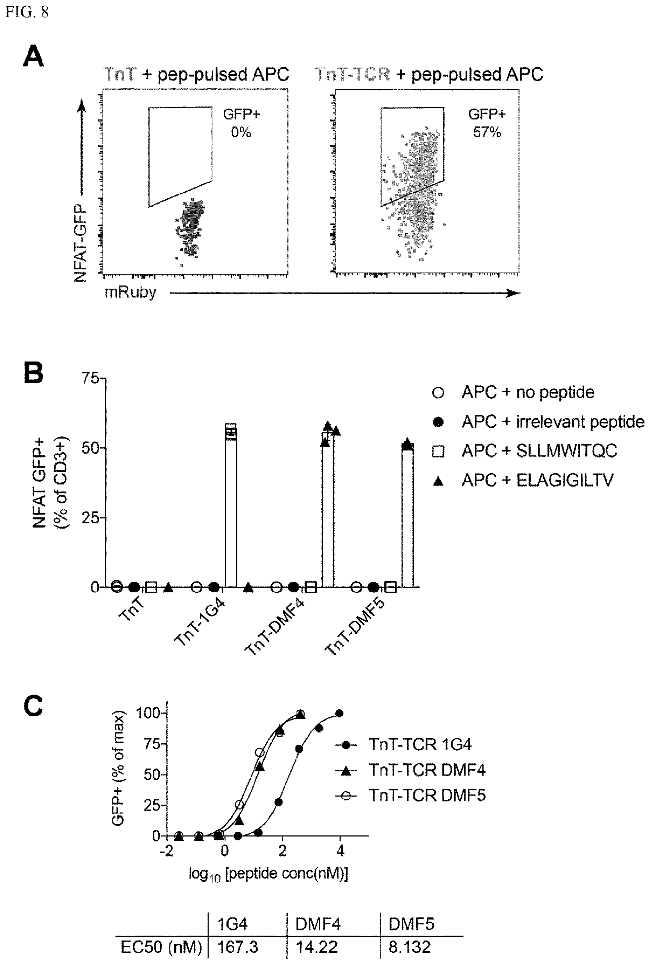

**FIG 8****. A.** Representative flow cytometry dot plots displaying NFAT-driven eGFP expression in TnT-TCR cells (right) but not in TnT cells (left) after overnight co-culture with peptide-pulsed APCs. In this example, TnT-TCR cells express the 1G4 TCR recognizing the NY-ESO-1-derived, HLA*A0201-restricted peptide SLLMWITQC (SEQ ID NO.15). **B.** NFAT-driven expression of eGFP in TnT and TnT-TCR cell lines after co-culture with APCs pulsed with no peptide, irrelevant peptide, mismatched peptide or cognate peptide. In addition to 1G4, TnT cells in this panel were reconstituted with DMF4 and DMF5 TCRs, both recognising the same MART-1-derived, HLA*A0201-restricted peptide ELAGIGILTV (SEQ ID NO.16). C. Normalized NFAT-driven eGFP expression in TnT-TCR cells after overnight co-culture with APCs pulsed with serially-diluted cognate peptide (n = 2). Non-linear fits were generated using the GraphPad Prism software: log(agonist) vs. response (three parameters).

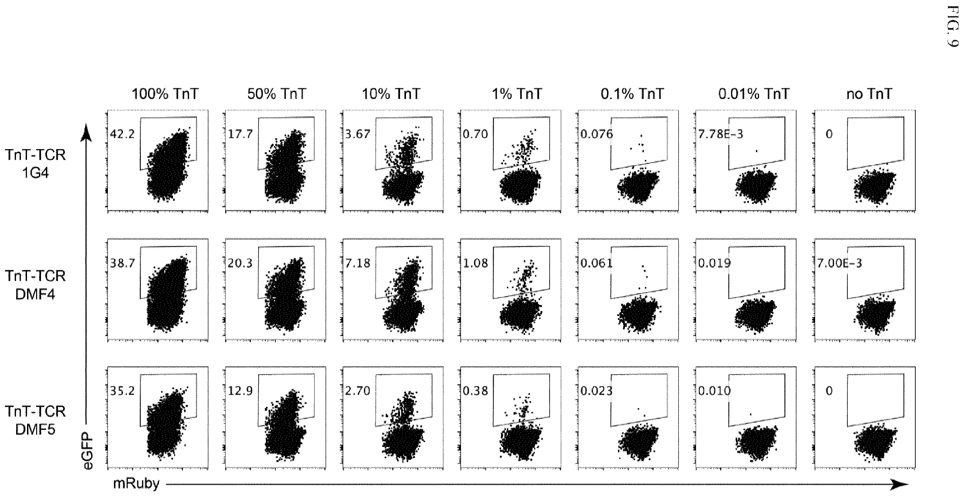

**FIG 9****.** Representative flow cytometry dot plots showing activated TnT-TCR cells at different spike-in proportions following overnight co-culture with peptide-pulsed APCs.

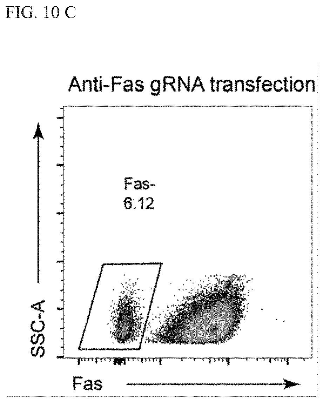

**FIG 10****. A.** Flow cytometric assessment of cell viability reveals significantly increased TnT-TCR cell death after overnight co-culture with APCs pulsed with target peptide (n = 9) relative to controls (n = 27). **B.** Flow cytometric assessment of cell viability showing increased levels of AICD at higher concentrations of pulsed target peptide. TnT-TCR cells in which the death receptor Fas is knocked out display a dramatic reduction in AICD (n = 2). C. Flow cytometric assessment of Fas knockout via Cas9 NHEJ. **D.** Fas knockout in TnT-TCR cells leads to substantial increases in the proportions of activated eGFP+ cells after overnight co-culture with peptide-pulsed APCs (n = 2). Data in panel A were analyzed by unpaired t test with Welch's correction, **** p < 0.0001. Non-linear fits in panels B and D were generated using the GraphPad Prism software: log(agonist) vs. response (three parameters).

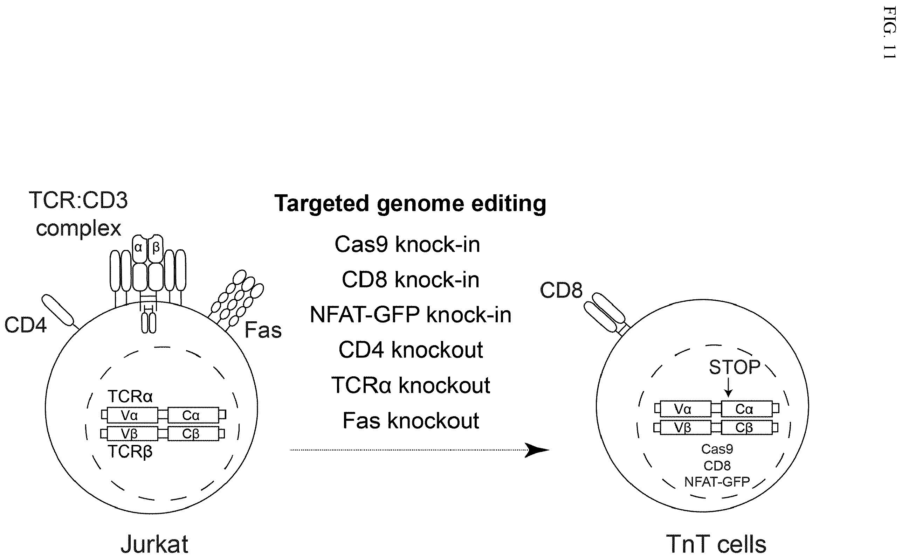

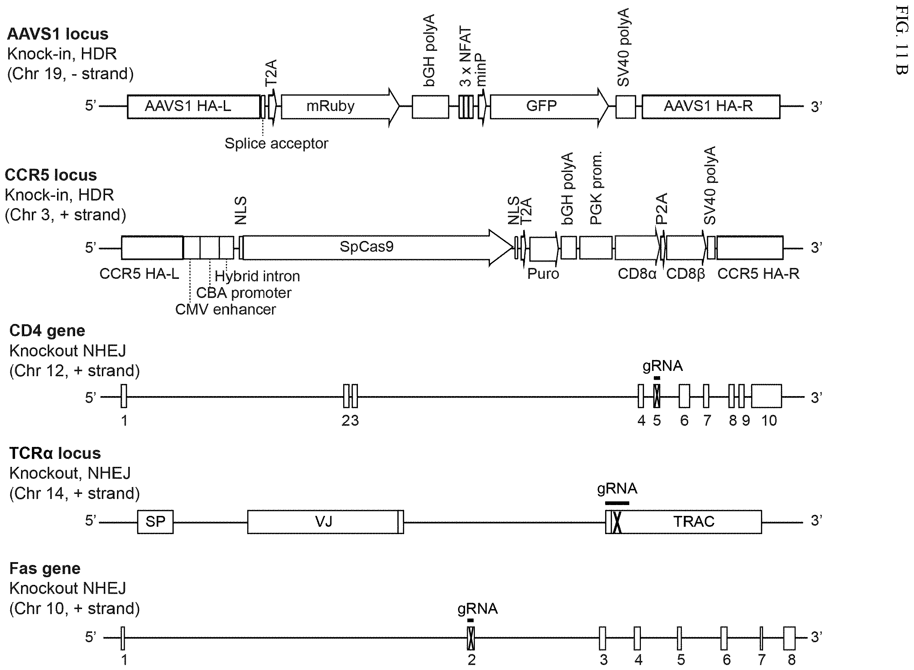

**FIG 11****.** Schematic representation of the multiple Cas9-guided genome editing steps performed for the generation of the TnT platform, as illustrated at the cellular (A) and genetic (B) levels.

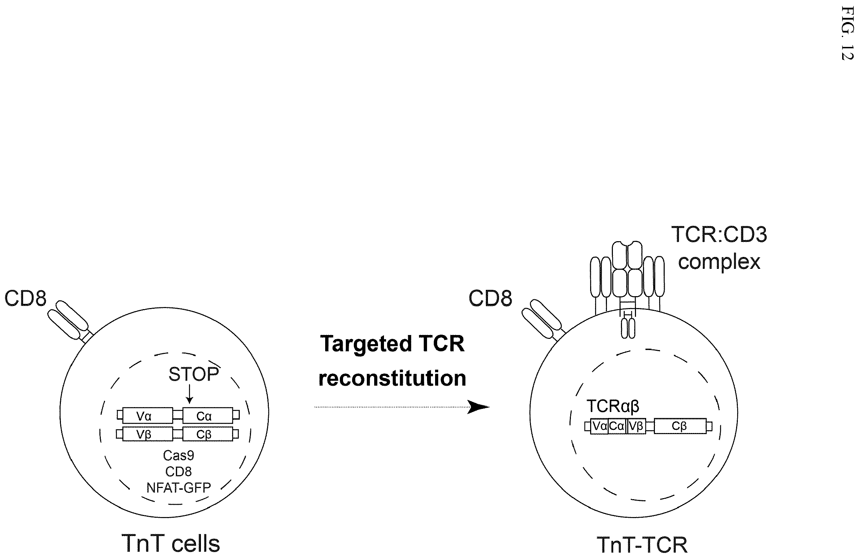

**FIG 12****.** Diagram illustrating CRISPR-Cas9-guided TCR reconstitution of TnT cells (left) for the generation TnT-TCR cells (right).

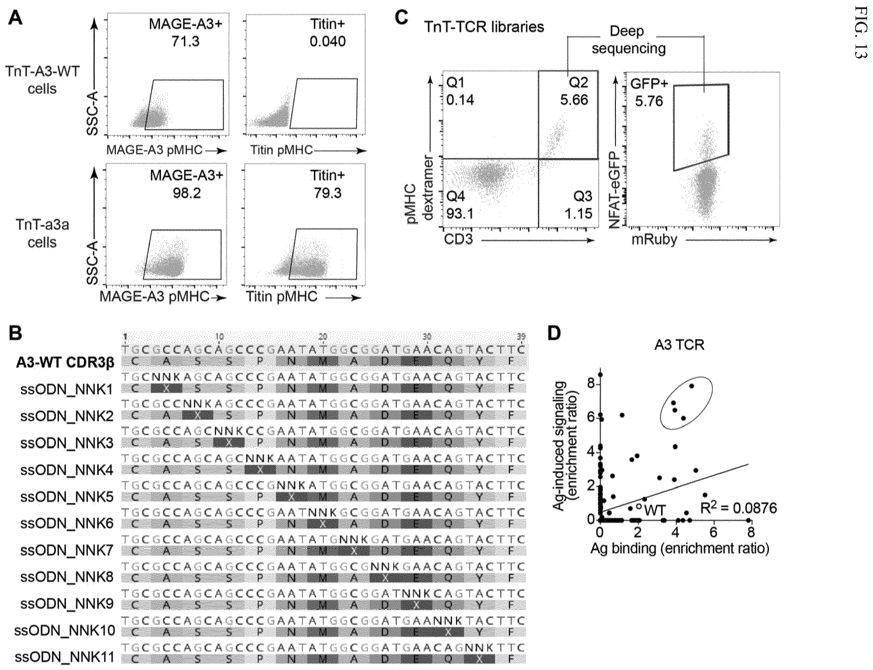

**FIG 13****. A.** Binding of TnT-TCR A3-WT and TnT-TCR a3a cells to MAGE-A3-MHC and Titin-MHC dextramers, as determined by flow cytometry. Only the engineered a3a TCR is able to bind the Titin ESDPIVAQY (SEQ ID NO. 14) off-target. **B.** Deep mutational scanning of the A3-WT TCR CDR3β region (SEQ ID NOs. 7 and 8) using plasmid-based mutagenesis. ssODN_NNK1 (SEQ ID NO. 28 and 29), ssODN_NNK2 (SEQ ID NO. 30 and 31), ssODN NNK3 (SEQ ID NO. 32 and 33), ssODN_NNK4 (SEQ ID NO. 34 and 35), ssODN_NNK5 (SEQ ID NO. 36 and 37), ssODN NNK6 (SEQ ID NO. 38 and 39), ssODN_NNK7 (SEQ ID NO. 40 and 41), ssODN_NNK8 (SEQ ID NO. 42 and 43), ssODN NNK9 (SEQ ID NO. 44 and 45), ssODN_NNK10 (SEQ ID NO. 46 and 47), ssODN_NNK111 (SEQ ID NO. 48 and 49). **C.** Fluorescence-activated cell sorting of TnT-TCR cells based on peptide-MHC dextramer binding (left) or NFAT-eGFP expression after co-culture with target-pulsed APCs (right). Sorted cells are subjected to deep sequencing to identify TCRs enriched in each population. D. Enrichment of A3-WT TCR point-mutants in MAGE-A3-MHC dextramer positive (X-axis) and NFAT-eGFP positive (Y-axis) cell fractions relative to original DMS library frequencies. The A3-WT TCR (no fill) and candidate optimized TCR point-mutants (encircled) are highlighted in the graph.

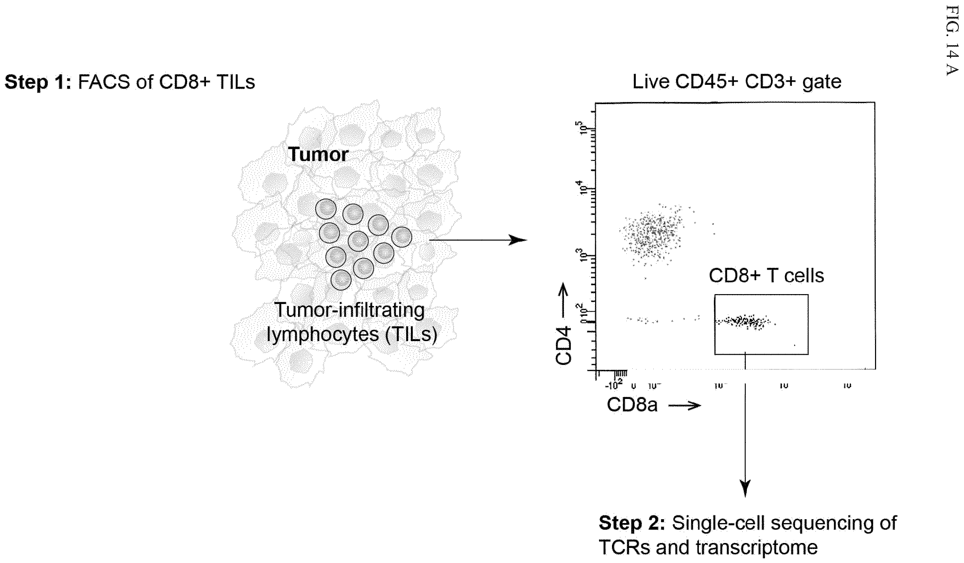

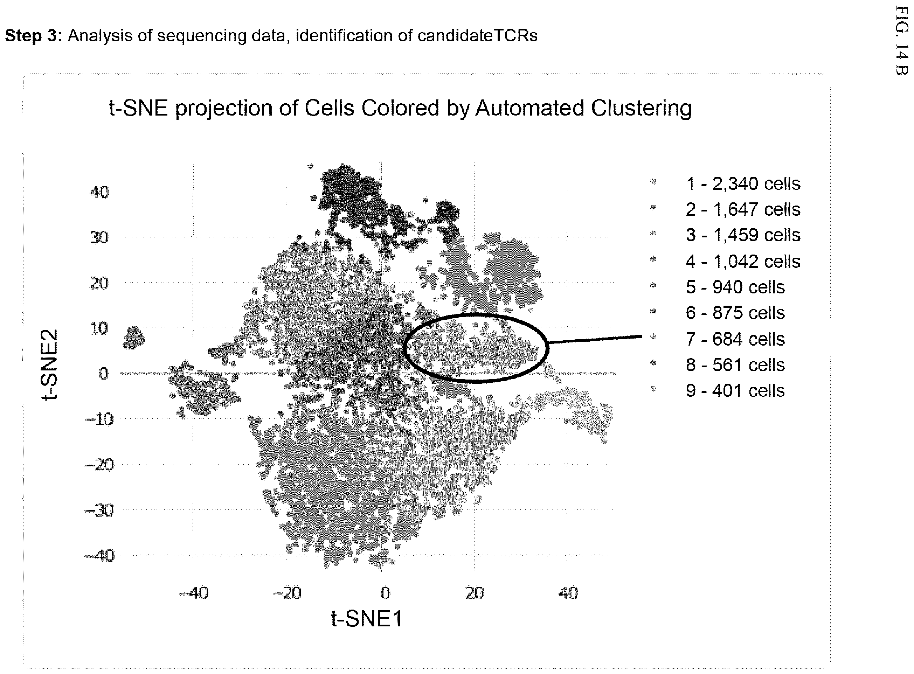

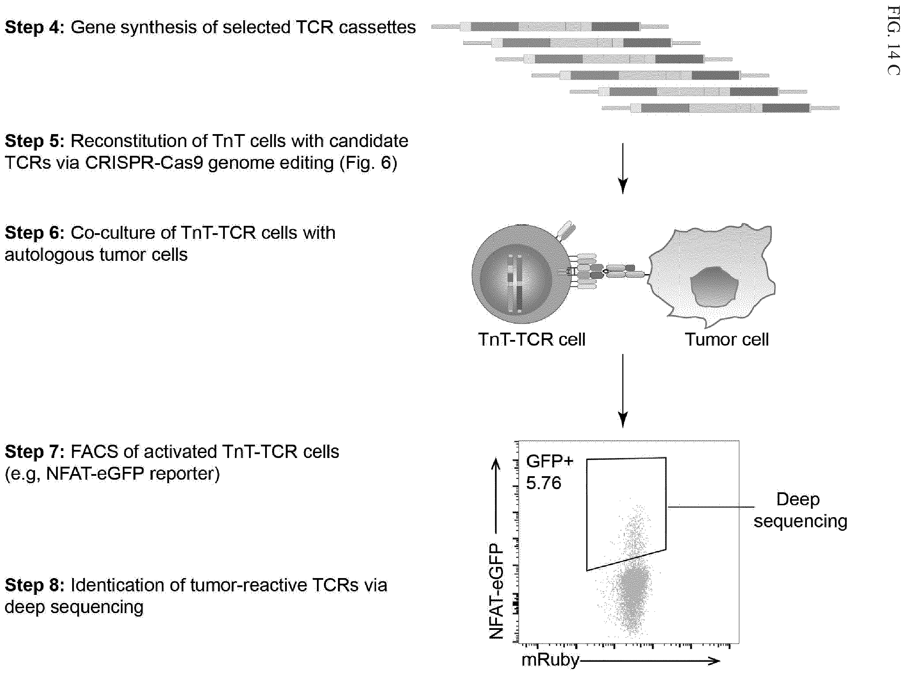

**FIG 14****.** Example workflow for the discovery of tumor-reactive TCRs using the TnT platform.

### EXAMPLES

### Example 1: Material and methods

### Cell Culture

Jurkat T cells (Clone E61, ATCC, TIB152™), Colo205 cells (ATCC, CCL-222™) and T2 cells (DSMZ, #ACC598) were cultured in RPMI-1640 medium (ATCC, #30-2001) with 10 % FBS (Gibco, #16000-044) and 1 % Penicillin-Streptomycin (Gibco, #15140-122). EJM cells (DSMZ #ACC560) were cultured in IMDM (Gibco, #31980-022) supplemented with 10 % FBS (Gibco, #16000-044) and 1 % Penicillin-Streptomycin (Gibco, #15140-122). For prolonged storage, cells were frozen in Bambanker freezing medium (GCLTEC, #BB02) and stored in liquid nitrogen.

### Peptide-MHC dextramer staining

T cells were seeded at 5 x 10⁴ cells per well, washed with FACS buffer (2 mM Ethylenediaminetetraacetic acid (EDTA) (invitrogen, 10135423) 2 % (v/v) FBS (Gibco, #16000-044) in DPBS (Gibco, #14190094)) and stained with serial dilutions of fluorophore-conjugated MHC-dextramers (all from Immudex: HLA-A*0201-SLLMWITQC (SEQ ID NO.15), HLA-A*0201-ELAGIGILTV (SEQ ID NO.16), HLA-A*0101-EVDPIGHLY (SEQ ID NO.11) and HLA-A*0101-ESDPIVAQY (SEQ ID NO.14)). After 10 min at room temperature (RT), additional stains were added for 20 min on ice. Cells were washed with FACS buffer and analyzed by flow cytometry.

### Co-culture assays

For peptide pulsing, APCs (e.g., T2 and Colo 205) cells were washed with serum-free RPMI-1640 and resuspended in serum-free RPMI-1640 containing 5 x 10⁻⁵ - 5 x 10⁻¹² M of peptide (all from Prolmmune: NY-ESO-1: SLLMWITQC (SEQ ID NO. 15), MART-1: ELAGIGILTV (SEQ ID NO. 16), MAGE-A3: EVDPIGHLY (SEQ ID NO.11), Titin: ESDPIVAQY (SEQ ID NO. 14), CMV: VTEHDTLLY (SEQ ID NO. 17))) at a final density of 1x10⁶ cells/mL. Peptide pulse was allowed for 2 h at 37° C. APCs cells were washed and resuspended at 10⁶ cells/mL. 1 x 10⁵ Jurkat cells and 5 x 10⁴ peptide-pulsed APCs cells were seeded into wells of a 96-well plate in a final volume of 150 µL complete medium containing 1 µg/mL anti-CD28 (BioLegend, #302933). For spike-in assays, TnT cells were mixed with ROD15 cells in frequencies ranging from 0.01 % to 100 %, and 1 x 10⁵ cells added into wells containing T2 cells (2:1 ratio). After overnight culture, cells were analyzed by flow cytometry. Positive controls consisted of TnT cells stimulated overnight in the presence of 10 µg/mL plate-bound anti-CD3 (OKT3, #317326) or 1x PMA/Ionomycin (∼1.4 µM, Invitrogen, #00497093).

### Cloning of TCR cassettes

TCR cassettes encoding a TCR alpha variable domain, a TCR alpha constant domain and a TCR beta variable domain were generated by custom gene synthesis (Twist Bioscience). Jurkat TCRβ VDJ 5' and 3' homology arms were introduced into the pTwist vector (Twist Bioscience) using XhoI-MluI restriction sites to generate the pJurTCRβ vector. For TCR cloning, TCR inserts and pJurTCRβ vector were digested with XbaI and BsaI restriction enzymes for 1 h at 37° C. Vector 5' ends were dephosphorylated by addition of 1 µL calf intestinal phosphatase (CIP) for a further 30 min. The digested vector and inserts were gel purified (ZymoClean Gel DNA Recovery Kit, Zymo Research, #D4001) and ligated using T4 ligase (NEB, #M0202F) at a molar ratio of 3:1 insert-to-vector for 2 h at RT. Ligation mixes were transformed into NEB5α bacteria (NEB, #C2988J) according to manufacturer's instructions. Positive bacterial clones were identified by colony PCR using the KAPA2G kit (KAPA Biosystems, #KK5020), primers RVL-127 5'-GCATGCCTCTGTGCCAACAG-3' (SEQ ID NO.18) and RVL-128 5'-TTTTATCTGTCATGGCCGTGACCG-3 (SEQ ID NO.19) and subsequent sequencing. Cloning of gene cassettes for use in other CRISPR-Cas9 genome editing experiments (e.g., Cas9 knock-in, CD8 knock-in, NFAT-eGFP knock-in) was performed in a similar manner as described above, but with different restriction enzymes and vectors.

### TCR reconstitution via CRISPR-Cas9 genome editing

HDR templates were generated by PCR amplification of TCR cassettes flanked by Jurkat TCRβ VDJ homology arms using primers RVL-127 and RVL-128 (SEQ IDs NOs 18 and 19). Cell transfection was performed using electroporation (SE Cell Line Solution Box, Lonza, #PBC1-02250) with the "CK 116" protocol on the 4D-Nucleofector (Lonza). For genome editing, 200 µM tracrRNA and 200 µM crRNA were mixed at equimolar concentration to form the gRNA complex. For HDR, 1.5 µg PCR-amplified HDR templates and 700 pmol of gRNA were used per transfection. Following transfection, cells were cultured for 16 h in complete media containing 30 µM HDR enhancer (Integrated DNA Technologies). Cells were then washed and resuspended in complete media in order to remove the HDR enhancer. At seven days post-transfection, cells with restored CD3 expression were single cell sorted and expanded for 2-3 weeks. Finally, clonal TnT-TCR populations were characterised by flow cytometry, PCR and Sanger sequencing. Other CRISPR-Cas9 genome editing experiments (e.g., Cas9 knock-in, CD8 knock-in, CD4 knockout, NFAT-eGFP knock-in, Fas knockout) were performed in a similar manner as described above, but with different HDR templates (for knock-in experiments) and crRNAs.

### crRNA sequences

CRISPR-RNA (crRNA) reagents targeting the CCR5 (SEQ ID NO. 20: 5'-TGACATCAATTATTATACAT-3'); eGFP (SEQ ID NO. 21: 5'-CAACTACAAGACCCGCGCCG-3'); AAVS1 (SEQ ID NO. 22: 5'-GGGGCCACTAGGGACAGGAT-3'); CD4 (SEQ ID NO. 23: 5'-GCACTGAGGGGCTACTACCA-3'); TRAC (SEQ ID NO. 24: 5'-CAGGGTTCTGGATATCTGT-3'); Jurkat CDR3β (SEQ ID NO. 6: 5'-TCGACCTGTTCGGCTAACTA-3'); and Fas (SEQ ID NO. 25: 5'-TTGGAAGGCCTGCATCATGA-3') loci were purchased from IDT. The sequences above reflect the DNA template used to design the crRNA constructs.

### Genotyping of TnT-TCR clones

Genomic DNA was extracted using the QuickExtract solution kit (Lucigen, #0905T). RNA isolation was performed using the TRIZol reagent and PureLink RNA Mini Kit (Invitrogen, #12183025). For cDNA synthesis, 1 µL Oligo dT, 1 µL dNTPs and 5 µL of RNA were annealed for 5 min at 65° C. 5X RT buffer, RiboLock and MaximaRT (all ThermoScientific) were added, incubated at 50° C for 30 min and inactivated at 85° C for 5 min. Genomic and cDNA PCRs were performed using the KAPA HiFi PCR kit (KAPA Biosystems, #KK2101). See appendix for primer sequences.

### Flow Cytometry and Cell Sorting

For flow cytometric analysis of surface antigens, cells were washed with FACS buffer and stained with appropriate fluorophore-conjugated antibodies (all BioLegend: CD3-APC (#300485), CD3-PE/Cy7 (#300420), CD69-APC (#310910), CD8-APC (#300912), CD95-PE/Cy7 (#302216)) and DAPI (ThermoScientific, #62248) for 20 min on ice. Cells were washed twice in FACS buffer, and fluorescence was measured using the Cytoflex S (Beckman Coulter) or LRSFortessa (BD Biosciences, custom-made) flow cytometers. FACS was performed on the Aria III instrument (BD Biosciences). T2 cells were incubated in 1:50 dilutions of TruStain FcX (BioLegend, #422301) for 10 min prior to staining.

### Generation of TCR deep mutational scanning (DMS) libraries

DMS targeted to the TCRβ CDR3 region was performed using a plasmid-based approach (Wrenbeck, E.E., et al. (2016) Nature methods 13, 928-930.). Single-stranded DNA oligonucleotides (ssODNs) with single-position NNK degenerate codons tiled across the CDR3β region were designed in-house and purchased from IDT. These ssODNs were then used to re-synthesize a plasmid containing the A3-WT TCR cassette according to the method of Wrenbeck et al. 2016. After completion of the protocol, deep sequencing (Amplicon-EZ, GENEWIZ) was performed to verify that the resulting plasmid preparations contained all of the desired point-mutations.

### Deep sequencing of transgenic TCRs

RNA was isolated from TnT-TCR cells and cDNA was generated as described above. The region flanking the TCR CDR3β was then amplified by PCR using primers RVL-144 5'-GAGGAGAACCCTGGACCTATG-3' (SEQ ID NO.26) and RVL-145 5'-GGAACACCTTGTTCAGGTCCTC-3' (SEQ ID NO.27), followed by gel extraction of PCR products and submission for analysis using the Amplicon-EZ service (GENEWIZ).

### Example 2: Generation of Cas9+ CD8+ Jurkat cells

The development of the TnT platform required extensive, multi-step CRISPR-Cas9-guided genome editing. Using the wild-type Jurkat E6.1 T cell line as a starting point, several genetic components were sequentially edited in order to facilitate the introduction and functional screening of antigen-specific TCRs. Each genome editing step consisted of transfection of gene-targeting gRNAs followed by fluorescence-activated cell sorting (FACS), expansion and clone selection. First, to simplify and increase genome editing efficiency, a gene encoding the Cas9 endonuclease was introduced via HDR into the CCR5 safe harbor locus. Within the same construct, but under the control of a different constitutive promoter, a sequence encoding enhanced green fluorescent protein (eGFP) was included to allow for FACS of modified cells (Figure 1A). Transfection of anti-GFP gRNA was used to confirm transgenic Cas9 activity in expanded eGFP+ clones (Figure 1B). Next, the eGFP gene present in the resulting cell line was replaced for a cassette encoding the two chains of human CD8 via Cas9 HDR (clone ROD6, Figure 2). Finally, the endogenous Jurkat CD4 co-receptor was knocked out via Cas9-induced NHEJ to generate a CD8+ / CD4- cell line thus allowing for TCR screening against MHC class I-restricted peptides (clone ROD10, Figure 3).

### Example 3: Introduction and characterization a fluorescent reporter of TCR signaling

The introduction of a fluorescent reporter of TCR signalling aimed to equip Cas9+ CD8+ Jurkat cells with the ability to support functional TCR screening. The selected reporter system consisted of an eGFP gene under the control of three tandem NFAT (nuclear factor of activated T cells) binding sites derived from the human IL-2 promoter (Macian, F., et al. (2001) Oncogene 20, 2476-2489; Mattila, P.S., et al. (1990) EMBO J 9, 4425-4433) (Figure 4A). Accordingly, a dual gene HDR template was designed to incorporate sequences encoding mRuby and NFAT-eGFP into the safe harbor AAVS1 locus (Figure 4A). The inventor's design allowed for mRuby to serve as a reporter of HDR under the control of the endogenous PPP1R12C promoter. Following transfection, mRuby+ clones were isolated by FACS and expanded (Figure 4B). Stimulation of selected clones with anti-CD3 antibody followed by flow cytometric assessment of eGFP expression was then used to identify successfully edited clones (Figure 4C). Targeted genomic integration of the NFAT-eGFP reporter in the selected clone ROD15 was confirmed by PCR from genomic DNA and Sanger sequencing (Figure 4D). The engineered Jurkat cell line was thus Cas9+ CD8+ mRuby+ and contained the NFAT-eGFP reporter at this stage of development.

### Example 4: Disruption of the endogenous Jurkat TCR

The use of primary T cells for the display of transgenic TCRs can be limited by TCR chain mispairing. In order to avoid this possibility in this platform, the endogenous Jurkat TCRα chain was targeted for Cas9-mediated NHEJ. To achieve this, the Jurkat cells engineered above were transfected with a gRNA targeting the first exon of the TCR alpha constant (TRAC) domain (Eyquem, J., et al. (2017) Nature 543, 113-117). As failure to express a functional TCRα chain prevents the formation and surface display of the CD3-TCR complex, TCRα knockout efficiency was assessed by flow cytometric analysis of CD3 surface expression. Accordingly, cells transfected with anti-TRAC gRNA displayed a substantial proportion of CD3⁻ cells (>65%), which were then isolated by FACS (Figure 5A). RT-PCR amplification of the TRAC region in selected clones revealed that three clones contained premature stop codons in the vicinity of the gRNA target site (Figure 5B). From these experiments, clone ROD20 was selected for further development (referred to as TnT cells from here onwards).

### Example 5: Reconstitution of TnT cells with tumor-specific TCRs

As a proof-of-concept, TnT cells were reconstituted with three TCRs previously explored for TCR gene therapy: TCR 1G4, which recognizes NY-ESO-1 (56), and TCRs DMF4 and DMF5, which both recognize MART-1 (52,82). For this purpose, gene cassettes encoding TCRα variable and constant domains, a self-processing T2A peptide and a TCRβ variable domain were designed for each 1G4, DMF4 and DMF5. These TCR cassettes were cloned into a vector containing homology arms mapping to the Jurkat TCRβ VDJ exon (Figure 6A). Crucially, a splice donor sequence was maintained at the 3' end of the constructs to allow for splicing with the endogenous Jurkat TCRBC1 gene. For targeted genome editing, TnT cells were co-transfected with a gRNA targeting the endogenous Jurkat TCRβ complementarity determining region 3 (CDR3β) and HDR templates containing TCR cassette and homology arm sequences in PCR product format (Figure 6B).

Integration of transgenic TCRs was assessed by restoration of CD3 expression and pMHC dextramer staining, indicating HDR efficiencies of 0.9% to 2.1%. Notably, development of an enhanced transfection protocol has led to HDR efficiencies of up to 20% for the same constructs (Figure 6C). After transfection, CD3+ cells were single cell sorted and expanded. Six clones of each TnT-TCR were further characterized by RT-PCR and flow cytometry. One lead clone per TnT-TCR was selected based on CD3 expression, CD8 expression, pMHC-dextramer binding and eGFP expression after stimulation with PMA-ionomycin. Correct integration and splicing of the DNA cassettes in selected TnT-TCR clones was confirmed by PCR of their genomic VDJβ locus (Figure 6D) and Sanger sequencing. Collectively, these experiments demonstrate the successful reprogramming of TnT cells with anti-tumor TCRs that specifically recognize their cognate pMHC.

### Example 6: Assessment of TnT platform functionality

Once tumor-reactive TCR reporter cell lines had been developed, their functionality in response to antigen was validated. To assess the binding avidity of 1G4, DMF4 and DMF5 TCRs, TnT-TCR cells were stained with serial dilutions of cognate fluorescently-labelled pMHC-dextramer, and binding was measured by flow cytometry (Figure 7A). The binding avidity of each TCR was determined by calculating the EC50 of the normalized dextramer saturation curves. DMF5 was the most highly avid TCR, followed by 1G4 and DMF4. Notably, the EC50 for DMF5 was 39 times lower than that for DMF4, underlining its substantially higher avidity for the MART-1 ELAGIGILTV (SEQ ID NO.16) peptide (Figure 7B).

To assess TCR function, co-culture assays of TnT-TCR cells and peptide-pulsed T2 cells were performed (Figure 8A). As controls, TnT-TCR clones were co-cultured with T2 cells pulsed with no peptide or 10 µg/mL mismatched peptide. This experiment showed that TnT-TCR cells were highly specific for their cognate peptide (Figure 8B). TCR function was quantified as the peptide concentration inducing half-maximal frequencies of eGFP+ cells. 1G4 displayed the highest EC50 value and thus the lowest functional avidity, followed by DMF4 and DMF5. In contrast to their 39-fold difference in binding to pMHC, the difference between DMF4 and DMF5 in terms of function was substantially reduced (< 2-fold, Figure 8C). The readout sensitivity of the TnT platform was assessed by means of a spike-in assay. To this end, TnT-TCR cells were spiked into cell suspensions of TCR-negative Jurkat cells at decreasing frequencies and co-cultured with peptide-pulsed T2 cells. Spiked-in TnT-TCRs were readily detected through eGFP expression after co-culture, and activated TnT-TCR cells were identified at frequencies as low as 0.01 % (Figure 9).

### Example 7: Development of TnT cells resistant to activation-induced cell death

Significant increases in TnT-TCR cell death after co-culture with APCs pulsed with target peptide relative to non-target peptide controls were observed (Figure 10A). Similar to expression of the eGFP reporter (Figure 8C), cell death correlated directly with the levels of pulsed target peptide (Figure 10B), thus suggesting AICD of TnT-TCR cells. As AICD is primarily mediated by Fas and Fas-L (Alderson, M.R., et al. (1995) The Journal of experimental medicine 181, 71-77.), the Fas death receptor was targeted in TnT-TCR cells for knockout via CRISPR-Cas9 NHEJ. For this purpose, the previously established TnT-1G4, TnT-DMF4 and TnT-DMF5 cell lines were transfected with a gRNA targeting exon 2 of the Fas gene (Figure 10C). Following transfection, CD3+ Fas- cells were sorted in bulk and used to set up co-culture assays with peptide-pulsed T2 cells. Flow cytometric analysis revealed a remarkable increase in eGFP+ cells as a proportion of total cells for all TnT-TCR cell lines (Figure 10D). This was the result of a substantial decrease in the proportion of dead cells relative to their Fas+ counterparts (Figure 10B). Based on these results, a Fas-knockout TnT clone was established as the lead cell line. As such, the TnT platform is currently defined as a Cas9+, CD8+, mRuby+, NFAT-eGFP-containing, CD3- and Fas- cell line (Figures 11A and 11B) that becomes CD3(TCR)+ upon targeted TCR reconstitution (Figure 12).

The TnT platform has been designed to support high-throughput functional discovery and engineering of TCRs with therapeutic potential. The examples below illustrate the applicability of the TnT platform for such purposes.

### Example 8: Engineering of TCRs with enhanced affinity to a melanoma antigen and with no cross-reactivity to a known off-target antigen

A first example is the engineering of a previously discovered TCR with specificity towards the cancer-testis (C/T) antigen MAGE-A3, a target expressed on as many as 75% of melanomas. In particular, the aim is to enhance TCR affinity towards MAGE-A3 while simultaneously eliminating cross-reactivity to the known off-target antigen Titin (expressed on cardiomyocytes). The C/T MAGE-A3 antigen was originally identified as a lead candidate for immunotherapy applications based on the fact that it is commonly overexpressed in several types of tumors but not present in healthy tissues other than the immune-privileged testis. However, naturally-occurring anti-MAGE-A3 TCRs display low affinities to their target, as high-affinity TCRs recognizing MAGE-A3 are likely eliminated during thymic selection. This applies to the parental anti-MAGE-A3 TCR (A3-WT, SEQ ID NOs 9 and 10) (utilized in the experiments described below) which is a low-avidity TCR originally isolated from a patient vaccinated with the MAGE-A3 HLA-A*0101-restricted peptide EVDPIGHLY (SEQ ID NO. 11). Importantly, the A3-WT TCR has been previously engineered for high-affinity binding to its target using phage display, resulting in the a3a TCR (SEQ ID NOs 12 and 13). A clinical trial in patients with myeloma and melanoma using the engineered a3a TCR resulted in unexpected and fatal cardiac toxicity in two patients. Cross-reactivity to a peptide derived from Titin (ESDPIVAQY, SEQ ID NO. 14) was identified retrospectively as causal to cardiac toxicity and suggests that high-affinity engineering of A3-WT TCR contributed to this effect. In order to confirm this observation, clones expressing the A3-WT and a3a TCRs have been generated using the TnT platform. In these experiments, it was confirmed that the a3a TCR, but not the A3-WT TCR, displays cross-reactivity to Titin (Figure 13A).

### Generation and screening of positional scanning libraries of anti-MAGE-A3 TCR

The first step towards engineering the A3-WT TCR consist of performing deep mutational scanning (DMS) of the complementarity-determining region 3 (CDR3) of its TCRβ chain (Figure 13B). Importantly, this TCR region typically mediates several contacts to peptide antigen and minimal contacts to MHC. DMS is performed by generating single-position saturation mutagenesis using a plasmid-based approach (Wrenbeck, E.E., et al. (2016) Nature methods 13, 928-930). Briefly, pooled single-stranded oligonucleotides (ssODNs) that have single-position degenerate codons (e.g., NNK) tiled across the CDR3β are used to re-synthesize a plasmid containing the A3-WT TCR cassette. Next, plasmid-based DMS libraries are used for the generation of homology-directed repair (HDR) templates via PCR. Such HDR templates, which contain homology arms mapping to the endogenous TnT TCRβ VDJ exon, are then used to reconstitute TnT cells with generated TCR variants via CRISPR-Cas9 HDR (Figure 6B). Transfected TnT cells are initially bulk-sorted via flow cytometry in order to select for cells that re-gain CD3 expression (indicates successful HDR) and show no binding to Titin-MHC multimers (negative selection step). Next, cells are expanded and re-sorted based on: (i) binding to MAGE-A3-MHC multimers and, (ii) NFAT-eGFP expression upon co-culture with APCs displaying MAGE-A3 peptide. Deep sequencing of TCR amplicons is then performed to identify TCR variants that are enriched in the MAGE-A3-binding and MAGE-A3-signaling fractions, respectively (Figures 13C and 13D).

### Selection of affinity-enhanced anti-MAGE-A3 TCRs devoid of Titin cross-reactivity from combinatorial libraries

Having determined the binding and functional activation sequence landscape of the A3-WT TCR, this information is used to engineer TCR variants with increased affinity and potency towards the MAGE-A3 EVDPIGHLY (SEQ ID NO.11) peptide. For this purpose, combinatorial mutagenesis libraries are designed in order to maximize the number of TCR variants that can be effectively screened in the TnT platform (Figure 12D). A current limitation of mammalian cell display technologies is reduced library size relative to display platforms based on yeast or phage. However, by performing DMS studies, it was demonstrated that generation of optimally-designed libraries can effectively overcome this limitation. In line with this, combinatorial libraries of the A3-WT CDR3β region are generated by identifying degenerate codons that mimic the amino acid enrichment ratios observed in DMS experiments. These libraries are custom made as ssODNs and transformed into double-stranded DNA (dsDNA) via PCR. Then, dsDNA is restriction-cloned into a plasmid containing homology arms mapping to the endogenous TnT TCRβ VDJ exon. As with DMS libraries, HDR templates are generated via PCR and transfected into TnT cells alongside an anti-TCRβ gRNA for TCR reconstitution. Cells that regain CD3 expression and do not bind to Titin-MHC are then bulk-sorted via flow cytometry and expanded. Next, cells displaying high levels of MAGE-A3 binding and signaling are isolated by flow cytometry and subjected to TCR deep sequencing. As a final negative selection step, the isolated cells are expanded and co-cultured with HLA-A*0101+ primary human skeletal muscle myoblasts (HSMM), which express high levels of Titin. Cells expressing NFAT-eGFP in this assay are subjected to TCR deep sequencing and excluded from further development.

### Example 9: Functional discovery of tumor-reactive TCRs from patient-derived libraries

Another example is the generation of a comprehensive catalogue of TCR sequences found in CD8+ tumor-infiltrating lymphocytes (TILs) of patients with different types of cancer, e.g., non-small cell lung cancer (NSCLC) or epithelial ovarian cancer (EOC). Single-cell deep sequencing of TCRs originating of these human TILs is then performed using the Chromium Single Cell Immune Profiling system (10x Genomics). First, bulk CD8+ T cells from tumor biopsies or resections are immunophenotyped and isolated by FACS. Isolated cells are then processed into single cell gel emulsions containing components required for reverse transcription of mRNA and barcoding of produced cDNA. Barcoding of cDNA molecules allows for the identification of sequences originating from single T cells, and consequently for the *in silico* reconstruction of naturally occurring TCRα/β pairings. Furthermore, it allows for transcriptomic analysis at the single cell level. Barcoded cDNA is then pooled and utilized for the generation of both gene expression and TCR sequencing libraries. Finally, both libraries are sequenced using the MiSeq or Next Seq platforms (Illumina) and the resulting data analyzed using the Cell Ranger software (10x Genomics).

Analysis of sequenced TCR and transcriptome data from TIL samples allows to determine the frequencies and gene expression profiles of individual CD8+ TILs. This information is then utilized to identify candidate tumor-reactive TCRs in a given patient based on their abundance and the expression of key exhaustion/activation markers. Utilizing this approach, the inventors have profiled and selected candidate tumor-reactive TCRs originating from single cell sequencing of TILs isolated from a squamous cell lung carcinoma tumor (Figures 14A, 14B and 14C). Accordingly, a library of the top-ranked TCRα/β genes (∼ 200 most likely to be tumor-specific clones) has been generated by gene synthesis for its introduction into engineered TnT cells using Cas9-assisted exchange (Figure 6B). Successfully reconstituted cells are then isolated by FACS based on restored CD3 expression. Next, transformed TnT cells are co-cultured with autologous patient tumor cells, followed by monitoring of NFAT-eGFP reporter expression in order to identify cells displaying tumor-reactive TCRs. Finally, sequencing of such tumor-reactive TCRs is performed after PCR amplification of the engineered TCRβ TnT locus.

The recent description of 'outsourced' antitumor immune responses also justifies the development of large TCR display libraries from naive donor T cells. It was reported that donor naive T cell pools contain T cell clones capable of recognizing tumors from a different individual. Thus, in addition to the construction of TIL TCR libraries, a secondary and more technically challenging goal is to generate TCR display libraries from bulk naive T cells (requirement of in-cell PCR for generation of TCRα/β genetic fusions).

While the approach allows for the identification of patient-specific tumor-reactive TIL TCRs, it also has the potential to be expanded by pooling several TCR libraries originating from individual patients (derived from TILs or naive T cells) in order to screen tumors from unrelated patients. Development of such a TCR display platform would represent a significant technological advance for the efficient and reliable identification of tumor-reactive TCRs for future use in personalized TCR gene therapies.

## Claims

1. An isolated cell derived from a T cell, the cell comprising one or more reporter systems, wherein the endogenous T cell receptor (TCR) alpha gene locus and/or beta gene locus of the cell is modified such that surface expression of a TCR-CD3 complex is disrupted.

2. The cell according to claim 1, wherein the isolated cell is derived from a Jurkat T cell.

3. The cell according to any one of claims 1 or 2, wherein the cell further comprises a co-receptor, in particular wherein the co-receptor is encoded by genes that are operably linked to their corresponding promoter or to a recombinant promoter,
in particular wherein the co-receptor is CD4 or CD8.

4. The cell according to any one of claims 1 to 3, wherein the one or more reporter system comprises a polynucleotide comprising a promoter region operably linked to a polynucleotide sequence encoding a detectable marker,
in particular wherein the promoter region comprises one or more transcriptional response elements,
in particular wherein at least one of the one or more transcriptional response elements is the nuclear factor of activated T cell (NFAT) response element,
in particular wherein the detectable marker is a fluorescent protein.

5. The cell according to any one of claims 1 to 4, wherein expression and/or activity of at least one receptor involved in programmed cell death or T cell exhaustion is reduced or disrupted,
wherein the receptor involved in programmed cell death or T cell exhaustion is a Fas receptor, TNFR2 and/or PD-1, in particular, wherein the receptor is a Fas receptor; and/or
wherein expression and/or activity of at least one protein involved in antigen presentation is reduced or disrupted, in particular wherein the protein is beta-2 microglobulin, and/or
wherein expression and/or activity of at least one protein promoting non-homologous end joining is reduced or disrupted, in particular wherein the protein is p53 or p53-binding protein 1, and/or
wherein expression and/or activity of at least one protein promoting homology directed repair is increased, in particular wherein the protein is BRCA1.

6. The cell according to any one of claims 1 to 5, wherein the cell comprises a gene encoding a recombinant endonuclease,
in particular wherein the recombinant endonuclease is *Streptococcus pyogenes* Cas9.

7. A method for producing a cell expressing a TCR-CD3 complex on the cell surface, the method comprising the steps of:
a) providing a cell according to any one of claims 1 to 6;
b) introducing a DNA fragment comprising coding sequences encoding a TCR alpha variable domain, a TCR beta variable domain and at least one of a TCR alpha constant domain and/or a TCR beta constant domain into the genome of the cell of step (a);
c) expressing the coding sequences encoded on the DNA fragment introduced in step (b); and
d) obtaining a cell expressing a TCR-CD3 complex on the cell surface.

8. The method according to claim 7, wherein the DNA fragment of step (b) further comprises homology arms at its 5' and 3' ends that are homologous to the endogenous TCR alpha locus or the endogenous TCR beta gene locus of the cell,
in particular wherein the DNA fragment further comprises a splice donor site that is located downstream of the most 3' coding sequence of the DNA fragment and upstream of the 3' homology arm.

9. The method according to claim 8, wherein the DNA fragment is introduced into the genome of the cell such that the coding sequences located on the DNA fragment replace the endogenous TCR alpha VJ exon or the TCR beta VDJ exon and are transcriptionally linked to the endogenous gene encoding the TCR alpha constant domain or the TCR beta constant domain, respectively,
in particular wherein the DNA fragment is introduced into the genome of the cell through CRISPR-Cas9-mediated homology-directed repair.

10. A library of DNA fragments, wherein the library comprises DNA fragments comprising a coding sequence encoding a TCR alpha variable domain, a coding sequence encoding a TCR beta variable domain and at least one of a coding sequence encoding a TCR alpha constant domain and/or a coding sequence encoding a TCR beta constant domain,
in particular wherein the coding sequences encoding the TCR alpha variable domain, the TCR beta variable domain and at least one of the TCR alpha constant domain and/or the TCR beta constant domain have been obtained by sequencing TCR genes in a population of T cells, in particular, wherein the population of T cells comprises tumor-infiltrating lymphocytes; or
wherein at least one DNA fragment in the library comprises one or more mutations in at least one coding sequence encoding a TCR alpha variable domain, a TCR beta variable domain and at least one of a TCR alpha constant domain and/or a TCR beta constant domain compared to a DNA fragment with a known sequence.

11. A method for determining the potential of a cell expressing a T cell receptor to bind to and/or to be activated by an antigen, the method comprising the steps of:
a) producing a cell expressing a T cell receptor using the method according to any one of claims 7 to 9;
b) contacting the cell of step (a) with an antigen; and
c) determining the potential of the cell to bind to the antigen and/or to be activated by the antigen,
in particular wherein the cell is determined to bind to the antigen, if the binding between the cell and the antigen is stronger compared to the binding between the cell and an irrelevant antigen, or, if the binding between the cell and the antigen is stronger compared to the binding between the antigen and a cell that expresses an irrelevant or no T cell receptor;
and/or
wherein the cell is determined to be activated by the antigen, if the activation of the cell by the antigen is higher compared to the activation of a cell that has been contacted with an irrelevant or no antigen, or, if the activation of the cell by the antigen is higher compared to the activation of a cell that expresses an irrelevant or no T cell receptor and has been contacted with the antigen.

12. A method for identifying at least one T cell receptor with optimized antigen-binding or antigen-induced signaling properties, the method comprising the steps of:
a) producing a plurality of cells using the method according to any one of claims 7 to 9, wherein the plurality of cells is produced by introducing the library of DNA fragments according to claim 10 into said cells;
b) contacting the plurality of cells of step (a) with a target antigen or with one or more non-target antigens;
c) isolating at least one cell from the plurality of cells, in which the binding between the cell and the target antigen and/or in which the activation of the cell by the target antigen is increased compared to a cell that expresses a T cell receptor that has been used as starting point for the optimized T cell receptor;
and/or,
in which the binding between the cell and the non-target antigen and/or in which the activation of the cell by the non-target antigen is decreased compared to a cell that expresses a T cell receptor that has been used as starting point for the optimized T cell receptor; and
d) identifying at least one T cell receptor with optimized antigen-binding or antigen-induced signaling properties by sequencing the DNA fragment comprised in the at least one cell isolated in step (c).

13. A method for identifying at least one antigen-specific T cell receptor, the method comprising the steps of:
a) producing a plurality of cells using the method according to any one of claims 7 to 9, wherein the plurality of cells is produced by introducing the library of DNA fragments according to claim 10 into said cells;
b) contacting the plurality of cells of step (a) with an antigen;
c) isolating at least one cell from the plurality of cells, in which the binding between the cell and the antigen of step (b) is stronger compared to the binding between the cell and an irrelevant antigen, or, in which the binding between the cell and the antigen is stronger compared to the binding between the antigen and a cell that expresses an irrelevant or no T cell receptor;
and/or
in which the activation of the cell by the antigen of step (b) is higher compared to the activation of a cell that has been contacted with an irrelevant or no antigen, or, in which the activation of the cell by the antigen is higher compared to the activation of a cell that expresses an irrelevant or no T cell receptor and has been contacted with the antigen; and
d) identifying at least one antigen-specific T cell receptor by sequencing the DNA fragment comprised in the at least one cell isolated in step (c).

14. The method according to claim 13, wherein the antigen is presented on the surface of a tumor cell,
in particular wherein the tumor cell and the polynucleotide sequences of the TCR coding sequences in the library of DNA fragments have been obtained from the same subject.

15. The method according to any one of claims 11 to 14, wherein the antigen or the target antigen is a peptide bound to a major histocompatibility complex (MHC), in particular wherein the peptide is presented by an MHC on the surface of an antigen-presenting cell (APC) or by an MHC multimer; and/or
wherein the non-target antigen is a peptide that is presented by an APC, by an MHC multimer, or wherein the non-target antigen is a cell that does not present the target antigen on its cell surface.


SEQUENCE LISTING

<110> ETH Zürich

<120> Cell line for TCR discovery and engineering and methods of use thereof

<130> 2019-070

<160> 49

<170> BiSSAP 1.3.6

<210> 1
<211> 30
<212> DNA
<213> Homo sapiens

<220>
<223> composite NFAT:AP-1 binding element

<400> 1
ggaggaaaaa ctgtttcata cagaaggcgt                                        30

<210> 2
<211> 1368
<212> PRT
<213> Streptococcus pyogenes

<220>
<223> Cas9

<400> 2

<210> 3
<211> 893
<212> DNA
<213> Artificial Sequence

<220>
<223> 5\222 homology arm for VDJ exon

<400> 3
gcatgcctct gtgccaacag caaaaatgtg cccttctctt ttgttggcaa gtaacttaac      60

caaccaaccc aaaaaaaaga tctttctctc agctttccat aatctctgag acgaagtagg     120

tttggagaag tggggttaca ggggaaaaag ccaggtgtta atgatgaaaa aacattgaac     180

ttttctaggg gtagtaataa gatttaattc aagactagaa cattttagct gcaaatcttc     240

aagaataaga caatattatc ccctttctgt tttattgtgg gactagagaa tgtgagagag     300

gttacattcc atgggctttg ggaatttaat atggttcaag gataaacaca cccaggtttt     360

tcactgcaga gaagagcttc aaatataatc agttttcagg tcatcagctc agctcttgta     420

tccctaacaa tgcagttgac atgcgtcttc tcagatgtct aactcctaac tcactgaggg     480

atactttaag tacatataaa ggactagaag caccaagcta ccagtgagag gaagaggaga     540

gtttgcagag aagctggctt gaaataagac aatgagttca tctttaaata cttgccattt     600

gaggtgcaga tggatatagt tggcaggctc ctatgtaagg catgttatgg agaagctacc     660

gtgaattgat aatatcaaaa caaatatcca gggagcctct gcaagtgtgc atctctattt     720

cacaccaatt atagttgagt taattcctgc ctgattcatc tcccagagat gcagcctcct     780

cttaaagaag ttgggggtgg tggcccattc agtgatgtca ctgacagatg cattctgtgg     840

ggataaaatg tcacaaaatt catttctttg ctcatgctca cagagggcct ggt            893

<210> 4
<211> 878
<212> DNA

<213> Artificial Sequence

<220>
<223> 3\222 homology arm for VDJ exon

<400> 4
aggaggggtg cgatgaggga ggactctgtc ctgggaaatg tcaaagagaa cagagatccc      60

agctcccgga gccagactga gggagacgtc atgtcatgtc ccgggattga gttcagggga     120

ggctccctgt gagggcgaat ccacccaggc ttcccagagg ctctgagcag tcacagctga     180

gcccagggtg atggggcaga agagggaagg ggagggggcc tctcatcata gttccctgag     240

atagcccaga gaaagcccgg tgggtaatga atgagccaca acacctctcc atctatctgc     300

ttcactgaca gaggttctct gtagattctt cgtatattcc tgtgctggat tttataggag     360

gccactctgt gtctcttttt gtcacctgcc tgagtcttgg gcaagctctg gaagggaaca     420

cagagtactg gaagcagagc tgctgtccct gtgagggaag agttcccatg aactcccaac     480

ctctgcctga atcccagctg tgctcagcag agactggggg gttttgaagt ggccctggga     540

ggctgtgctc tggaaacacc atatattttg gagagggaag ttggctcact gttgtaggtg     600

agtaagtcaa ggctggacag ctgggaactt gcaaaaaggg gctggaatcc agacggagcc     660

tttgtctcta gtgcttaggt gaaagtgtat ttttgtcagg aaggcctatg aggcagatga     720

ggaggggata gcctccctct cctctccact attttgtaga ctgcctgtgc caagttaggt     780

tcccctactg agagatgggt agactcagct tggaaggggt caccttgaac atctcctgtc     840

tccttgaagg gtgccggtca cggccatgac agataaaa                             878

<210> 5
<211> 19
<212> PRT
<213> Porcine teschovirus 1

<220>
<223> 2A peptide derived from the porcine teschovirus-1 polyprotein

<400> 5
Ala Thr Asn Phe Ser Leu Leu Lys Gln Ala Gly Asp Val Glu Glu Asn
1               5                   10                  15
Pro Gly Pro

<210> 6
<211> 20
<212> RNA
<213> Artificial Sequence

<220>
<223> Jurkat VDJ crRNA

<400> 6

tcgacctgtt cggctaacta                                                    20

<210> 7
<211> 13
<212> PRT
<213> Homo sapiens

<220>
<223> MAGE A3 TCR beta CDR3

<400> 7
Cys Ala Ser Ser Pro Asn Met Ala Asp Glu Gln Tyr Phe
1               5                   10

<210> 8
<211> 39
<212> DNA
<213> Homo sapiens

<220>
<223> MAGE A3 TCR beta CDR3

<400> 8
tgcgccagca gcccgaatat ggcggatgaa cagtacttc                               39

<210> 9
<211> 133
<212> PRT
<213> Homo sapiens

<220>
<223> Wild type MAGE-A3 TCR alpha variable domain

<400> 9
Met Glu Thr Leu Leu Gly Leu Leu Ile Leu Trp Leu Gln Leu Gln Trp
1               5                   10                  15
Val Ser Ser Lys Gln Glu Val Thr Gln Ile Pro Ala Ala Leu Ser Val
            20                  25                  30
Pro Glu Gly Glu Asn Leu Val Leu Asn Cys Ser Phe Thr Asp Ser Ala
        35                  40                  45
Ile Tyr Asn Leu Gln Trp Phe Arg Gln Asp Pro Gly Lys Gly Leu Thr
    50                  55                  60
Ser Leu Leu Leu Ile Gln Ser Ser Gln Arg Glu Gln Thr Ser Gly Arg
65                  70                  75                  80
Leu Asn Ala Ser Leu Asp Lys Ser Ser Gly Arg Ser Thr Leu Tyr Ile
                85                  90                  95
Ala Ala Ser Gln Pro Gly Asp Ser Ala Thr Tyr Leu Cys Ala Val Arg
            100                 105                 110
Pro Gly Gly Ala Gly Ser Tyr Gln Leu Thr Phe Gly Lys Gly Thr Lys
        115                 120                 125
Leu Ser Val Ile Pro
    130

<210> 10
<211> 131
<212> PRT
<213> Homo sapiens

<220>
<223> Wild type MAGE-A3 TCR variable beta domain

<400> 10
Met Gly Ser Arg Leu Leu Cys Trp Val Leu Leu Cys Leu Leu Gly Ala
1               5                   10                  15
Gly Pro Val Lys Ala Gly Val Thr Gln Thr Pro Arg Tyr Leu Ile Lys
            20                  25                  30
Thr Arg Gly Gln Gln Val Thr Leu Ser Cys Ser Pro Ile Ser Gly His
        35                  40                  45
Arg Ser Val Ser Trp Tyr Gln Gln Thr Pro Gly Gln Gly Leu Gln Phe
    50                  55                  60
Leu Phe Glu Tyr Phe Ser Glu Thr Gln Arg Asn Lys Gly Asn Phe Pro
65                  70                  75                  80
Gly Arg Phe Ser Gly Arg Gln Phe Ser Asn Ser Arg Ser Glu Met Asn
                85                  90                  95
Val Ser Thr Leu Glu Leu Gly Asp Ser Ala Leu Tyr Leu Cys Ala Ser
            100                 105                 110
Ser Pro Asn Met Ala Asp Glu Gln Tyr Phe Gly Pro Gly Thr Arg Leu
        115                 120                 125
Thr Val Thr
    130

<210> 11
<211> 9
<212> PRT
<213> Homo sapiens


<220>
<223> MAGE A3 peptide antigen

<400> 11
Glu Val Asp Pro Ile Gly His Leu Tyr
1               5

<210> 12
<211> 133
<212> PRT
<213> Artificial Sequence


<220>
<223> Engineered a3a MAGE-A3 TCR variable alpha domain

<400> 12
Met Glu Thr Leu Leu Gly Leu Leu Ile Leu Trp Leu Gln Leu Gln Trp
1               5                   10                  15
Val Ser Ser Lys Gln Glu Val Thr Gln Ile Pro Ala Ala Leu Ser Val
            20                  25                  30
Pro Glu Gly Glu Asn Leu Val Leu Asn Cys Ser Phe Thr Asp Ser Ala
        35                  40                  45
Ile Tyr Asn Leu Gln Trp Phe Arg Gln Asp Pro Gly Lys Gly Leu Thr
    50                  55                  60
Ser Leu Leu Leu Val Arg Pro Tyr Gln Arg Glu Gln Thr Ser Gly Arg
65                  70                  75                  80
Leu Asn Ala Ser Leu Asp Lys Ser Ser Gly Arg Ser Thr Leu Tyr Ile
                85                  90                  95
Ala Ala Ser Gln Pro Gly Asp Ser Ala Thr Tyr Leu Cys Ala Val Arg
            100                 105                 110
Pro Gly Gly Ala Gly Ser Tyr Gln Leu Thr Phe Gly Lys Gly Thr Lys
        115                 120                 125

Leu Ser Val Ile Pro
    130

<210> 13
<211> 131
<212> PRT
<213> Artificial Sequence

<220>
<223> Engineered a3a MAGE-A3 TCR beta chain

<400> 13
Met Gly Ser Arg Leu Leu Cys Trp Val Leu Leu Cys Leu Leu Gly Ala
1               5                   10                  15
Gly Pro Val Lys Ala Gly Val Thr Gln Thr Pro Arg Tyr Leu Ile Lys
            20                  25                  30
Thr Arg Gly Gln Gln Val Thr Leu Ser Cys Ser Pro Ile Ser Gly His
        35                  40                  45
Arg Ser Val Ser Trp Tyr Gln Gln Thr Pro Gly Gln Gly Leu Gln Phe
    50                  55                  60
Leu Phe Glu Tyr Phe Ser Glu Thr Gln Arg Asn Lys Gly Asn Phe Pro
65                  70                  75                  80
Gly Arg Phe Ser Gly Arg Gln Phe Ser Asn Ser Arg Ser Glu Met Asn
                85                  90                  95
Val Ser Thr Leu Glu Leu Gly Asp Ser Ala Leu Tyr Leu Cys Ala Ser
            100                 105                 110
Ser Pro Asn Met Ala Asp Glu Gln Tyr Phe Gly Pro Gly Thr Arg Leu
        115                 120                 125
Thr Val Thr
    130

<210> 14
<211> 9
<212> PRT
<213> Homo sapiens

<220>
<223> Titin derived non-target peptide antigen

<400> 14
Glu Ser Asp Pro Ile Val Ala Gln Tyr
1               5

<210> 15
<211> 9
<212> PRT
<213> Homo sapiens

<220>
<223> NY-ESO-1 peptide antigen

<400> 15
Ser Leu Leu Met Trp Ile Thr Gln Cys
1               5

<210> 16
<211> 10
<212> PRT
<213> Homo sapiens

<220>
<223> MART-1 peptide antigen

<400> 16
Glu Leu Ala Gly Ile Gly Ile Leu Thr Val
1               5                   10

<210> 17
<211> 9
<212> PRT
<213> Homo sapiens


<220>
<223> CMV peptide antigen

<400> 17
Val Thr Glu His Asp Thr Leu Leu Tyr
1               5

<210> 18
<211> 20
<212> DNA
<213> Artificial Sequence


<220>
<223> Oligonucleotide RVL-127

<400> 18
gcatgcctct gtgccaacag                                                   20


<210> 19
<211> 24
<212> DNA
<213> Artificial Sequence


<220>
<223> Oligonucleotide RVL-128

<400> 19
ttttatctgt catggccgtg accg                                              24


<210> 20
<211> 20
<212> RNA
<213> Artificial Sequence


<220>
<223> CCR5 crRNA

<400> 20
tgacatcaat tattatacat                                                   20


<210> 21
<211> 20
<212> RNA

<213> Artificial Sequence

<220>
<223> eGFP crRNA

<400> 21
caactacaag acccgcgccg                                                    20

<210> 22
<211> 20
<212> RNA
<213> Artificial Sequence

<220>
<223> AAVS1 crRNA

<400> 22
ggggccacta gggacaggat                                                    20

<210> 23
<211> 20
<212> RNA
<213> Artificial Sequence

<220>
<223> CD4 crRNA

<400> 23
gcactgaggg gctactacca                                                    20

<210> 24
<211> 19
<212> RNA
<213> Artificial Sequence

<220>
<223> TRAC crRNA

<400> 24
cagggttctg gatatctgt                                                     19

<210> 25
<211> 20
<212> RNA
<213> Artificial Sequence

<220>
<223> Fas crRNA

<400> 25
ttggaaggcc tgcatcatga                                                    20

<210> 26

<211> 21
<212> DNA
<213> Artificial Sequence

<220>
<223> Oligonucleotide RVL-144

<400> 26
gaggagaacc ctggacctat g                                                21

<210> 27
<211> 22
<212> DNA
<213> Artificial Sequence

<220>
<223> Oligonucleotide RVL-145

<400> 27
ggaacacctt gttcaggtcc tc                                               22

<210> 28
<211> 39
<212> DNA
<213> Artificial Sequence

<220>
<223> ssODN_NNK1

<400> 28
tgcnnkagca gcccgaatat ggcggatgaa cagtacttc                             39

<210> 29
<211> 13
<212> PRT
<213> Artificial Sequence

<220>
<223> ssODN_NNK1

<400> 29
Cys Xaa Ser Ser Pro Asn Met Ala Asp Glu Gln Tyr Phe
1               5                   10

<210> 30
<211> 39
<212> DNA
<213> Artificial Sequence

<220>
<223> ssODN_NNK2

<400> 30
tgcgccnnka gcccgaatat ggcggatgaa cagtacttc                             39

<210> 31
<211> 13
<212> PRT
<213> Artificial Sequence

<220>
<223> ssODN_NNK2

<400> 31
Cys Ala Xaa Ser Pro Asn Met Ala Asp Glu Gln Tyr Phe
1               5                   10

<210> 32
<211> 39
<212> DNA
<213> Artificial Sequence

<220>
<223> ssODN_NNK3

<400> 32
tgcgccagcn nkccgaatat ggcggatgaa cagtacttc                              39

<210> 33
<211> 13
<212> PRT
<213> Artificial Sequence

<220>
<223> ssODN_NNK3

<400> 33
Cys Ala Ser Xaa Pro Asn Met Ala Asp Glu Gln Tyr Phe
1               5                   10

<210> 34
<211> 39
<212> DNA
<213> Artificial Sequence

<220>
<223> ssODN_NNK4

<400> 34
tgcgccagca gcnnkaatat ggcggatgaa cagtacttc                              39

<210> 35
<211> 13
<212> PRT
<213> Artificial Sequence

<220>
<223> ssODN_NNK4

<400> 35

Cys Ala Ser Ser Xaa Asn Met Ala Asp Glu Gln Tyr Phe
1               5                   10

<210> 36
<211> 39
<212> DNA
<213> Artificial Sequence

<220>
<223> ssODN_NNK5

<400> 36
tgcgccagca gcccgnnkat ggcggatgaa cagtacttc                              39

<210> 37
<211> 13
<212> PRT
<213> Artificial Sequence

<220>
<223> ssODN_NNK5

<400> 37
Cys Ala Ser Ser Pro Xaa Met Ala Asp Glu Gln Tyr Phe
1               5                   10

<210> 38
<211> 39
<212> DNA
<213> Artificial Sequence

<220>
<223> ssODN_NNK6

<400> 38
tgcgccagca gcccgaatnn kgcggatgaa cagtacttc                              39

<210> 39
<211> 13
<212> PRT
<213> Artificial Sequence

<220>
<223> ssODN_NNK6

<400> 39
Cys Ala Ser Ser Pro Asn Xaa Ala Asp Glu Gln Tyr Phe
1               5                   10

<210> 40
<211> 39
<212> DNA
<213> Artificial Sequence

<220>
<223> ssODN_NNK7

<400> 40
tgcgccagca gcccgaatat gnnkgatgaa cagtacttc                                39

<210> 41
<211> 13
<212> PRT
<213> Artificial Sequence

<220>
<223> ssODN_NNK7

<400> 41
Cys Ala Ser Ser Pro Asn Met Xaa Asp Glu Gln Tyr Phe
1               5                   10

<210> 42
<211> 39
<212> DNA
<213> Artificial Sequence

<220>
<223> ssODN_NNK8

<400> 42
tgcgccagca gcccgaatat ggcgnnkgaa cagtacttc                                39

<210> 43
<211> 13
<212> PRT
<213> Artificial Sequence

<220>
<223> ssODN_NNK8

<400> 43
Cys Ala Ser Ser Pro Asn Met Ala Xaa Glu Gln Tyr Phe
1               5                   10

<210> 44
<211> 39
<212> DNA
<213> Artificial Sequence

<220>
<223> ssODN_NNK9

<400> 44
tgcgccagca gcccgaatat ggcggatnnk cagtacttc                                39

<210> 45
<211> 13
<212> PRT
<213> Artificial Sequence

<220>
<223> ssODN_NNK9

<400> 45
Cys Ala Ser Ser Pro Asn Met Ala Asp Xaa Gln Tyr Phe
1               5                   10

<210> 46
<211> 39
<212> DNA
<213> Artificial Sequence


<220>
<223> ssODN_NNK10

<400> 46
tgcgccagca gcccgaatat ggcggatgaa nnktacttc                              39


<210> 47
<211> 13
<212> PRT
<213> Artificial Sequence


<220>
<223> ssODN_NNK10

<400> 47
Cys Ala Ser Ser Pro Asn Met Ala Asp Glu Xaa Tyr Phe
1               5                   10

<210> 48
<211> 39
<212> DNA
<213> Artificial Sequence


<220>
<223> ssODN_NNK11

<400> 48
tgcgccagca gcccgaatat ggcggatgaa cagnnkttc                              39


<210> 49
<211> 13
<212> PRT
<213> Artificial Sequence


<220>
<223> ssODN_NNK11

<400> 49
Cys Ala Ser Ser Pro Asn Met Ala Asp Glu Gln Xaa Phe
1               5                   10